# EUROPÄISCHE PATENTSCHRIFT

(11) **EP 0 295 210 B1**
(45) Veröffentlichungstag und Bekanntmachung des Hinweises auf die Patenterteilung: **04.08.1993**
(21) Anmeldenummer: 88810361.1
(22) Anmeldetag: 02.06.1988
(51) Int. Cl.: C07D 239/42, A01N 43/54

(54) **Mikrobizide**
Microbicides
Microbicides

(30) Priorität: 11.06.1987 CH 2188/87; 11.04.1988 CH 1318/88
(43) Veröffentlichungstag der Anmeldung: 14.12.1988
(73) Patentinhaber: CIBA-GEIGY AG, 4002 Basel (CH)
(72) Erfinder: Hubele, Adolf, Dr., CH-4312 Magden (CH)

(56) Entgegenhaltungen:
- EP-A- 0 135 472
- EP-A- 0 172 786
- EP-A- 0 264 348
- EP-A- 0 270 111
- DD-A- 151 404
- CHEMICAL ABSTRACTS, Band 103, Nr. 1, 8. Juli 1985; A. KREUTZBERGER et al.: "Antimycotic substances" Seite 569, Zusammenfassung-Nr. 6301r
- PATENT ABSTRACTS OF JAPAN, Band 5, Nr. 132 (C-68)(804), 22. August 1981; & JP - A - 56 65 804
- PATENT ABSTRACTS OF JAPAN, Band 11, Nr. 317 (C-452)(2764), 15. Oktober 1987; & JP - A - 62 106 084

## Beschreibung

### Beschreibung für die Vertragstaaten: AT, BE, ES, GR, LU, SE

Die vorliegende Erfindung betrifft neue 2-Anilino-pyrimidin-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von Schädlingen, vor allem schädlichen Insekten und Pflanzenschädigenden Mikroorganismen, vorzugsweise Pilzen.

Die erfindungsgemässen Verbindungen entsprechen der allgemeinen Formel I 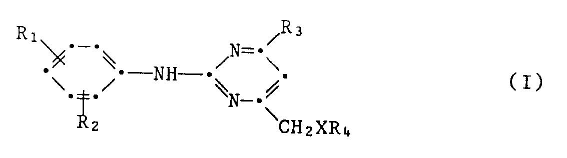 in welcher bedeuten:
R₁ und R₂ unabhängig voneinander Wasserstoff, Halogen, C1-C3-Alkyl, C₁-C₂-Halogenalkyl, C₁-C₃-Alkoxy oder C₁ -C₃ Halogenalkoxy;
R₃ Wasserstoff, C1-C4-Alkyl; durch Halogen oder Hydroxy substituiertes C1-C2-Alkyl, Cyclopropyl oder durch Halogen und/oder Methyl substituiertes Cyclopropyl;
R₄ C₁-C₈-Alkyl; durch Halogen, Hydroxy, Cyano, C₁-C₄-Alkoxy, C₃-C₄-Alkoxyalkoxy oder C₁-C₃-Alkylthio substituiertes C₂-C₆-Alkyl; C₃-C₆-Alkenyl; C₃-C₆-Alkinyl; C₃-C₇-Cycloalkyl oder durch Methyl substituiertes C₃-C₇-Cycloalkyl;
X Sauerstoff oder Schwefel.

Unter Alkyl selbst oder als Bestandteil eines anderen Substituenten, wie Halogenalkyl, Alkoxy oder Halogenalkoxy, sind je nach Anzahl der benannten Kohlenstoffatome beispielsweise zu verstehen: Methyl, Ethyl, Propyl, Butyl, Pentyl, Hexyl, Heptyl, Octyl sowie ihre Isomeren, wie z.B. Isopropyl, Isobutyl, tert.-Butyl, Isopentyl usw.. Halogen steht für Fluor, Chlor, Brom oder Jod. Halogenalkyl und Halogenalkoxy bezeichnen einfach bis perhalogenierte Reste, wie z.B. CHCI₂, CH₂F, CC1₃, CH₂CI, CHF₂, CH₂CH₂Br, C₂Cl₅, CHBr, CHBrCI usw., vorzugsweise CF₃. Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl. Cycloalkyl steht je nach Zahl der genannten Kohlenstoffatome z.B. für Cyclopropyl, Cyclobutyl, Cyclopentyl, Cyclohexyl oder Cycloheptyl.

Verbindungen mit einer N-Pyrimidylanilinstruktur sind bereits bekannt. So sind in der publizierten japanischen Patentanmeldung Sho 56-65804 sowie in der DDR-Patentschrift 151404 und in Chemical Abstracts, Bd. 103, Nr. 18, Juli 1985, 6301 r Verbindungen, die diese Grundstruktur besitzen, als wirksam gegen Pflanzenschädlinge beschrieben. Des weiteren sind aus EP-A-264 348, EP-A-135 472 und aus EP-A-172 786 2-Nitroanilinpyrimidinebekannt. Die bekannten Verbindungen konnten jedoch bisher die in der Praxis an sie gestellten Forderungen nicht in vollem Masse befriedigen. Die erfindungsgemässen Verbindungen der Formel I unterscheiden sich in charakteristischer Weise von den bekannten Verbindungen durch die Einführung spezifischer Substituenten und deren Kombination in die Anilinopyrimidin-Struktur, wodurch bei den neuen Verbindungen eine unerwartet hohe fungizide Wirksamkeit und insektizide Wirkung erreicht wird.

Die Verbindungen der Formel 1 sind bei Raumtemperatur stabile Oele, Harze oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende insektizide und fungizide Wirkung, sondern auch durch besonders gute Pflanzenverträglichkeit aus.

Eine wichtige Gruppe von Pflanzenfungiziden sind jene der Formel I, bei denen R₁ und R₂ Wasserstoff bedeuten.

Eine besondere Gruppe von Anilino-pyrimidin-Derivaten stellen Verbindungen der Formel 1 dar, in denen
R₁ und R₂ unabhängig voneinander Wasserstoff, Halogen, C₁-C₃-Alkyl, C₁-C₂-Halogenalkyl, C₁-C₃-Alkoxy oder C₁ -C₃ Halogenalkoxy;
R₃ Wasserstoff, C₁-C₄-Alkyl oder Cyclopropyl; R₄ C₁-C₈-Alkyl; durch Halogen, Hydroxy, Cyano, C₁-C₄-Alkoxy, C3-C4-Alkoxyalkoxy, C₁-C₃-Alkylthio substituiertes C₂-C₆-Alkyl; C₃-C₆-Alkenyl; C₃-C₆-Alkinyl; C₅-C₇-Cycloalkyl oder durch Methyl substituiertes C₅-C₇-Cycloalkyl; und
X Sauerstoff oder Schwefel bedeuten.

Folgende Wirkstoffgruppen sind aufgrund ihrer ausgeprägten bioziden, insbesondere pflanzenfungiziden, Aktivität bevorzugt:

Gruppe 1 a: Verbindungen der Formel I, worin bedeuten:
R₁ und R₂ unabhängig voneinander Wasserstoff, Fluor, Chlor, Brom, C₁-C₃-Alkyl, C₁-C₂-Halogenalkyl, Ci-C₃-Alkoxy oder C₁-C₂-Halogenalkoxy;
R₃ Wasserstoff, C₁-C₃-Alkyl oder Cyclopropyl;
R₄ C₁-C₆-Alkyl; durch Halogen, Hydroxy, Cyano, C₁-C₃-Alkoxy, C₃-C₄-Alkoxyalkoxy oder C₁-C₂-Alkylthio substituiertes C₂-C₄-Alkyl; C₃-Alkenyl; C₃-Alkinyl oder unsubstituiertes oder durch Methyl substituiertes C₆-C₇-Cycloalkyl;
X Sauerstoff oder Schwefel.

Unter den vorstehend genannten Verbindungen stellen jene eine besonders bevorzugte Gruppe dar, bei denen R₁ = R₂ = Wasserstoff ist
(= Gruppe 1aa).

Gruppe 1 b: Verbindungen der Formel I, worin bedeuten:
R₁ und R₂ unabhängig voneinander Wasserstoff, Fluor, Chlor, Brom, C₁-C₃-Alkyl, C₁-C₃-Alkoxy, Trifluormethyl, -OCHF₂, -OCF₂CHF₂, -OCF₂CHCIF, -OCF₂CHCI₂ oder -OCF₂CCI₂F;
R₃ Wasserstoff, Methyl, Ethyl oder n-Propyl;
R₄ C₁-C₄-Alkyl; durch Halogen, Hydroxy, Cyano, C₁-C₂-Alkoxy oder C₁-C₂-Alkylthio substituiertes C₂-C₃-Alkyl; C₃-C₆-Alkenyl; C₃-C₆-Alkinyl oder Cyclohexyl;
X Sauerstoff oder Schwefel.

Unter den vorstehend genannten Verbindungen stellen jene eine besonders bevorzugte Gruppe dar, bei denen R₁ = R₂ = Wasserstoff ist
(= Gruppe 1bb).

Gruppe 1 c: Verbindungen der Formel I, worin bedeuten:
R₁ und R₂ unabhängig voneinander Wasserstoff, Fluor, Chlor, Brom, C₁-C₂-Alkyl, C₁-C₂-Alkoxy, Trifluormethyl, -OCHF₂, -OCF₃, -OCF₂CHF₂ oder -OCF₂CHCIF;
R₃ Wasserstoff oder C₁-C₂-Alkyl;
R₄ C₁-C₃-Alkyl; durch Halogen, Hydroxy, Cyano, C₁-C₂-Alkoxy oder C₁-C₂-Alkylthio substituiertes C₂-Alkyl; C₃-C₄-Alkenyl; C₃-C₄-Alkinyl;
X Sauerstoff oder Schwefel.

Unter den vorstehend genannten Verbindungen stellen jene eine besonders bevorzugte Gruppe dar, bei denen R₁ = R₂ = Wasserstoff ist und X Sauerstoff bedeutet (= Gruppe 1cc).

Gruppe 1 d: Verbindungen der Formel I, worin bedeuten:
R₁ und R₂ unabhängig voneinander Wasserstoff, Fluor, Chlor, Brom, C₁-C₂-Alkyl, C₁-C₂-Alkoxy, Trifluormethyl oder -OCHF₂;
R₃ Methyl;
R₄ C₁-C₂-Alkyl; durch Fluor, Chlor, Brom, Cyano, C₁-C₂-Alkoxy oder Methylthio substituiertes C₂-Alkyl; Allyl oder Propargyl;
X Sauerstoff oder Schwefel.

Unter den vorstehend genannten Verbindungen stellen jene eine besonders bevorzugte Gruppe dar, bei denen R₁ = R₂ = Wasserstoff ist und X Sauerstoff bedeutet (= Gruppe 1dd).

Gruppe 2a: Verbindungen der Formel I, worin bedeuten:
R₁ und R₂ unabhängig voneinander Wasserstoff, Halogen, C₁-C₂-Alkyl, Halogenmethyl, C₁-C₂-Alkoxy oder C₁ -C₂-Halogenalkoxy;
R₃ Wasserstoff, C₁-C₃-Alkyl; durch Halogen oder Hydroxy substituiertes C₁-C₂-Alkyl; Cyclopropyl oder durch Halogen und/oder Methyl ein- bis dreifach gleich oder verschieden substituiertes Cyclopropyl;
R₄ C₁-C₄-Alkyl; durch Halogen, Cyano, C₁-C₂-Alkoxy oder C₁-C₂-Alkylthio substituiertes C₂-C₃-Alkyl; C₃-C₆-Alkenyl; C₃-C₆-Alkinyl oder C₃-C₇-Cycloalkyl;
X Sauerstoff oder Schwefel.

Unter den vorstehend genannten Verbindungen stellen jene eine besonders bevorzugte Gruppe dar, bei denen R₁ = R₂ = Wasserstoff ist
(= Gruppe 2aa).

Gruppe 2b: Verbindungen der Formel I, worin bedeuten:
R₁ und R₂ unabhängig voneinander Wasserstoff, Fluor, Chlor, Brom, Methyl, Trifluormethyl, Methoxy oder Difluormethoxy;
R₃ Wasserstoff, C₁-C₃-Alkyl; durch Halogen oder Hydroxy substituiertes C₁-C₂-Alkyl; Cyclopropyl, oder durch Halogen und/oder Methyl ein- bis dreifach gleich oder verschieden substituiertes Cyclopropyl;
R₄ C₁-C₃-Alkyl; durch Fluor, Chlor oder C₁-C₂-Alkoxy substituiertes C₂-C₃-Alkyl; C₃-C₆-Alkenyl; C₃-C₆-Alkinyl oder C₃-C₆-Cycloalkyl;
X Sauerstoff oder Schwefel.

Unter den vorstehend genannten Verbindungen stellen jene eine besonders bevorzugte Gruppe dar, bei denen R₁ = R₂ = Wasserstoff ist
(= Gruppe 2bb).

Gruppe 2c: Verbindungen der Formel I, worin bedeuten:
R₁ und R₂ unabhängig voneinander Wasserstoff, Fluor, Chlor, Methyl, Trifluormethyl, Methoxy oder Difluormethoxy;
R₃ C₁-C₃-Alkyl; durch Halogen oder Hydroxy substituiertes Methyl; Cyclopropyl oder durch Halogen oder Methyl ein- bis dreifach substituiertes Cyclopropyl;
R₄ C1-C3-Alkyl; durch Fluor, Chlor oder Methoxy substituiertes C₂-C₃-Alkyl; C₃-C₄-Alkenyl; C₃-C₄-Alkinyl; Cyclopropyl; Cyclohexyl;
X Sauerstoff.

Unter den vors tehend genannten Verbindungen stellen jene eine besonders bevorzugte Gruppe dar, bei denen R₁ = R₂ = Wasserstoff ist
(= Gruppe 2cc).

Gruppe 2d: Verbindungen der Formel I, worin bedeuten:
R₁ und R₂ Wasserstoff;
R₃ C₁-C₃-Alkyl; durch Fluor, Chlor oder Brom substituiertes Methyl;
Cyclopropyl oder durch Chlor oder Methyl substituiertes Cyclopropyl;
R₄ C₁-C₃-Alkyl, 2-Chlorethyl, 2,2,2-Trifluorethyl, Allyl oder Propargyl;
X Sauerstoff.

Besonders bevorzugte Einzelsubstanzen sind z.B.:
2-Phenylamino-4-methyl-6-methoxymethyl-pyrimidin (Verb.Nr.1);
2-Phenylamino-4-cyclopropyl-6-methoxymethyl-pyrimidin (Verb.Nr.10).

Die Verbindungen der Formel I werden hergestellt, indem man
1. ein Phenylguanidinsalz der Formel Ila 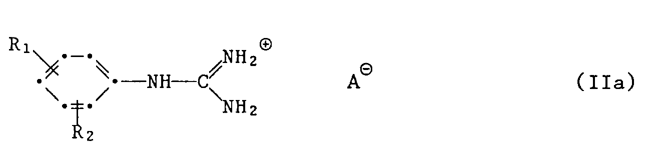 oder das zugrundeliegende Guanidin der Formel Ilb 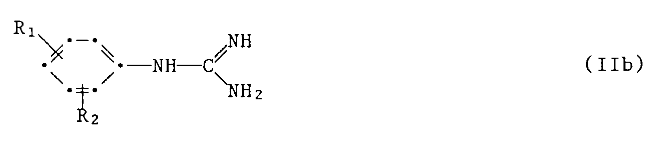 mit einem Diketon der Formel lll 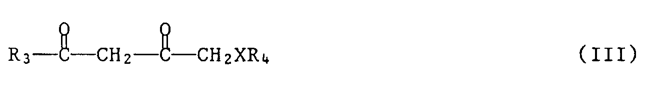 ohne Lösungsmittel oder in einem aprotischen, bevorzugt aber in einem protischen Lösungsmittel bei Temperaturen von 60 °C bis 160 °C, bevorzugt 60 °C bis 110 °C, umsetzt oder
2. in einem mehrstufigen Verfahren:
   2.1 Harnstoff der Formel IV 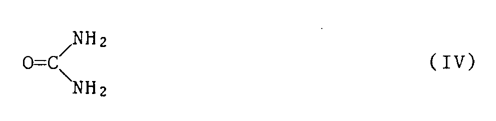 mit einem Keton der Formel lll 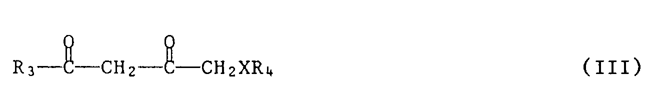 in Gegenwart einer Säure in einem inerten Lösungsmittel bei Temperaturen von 20 °C bis 140 °C, bevorzugt 40 ° C bis 100 ° C, zur Reaktion bringt und danach bei Rückflusstemperatur des verwendeten Lösungsmittels zu einer Pyrimidinverbindung der Formel V 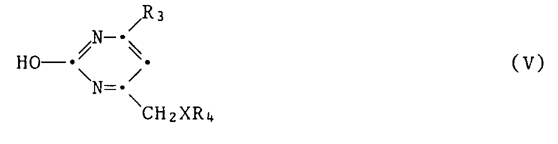 cyclisiert und
   2.2 die erhaltene Verbindung der Formel V weiter mit überschüssigem POCl₃ ohne Lösungsmittel oder in einem gegen POCl₃ inerten Lösungsmittel bei Temperaturen von 50 ° C bis 110 ° C, bevorzugt bei der Rückflusstemperatur des POC!₃, zu der Verbindung der Formel VI 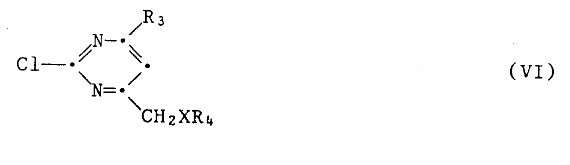 umsetzt und
   2.3 die erhaltene Verbindung der Formel VI weiter mit einer Anilinverbindung der Formel VII 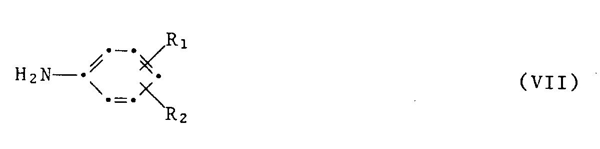 je nach Verfahrensbedingungen entweder
      a) in Gegenwart eines Protonenakzeptors, wie der überschüssigen Anilinverbindung der Formel VII oder einer anorganischen Base, ohne Lösungsmittel oder in einem protischen oder aprotischen Lösungsmittel oder
      b) in Gegenwart einer Säure in einem inerten Lösungsmittel bei jeweils Temperaturen von 60 ° C bis 120 ° C, bevorzugt 80 ° C bis 100 ° C, zur Reaktion bringt; oder
3. in einem zwei-stufigen Verfahren:
   3.1 ein Guanidiniumsalz der Formel VIII 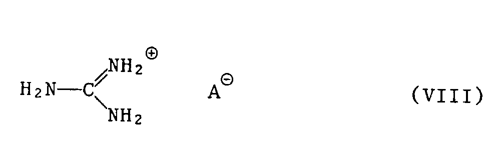 mit einem Diketon der Formel lll 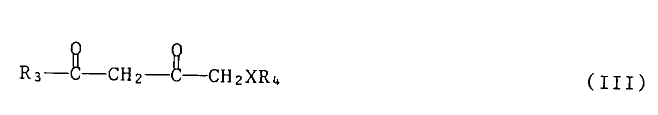
      a) ohne Lösungsmittel bei Temperaturen von 100°C bis 160 ° C, bevorzugt 120-150 ° C, oder
      b) in einem protischen oder aprotischen Lösungsmittel oder einem Gemisch aus protischen und aprotischen Lösungsmitteln bei Temperaturen von 30 ° C bis 140 ° C, bevorzugt 60 ° C bis 120 ° C, zu einer Pyrimidinverbindung der Formel IX 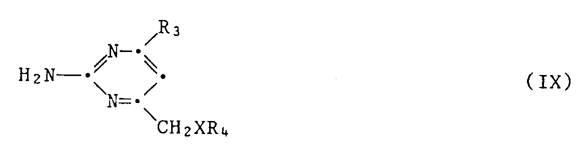 cyclisiert und
   3.2 die erhaltene Verbindung der Formel IX mit einer Verbindung der Formel X 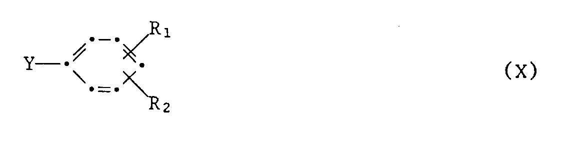 unter Abspaltung von HY in Gegenwart eines Protonenakzeptors in aprotischen Lösungsmitteln bei Temperaturen von 30 ° C bis 140 ° C, bevorzugt 60 ° C bis 120 ° C, umsetzt, wobei in den Formeln 11 bis X die Substituenten R₁ bis R₄ und X die unter Formel I angegebenen Bedeutungen besitzen, A^{S} ein Säureanion und Y Halogen darstellen.

In den vorstehend beschriebenen Verfahren kommen bei den Verbindungen der Formeln Ila und VIII mit dem Säureanion A^{S} beispielsweise folgende Salze in Betracht: Carbonat, Hydrogencarbonat, Nitrat, Halogenid, Sulfat oder Hydrogensulfat. Unter Halogenid sind Fluorid, Chlorid, Bromid oder Jodid, bevorzugt Chlorid oder Bromid zu verstehen.

Als Säuren finden vornehmlich anorganische Säuren, wie z.B. Halogenwasserstoffsäuren, beispielsweise Fluorwasserstoffsäure, Chlorwasserstoffsäure oder Bromwasserstoffsäure, sowie Schwefelsäure, Phosphorsäure oder Salpetersäure Verwendung; jedoch können auch geeignete organische Säuren angewendet werden.

Als Protonenakzeptoren dienen z.B. anorganische Basen, wie beispielsweise Alkali- oder Erdalkaliverbindungen, z.B. die Hydroxide, Oxide oder Karbonate von Lithium, Natrium, Kalium, Magnesium, Calcium, Strontium und Barium oder auch Hydride, wie z.B. Natriumhydrid.

In den vorstehend beschriebenen Verfahren können in Anpassung an die jeweiligen Reaktionsbedingungen beispielsweise folgende Lösungsmittel verwendet werden:
Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Chlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Dichlorbenzol, Dibrombenzol, Chlortoluol, Trichlortoluol; Ether, wie Ethylpropylether, Methyl-tert.-butylether, n-Butylethylether, Di-n-butylether, Di-isobutylether, Diisoamylether, Diisopropylether, Anisol, Cyclohexylmethylether, Diethylether, Ethylenglykoldimethylether, Tetrahydrofuran, Dioxan, Thioanisol, Dichlordiethylether; Nitrokohlenwasserstoffe wie Nitromethan, Nitroethan, Nitrobenzol, Chlornitrobenzol, o-Hitrotoluol; Nitrile wie Acetonitril, Butyronitril, Isobutyronitril, Benzonitril, m-Chlorbenzonitril; aliphatische oder cycloaliphatische Kohlenwasserstoffe, wie Heptan, Hexan, Octan, Nonan, Cymol, Benzinfraktionen innerhalb eines Siedepunktintervalles von 70 _{°} C bis 190°C, Cyclohexan, Methylcyclohexan, Dekalin, Petrolether, Ligroin, Trimethylpentan wie 2,3,3-Trimethylpentan; Ester wie Ethylacetat, Acetessigester, Isobutylacetat; Amide, z.B. Formamid, Methylformamid, Dimethylformamid; Ketone, wie Aceton, Methylethylketon; Alkohole, insbesondere niedere aliphatische Alkohole, wie z.B. Methanol, Ethanol, n-Propanol, iso-Propanol sowie die Butanole; gegebenenfalls auch Wasser. Auch Gemische der genannten Lösungs- und Verdünnungsmittel kommen in Betracht.

Zu den vorstehend beschriebenen Herstellungsverfahren analoge Synthesemethoden sind in der Literatur an folgenden Stellen publiziert:
Verfahren 1: A.Kreutzberger und J.Gillessen, J.Heterocyclic Chem.22, 101 (1985).
Verfahren 2, Stufe 2.1: O. Stark, Ber.Dtsch.Chem.Ges.42, 699 (1909); J. Hale, J.Am.Chem.Soc.36, 104 (1914); G.M. Kosolapoff, J.Org.Chem.26, 1895 (1961). Stufe 2.2: St. Angerstein, Ber.Dtsch.Chem.Ges.34, 3956 (1901); G.M. Kosolapoff, J.Org.Chem.26, 1895 (1961). Stufe 2.3: M.P.V. Boarland and J.F.W. McOmie, J.Chem.Soc. 1951, 1218; T.Matsukawa und K. Shirakuwa, J.Pharm.Soc.Japan 71, 933 (1951); Chem.Abstr.46, 4549 (1952).
Verfahren 3: Ä. Combes und C. Combes, Bull. Soc. Chem.(3), 7, 791 (1892); W.J. Hale und F.C. Vibrans, J.Am.Chem.Soc.40, 1046 (1918).

Die beschriebenen Herstellungsverfahren einschliesslich aller Teilschritte sind ein Bestandteil der vorliegenden Erfindung.

Ueberraschenderwei se wurde nun gefunden, dass Verbindungen der Formel I ein für praktische Bedürfnisse sehr günstiges biozides Spektrum zur Bekämpfung von Insekten und phytopathogenen Mikroorganismen, insbesondere Fungi aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel 1 sind z.B. gegen die den folgenden Klassen angehörenden phytopathogenen Pilze wirksam: Fungi imperfecti (insbesondere Botrytis, Helminthosporium, Fusarium, Septoria, Cercospora und Alternaria); Basidiomyceten (z.B. die Gattungen Hemileia, Rhizoctonia, Puccinia); und Ascomyceten (z.B. Venturia, Podosphaera, Erysiphe, Monilinia, Uncinula). Sie wirken auch gegen Oomyceten (Perenosporales, Phytophthora, Plasmopara, Pythium). Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Verbindungen der Formel 1 sind darüberhinaus gegen Schadinsekten wirksam, z.B. gegen Schädlinge an Getreide wie Reis.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel 1 bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Hirn- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen-und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel 1 bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha, bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen C₈ bis Cl 2, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden. Besonders vorteilhafte applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, die man beispielsweise aus Sojabohnen gewinnen kann.

Als oberflächenaktive Verbindungen kommen je nach Art des zu formulierenden Wirkstoffs der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren (C₁₀-C₂₂), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdlalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind in wasserlöslichen 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

### Beispiel 1: Herstellung von 2-Phenylamino-4-methyl-6-methoxymethylpyrimidin

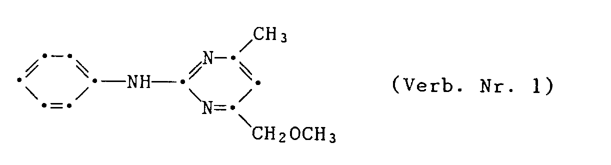

Ein Gemisch von 7,5 g Phenylguanidin-hydrogencarbonat und 7,4 g Methoxyacetylaceton werden unter Rühren vier Stunden auf 100°C erwärmt, wobei die einsetzende Kohlendioxidentwicklung mit fortschreitender Reaktionsdauer nachlässt. Nach dem Abkühlen auf Raumtemperatur wird die braune Emulsion mit 60 ml Diethylether versetzt, dreimal mit je 20 ml Wasser gewaschen, über Natriumsulfat getrocknet, filtriert und das Lösungsmittel verdampft. Die 10,5 g öliger Rückstand werden in 140 ml Diethylether gelöst und unter Rühren mit 4,4 g 65 %iger Salpetersäure versetzt. Die Suspension des ausgefallenen Nitratsalzes wird noch 20 Minuten nachgerührt, filtriert und mit 100 ml Diethylether nachgewaschen. Ein Gemisch, bestehend aus 11 g des Nitratsalzes, 80 ml Diethylether und 60 ml Wasser wird unter Rühren mit 6 g 30 %iger Natronlauge versetzt, die organische Phase abgetrennt, zweimal mit je 40 ml Wasser gewaschen, über Natriumsulfat getrocknet, filtriert und das Lösungsmittel verdampft. Die 8,7 g des hellbraunen öligen Rückstandes werden mit 100 ml Petrolether (Sdp. 50-70 °C) zur Kristallisation gebracht, filtriert und getrocknet. Die 8 g des hellen, beige-farbenen Kristallpulvers schmelzen bei 59-60 ° C; Ausbeute: 92 % d.Th. bezogen auf Phenylguanidin-hydrogencarbonat.

### Beispiel 2: Herstellung von 2-(p-Chlorphenylamino)-4-methyl-6-methoxymethyl-pyrimidin

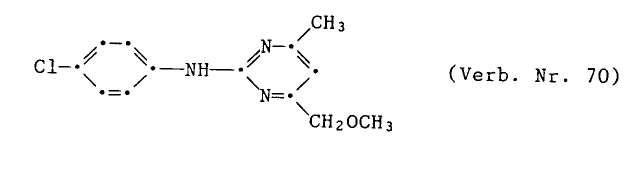

Eine Lösung von 6,4 g 4-Chloranilin und 8,6 g 2-Chlor-4-methyl-6-methoxymethyl-pyrimidin wird unter Rühren mit 5 ml konz. Salzsäure auf pH 1 gebracht und anschliessend 20 Stunden unter Rückfluss erhitzt. Nach dem Abkühlen auf Raumtemperatur wird die braune Emulsion mit 12 ml 30%igem Ammoniak alkalisch gestellt, auf 100 ml Eiswasser gegossen und dreimal mit je 50 ml Ethylacetat extrahiert. Die vereinigten Extrakte werden mit 50 ml Wasser gewaschen, über Natriumsulfat getrocknet, filtriert und das Lösungsmittel verdampft. Die 11,8 g rotes Oel werden über eine 30 cm lange Kieselgelsäule mittels Dichlormethan/Diethylether (3:2) chromatographiert. Nach Verdampfen des Laufmittels wird der zunächst ölige Rückstand durch Anreiben mit Petrolether zur Kristallisation gebracht. Nach dem Umkristallisieren aus Diisopropylether/Petrolether (50-70 ° C) werden 9,8 g beige-farbenes Kristallpulver erhalten, das bei 57-59 ° C schmilzt; Ausbeute: 74 % d. Th.

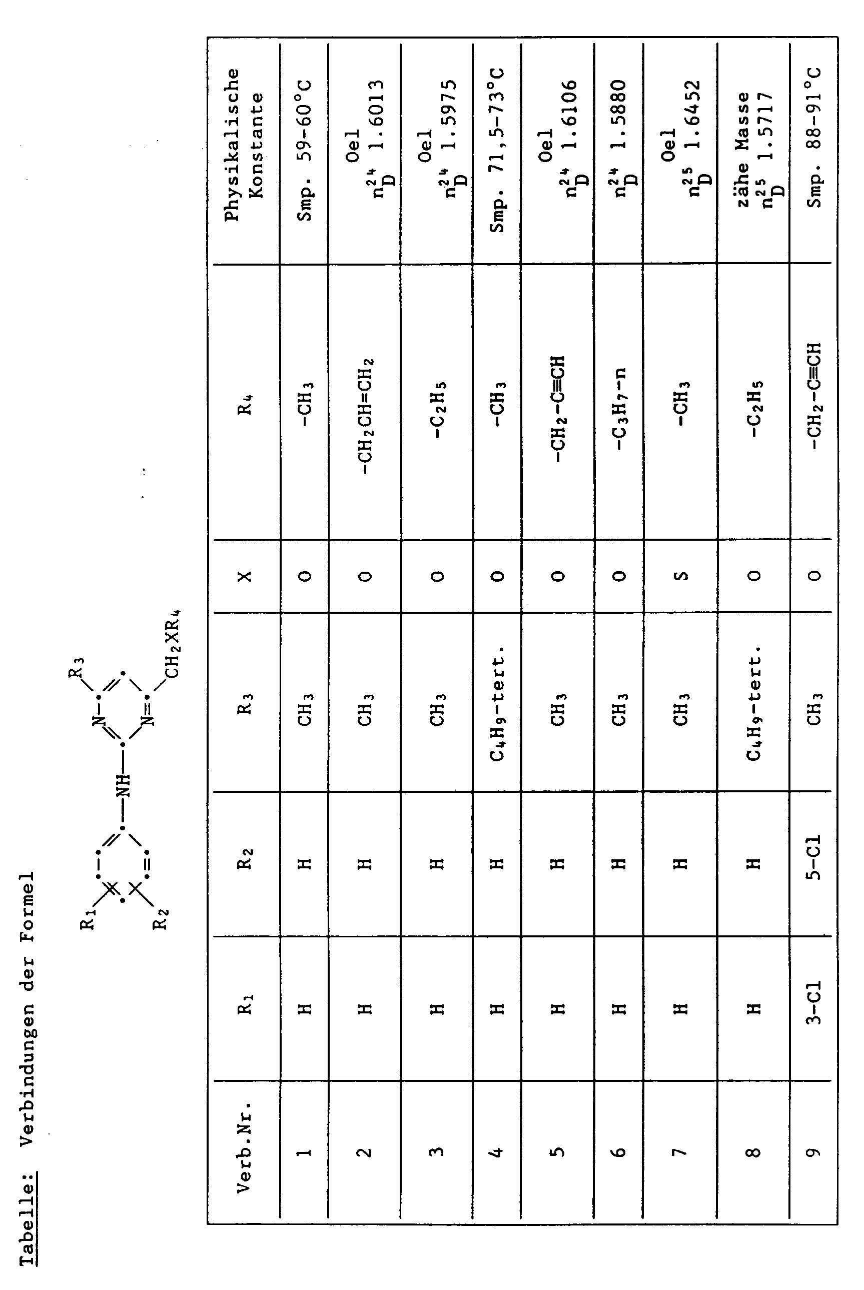

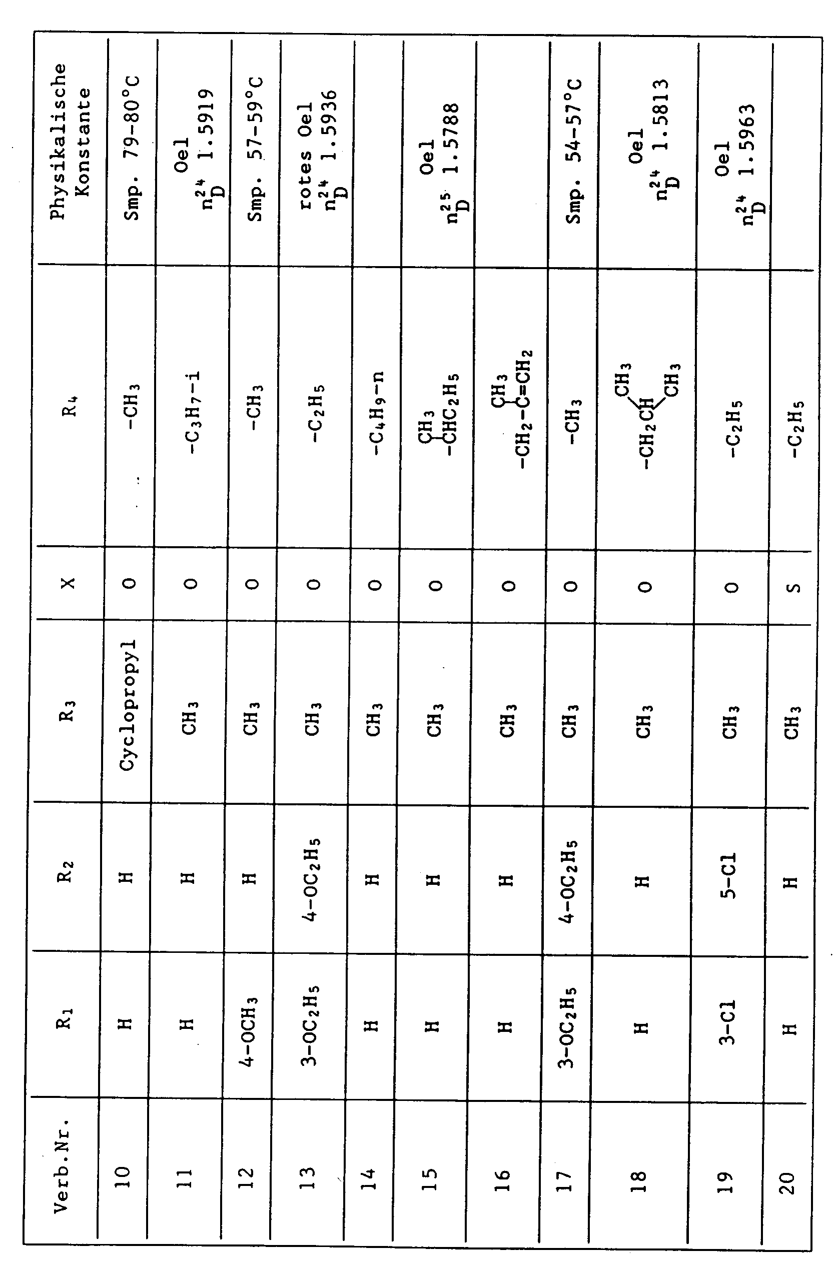

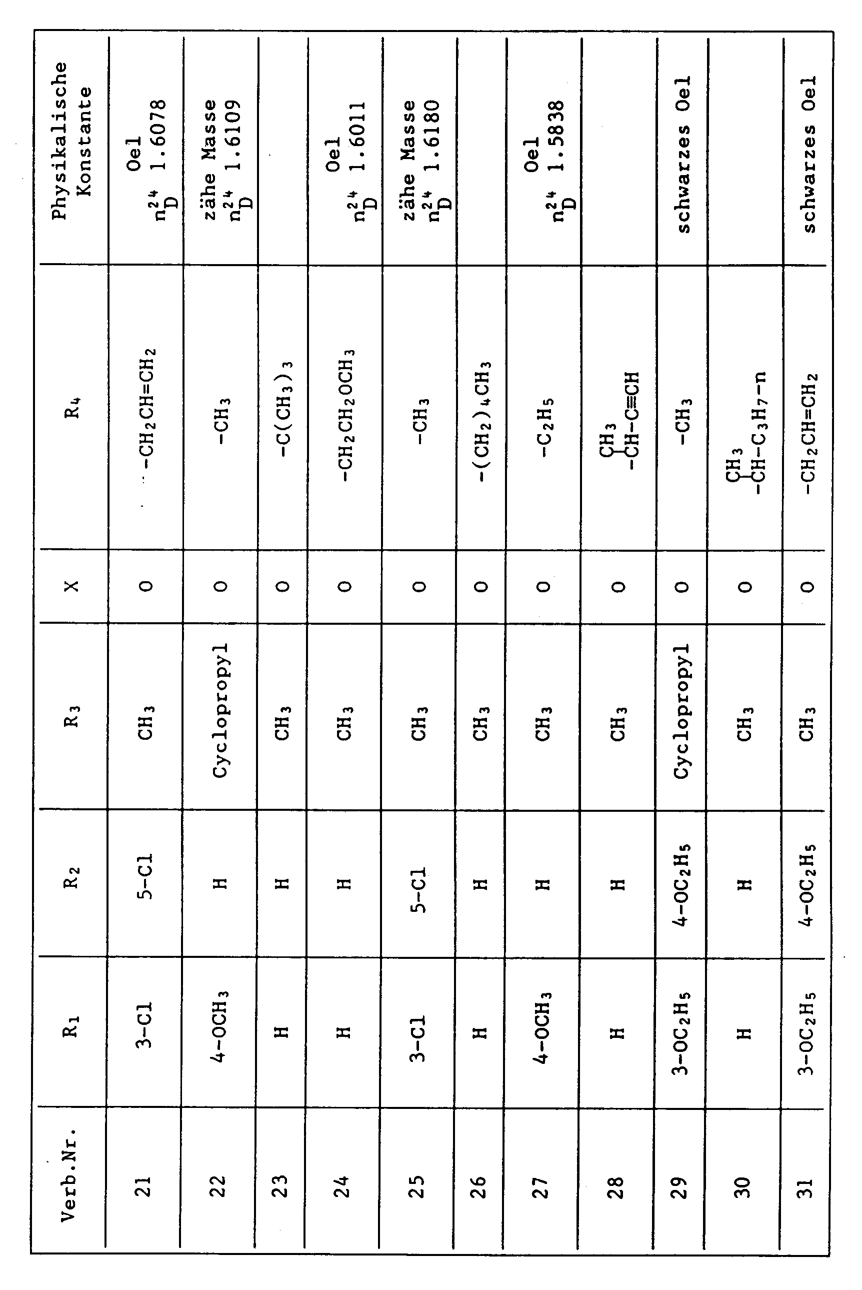

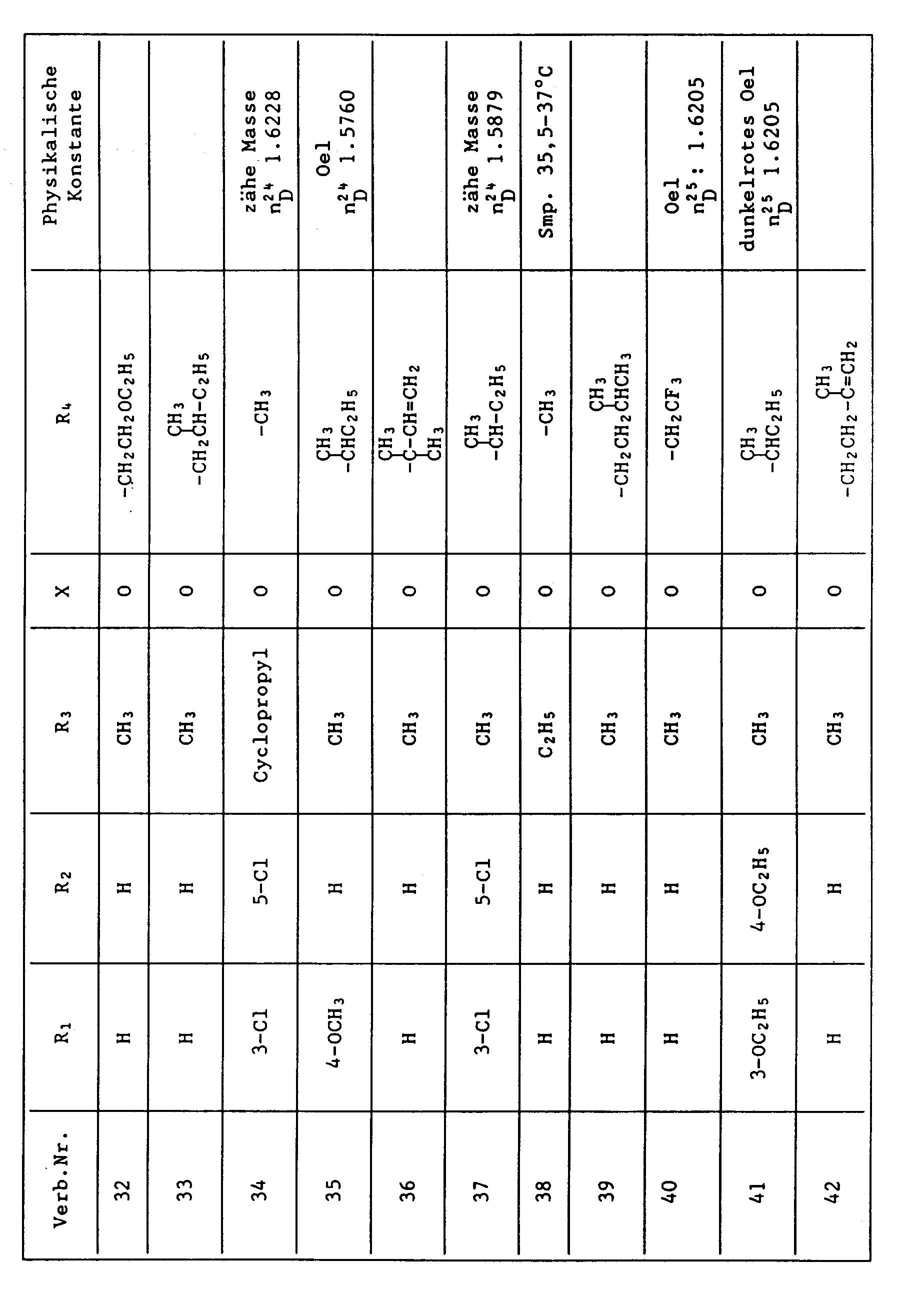

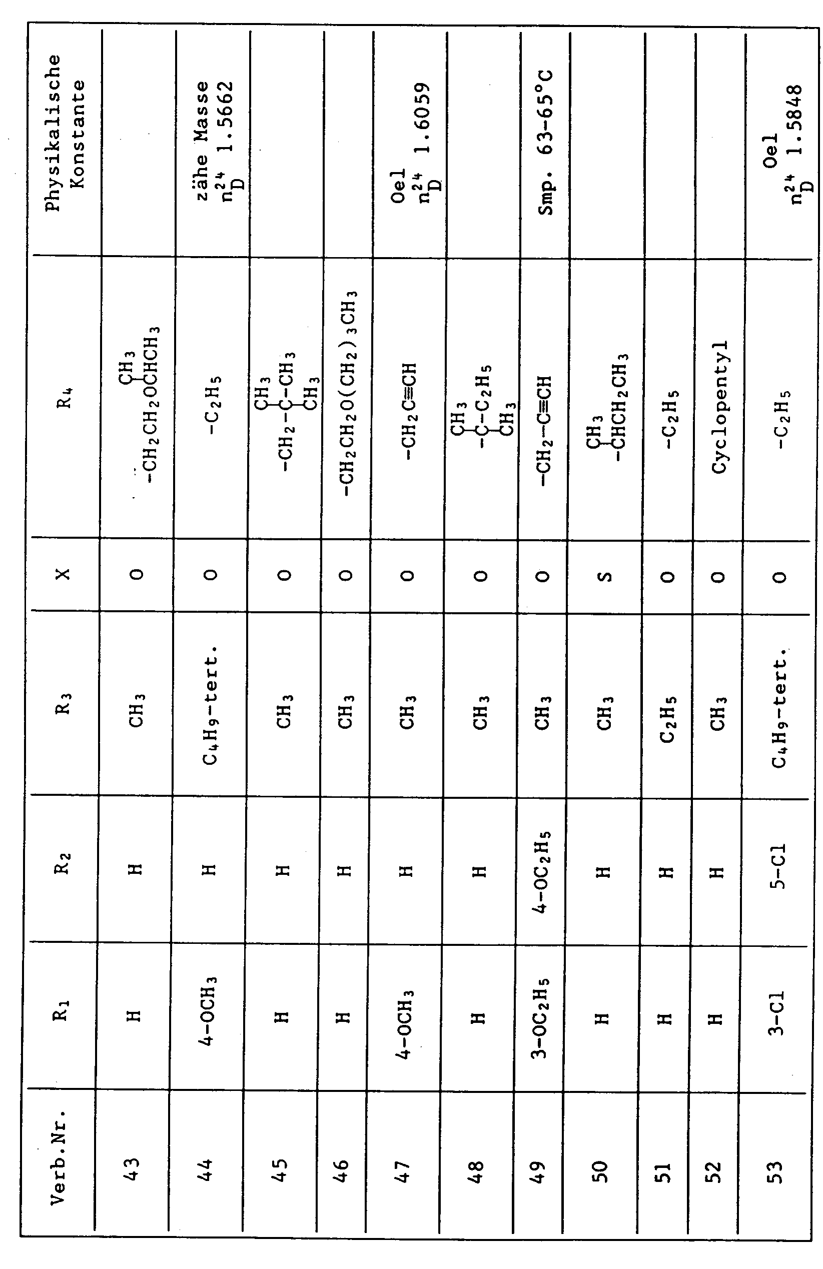

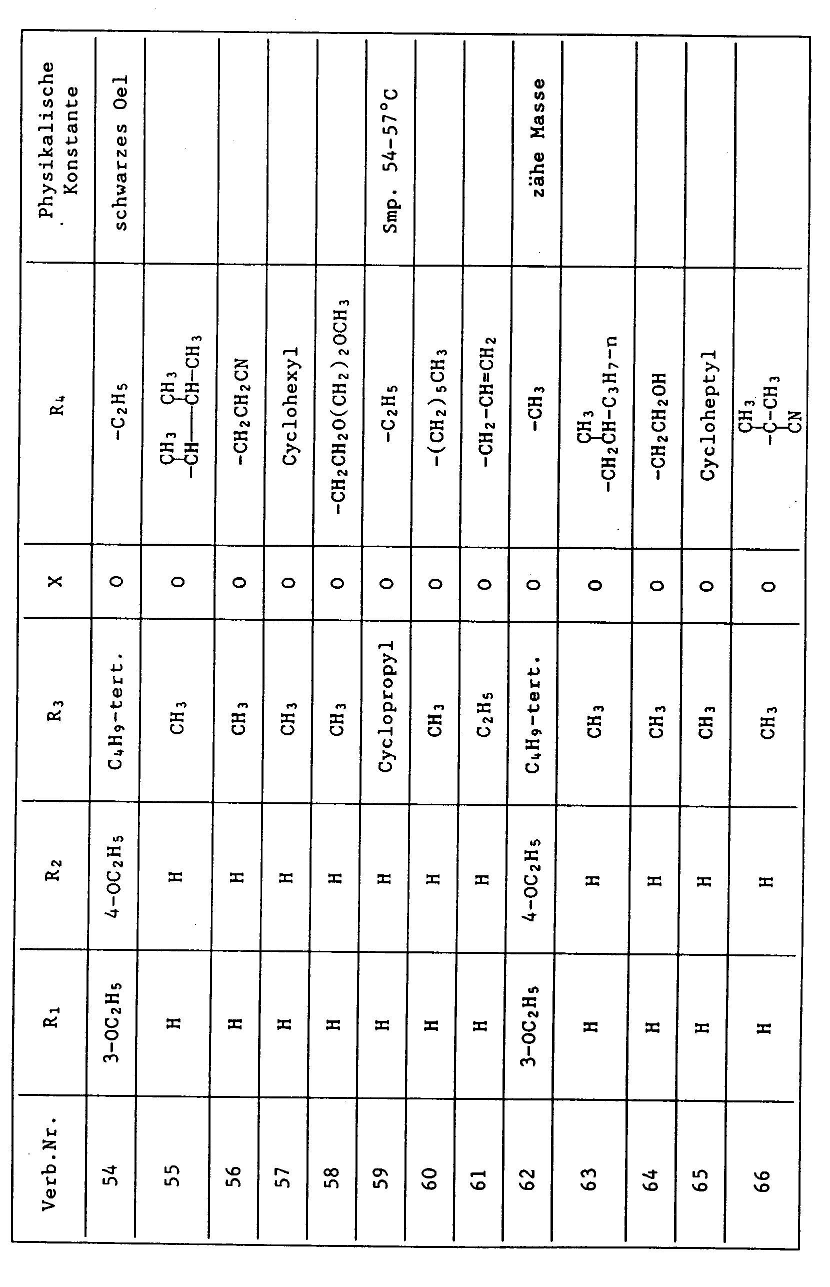

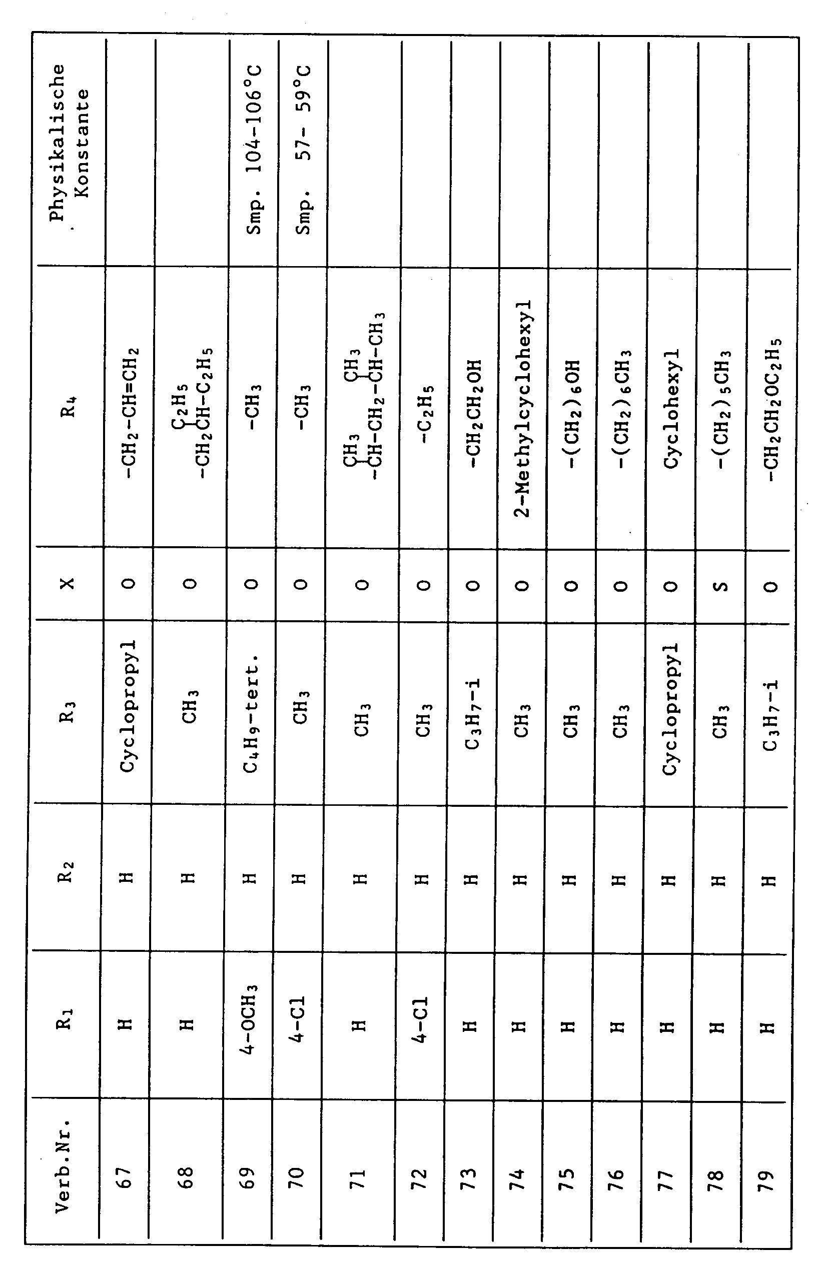

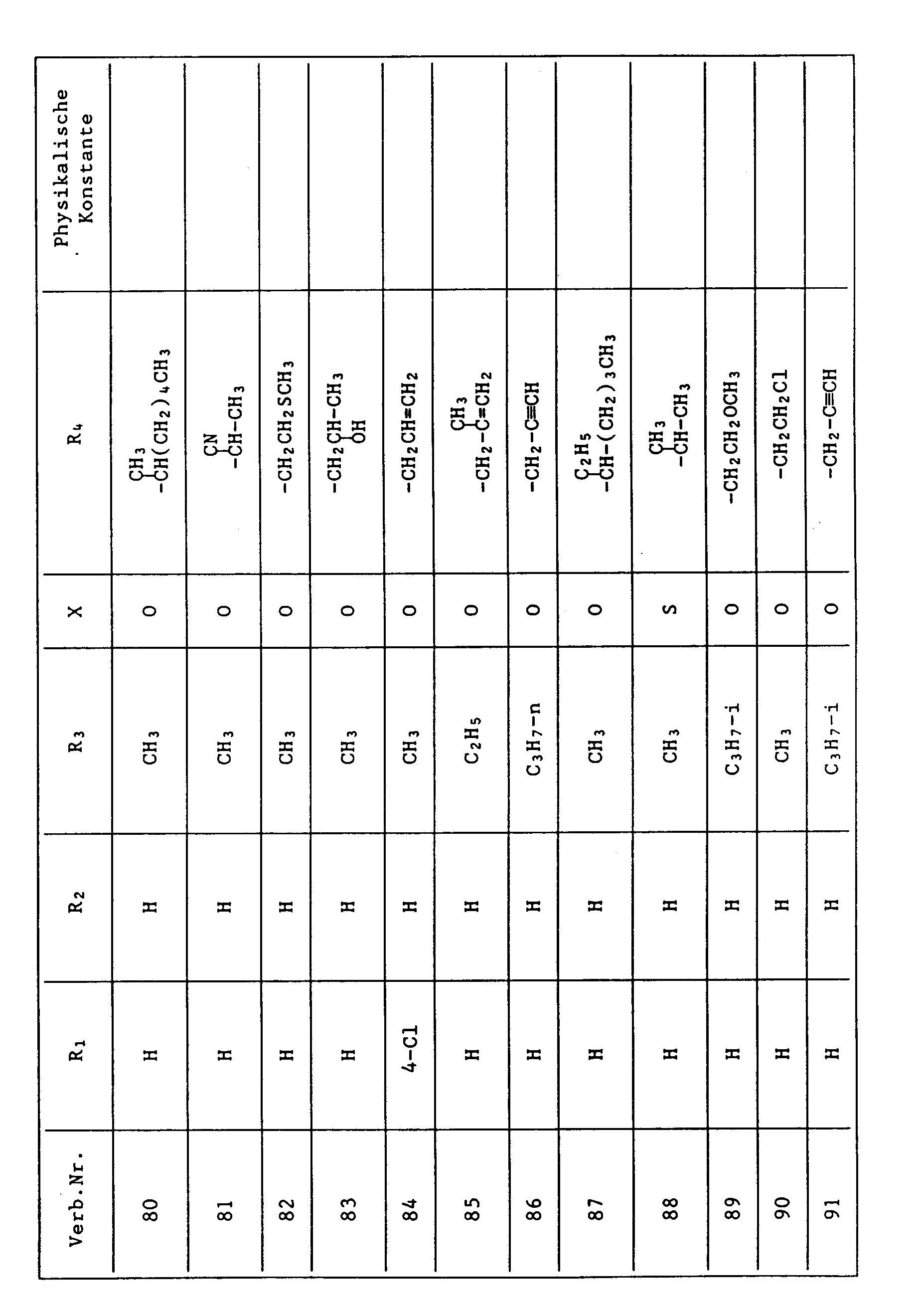

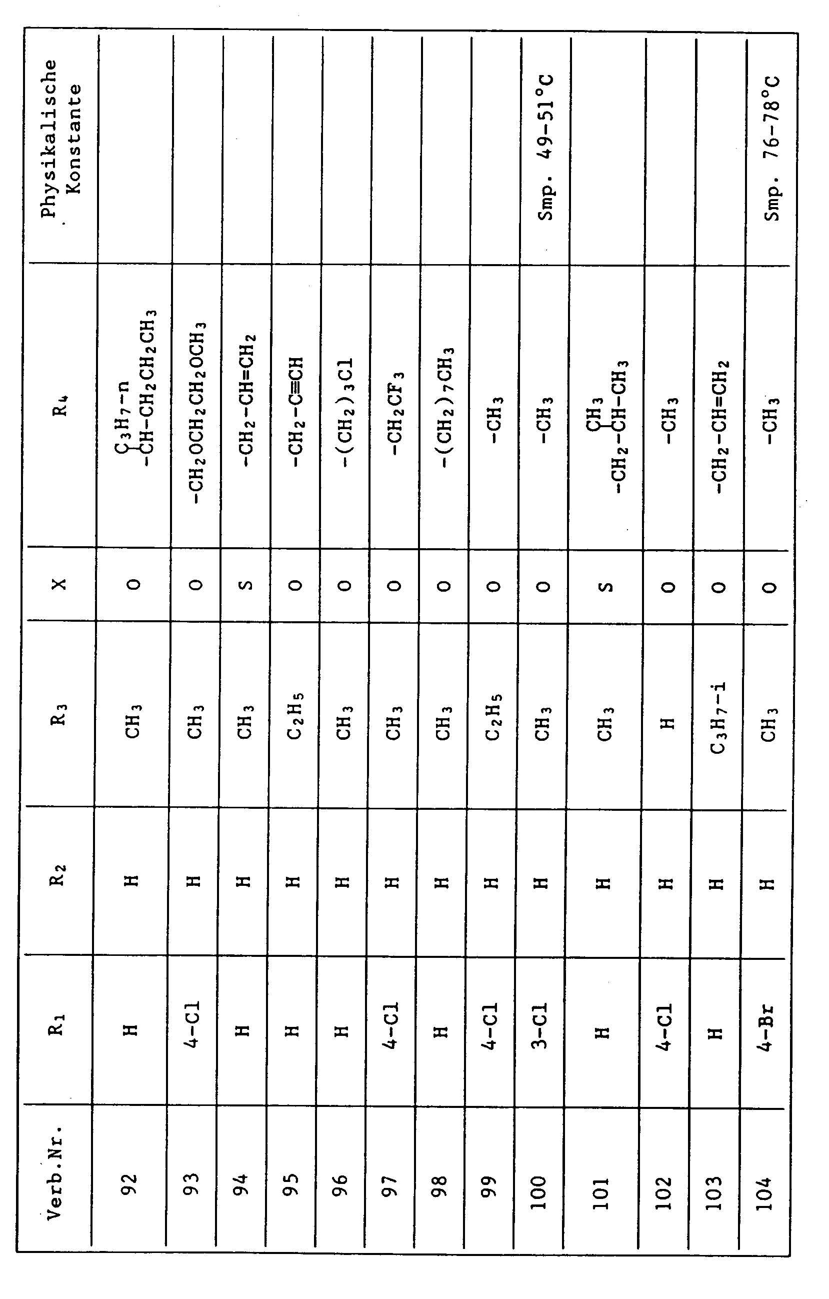

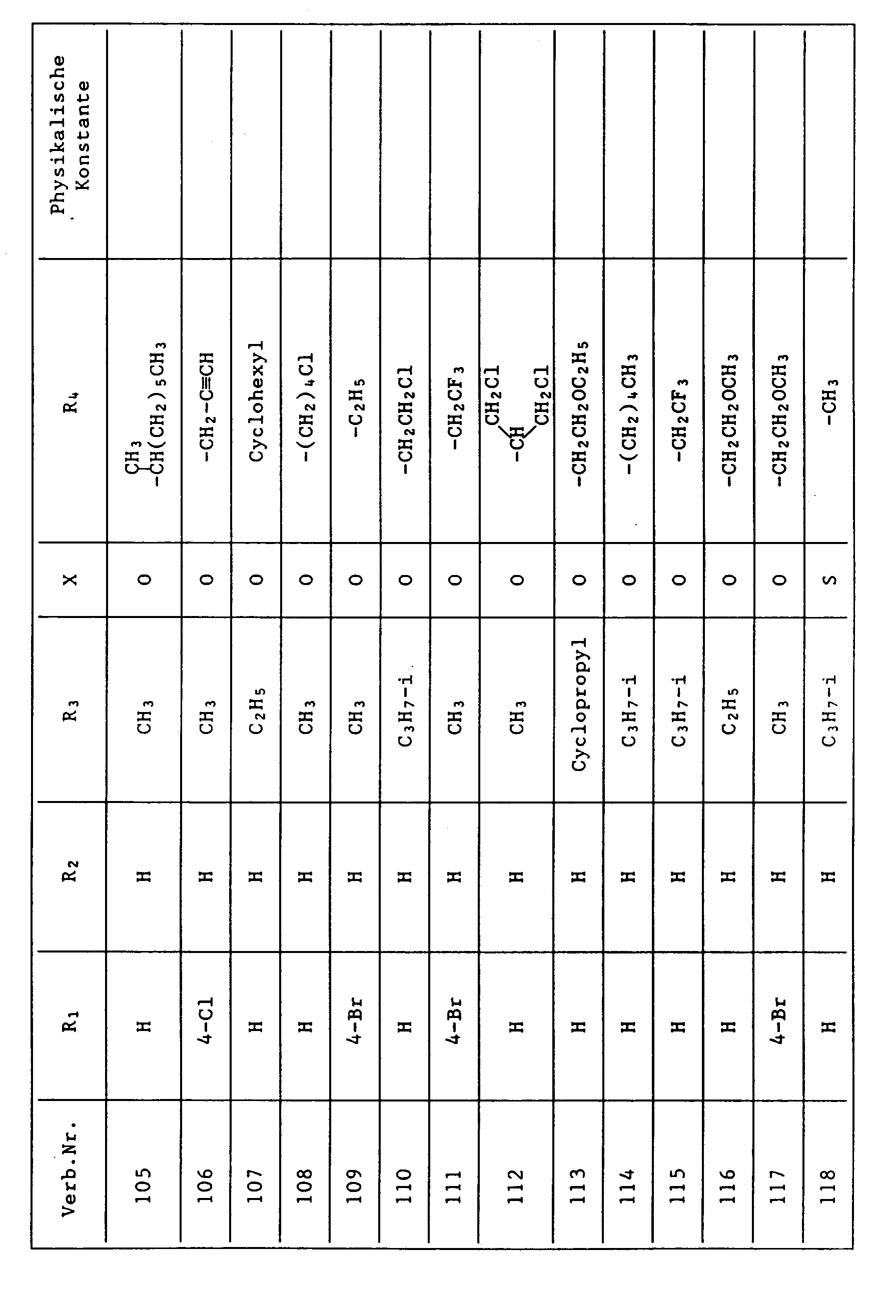

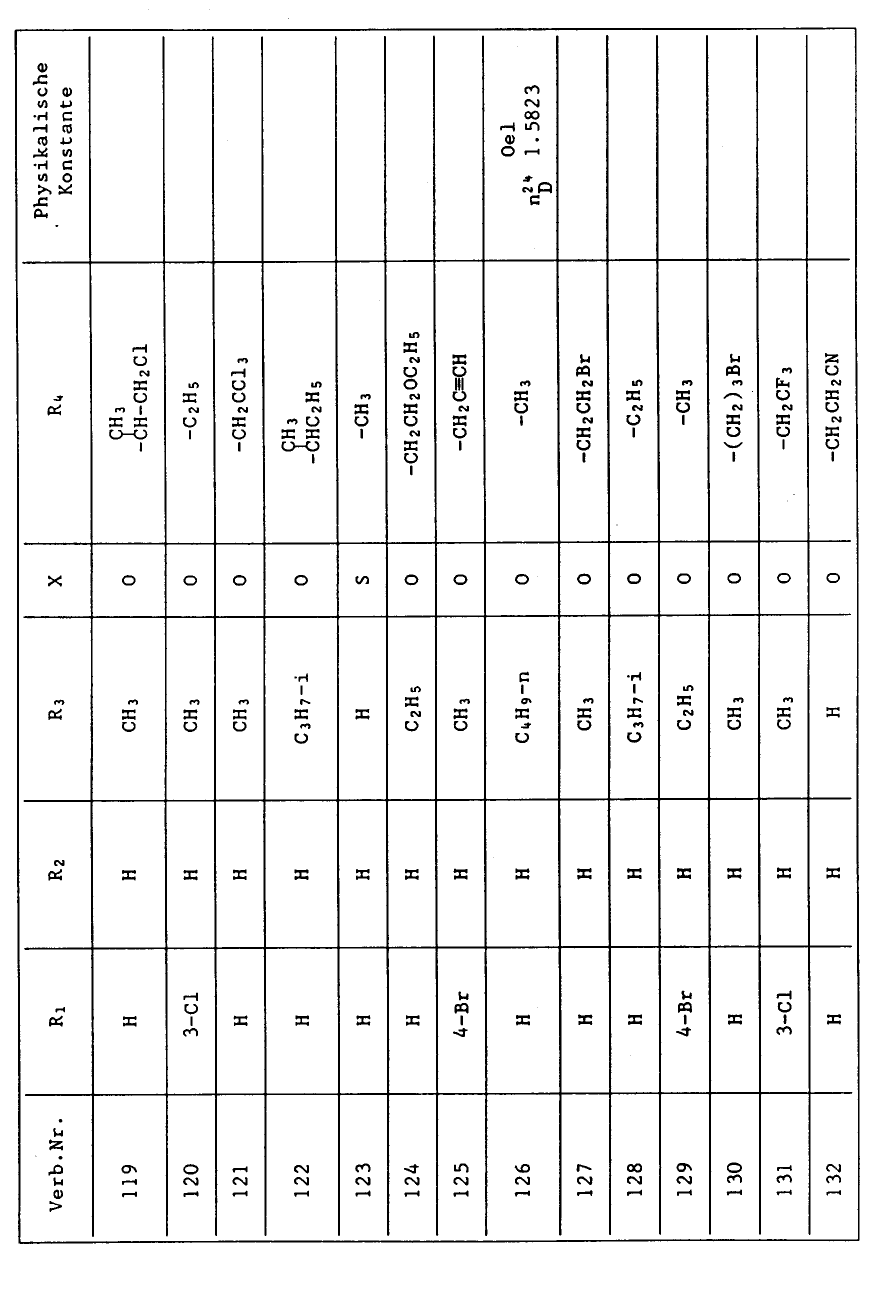

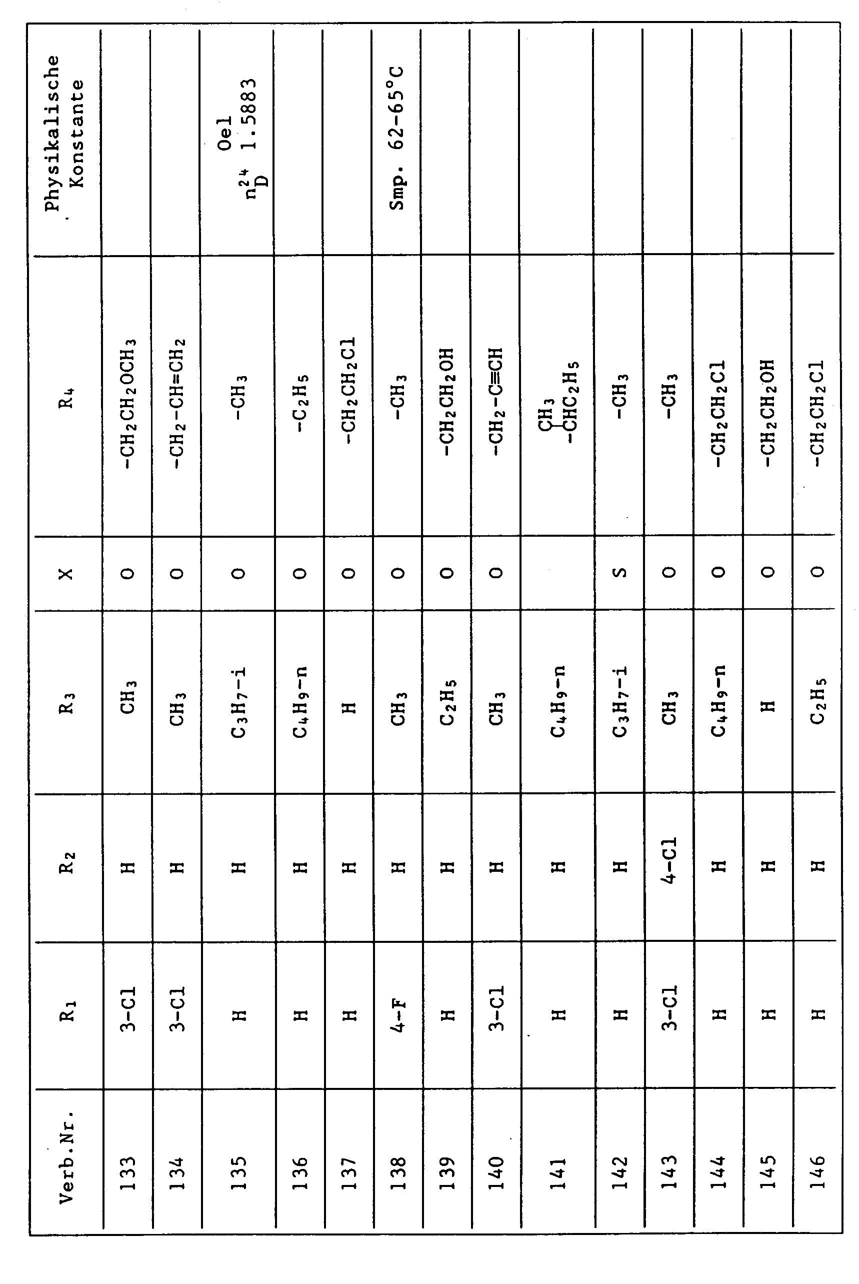

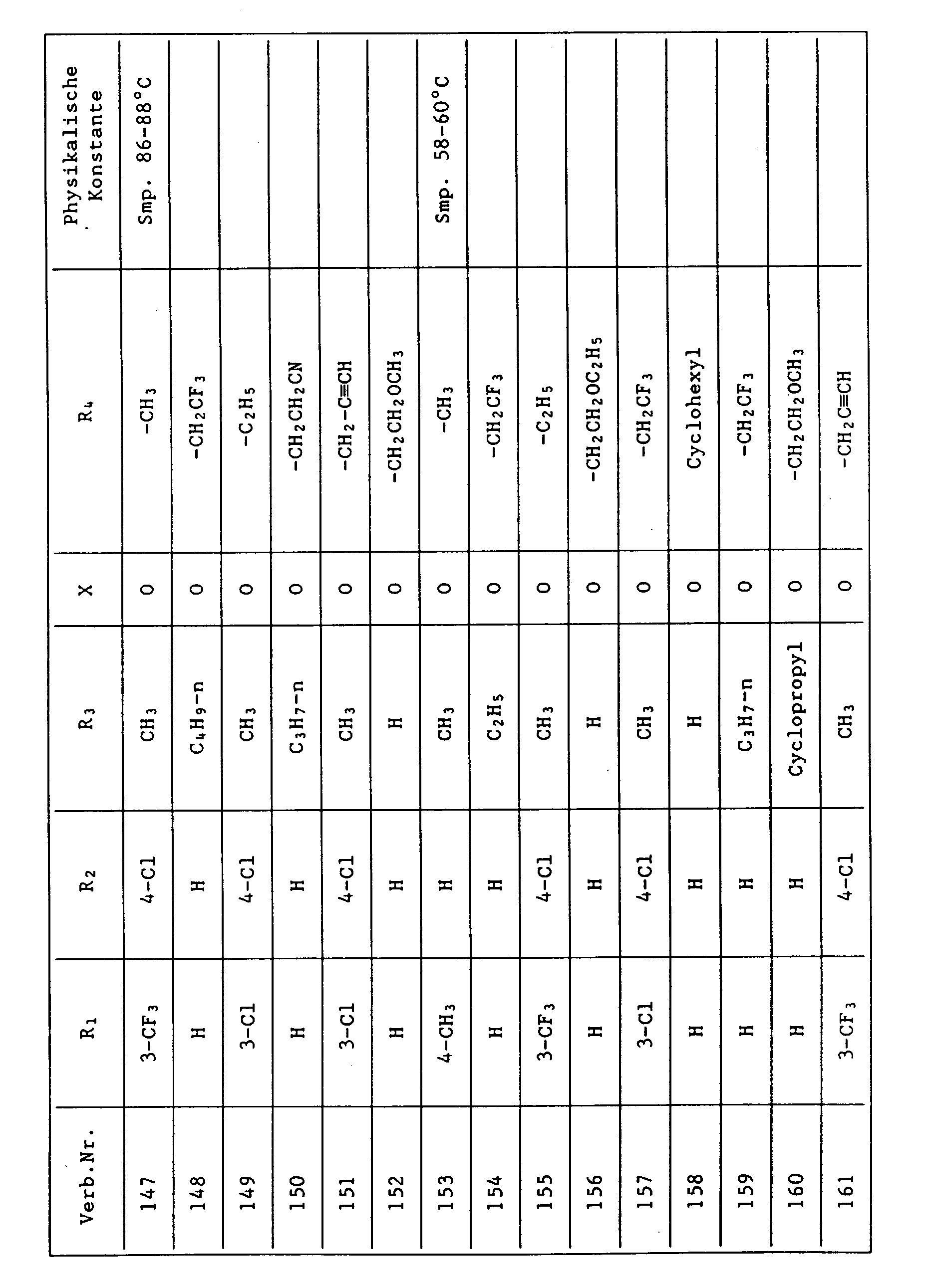

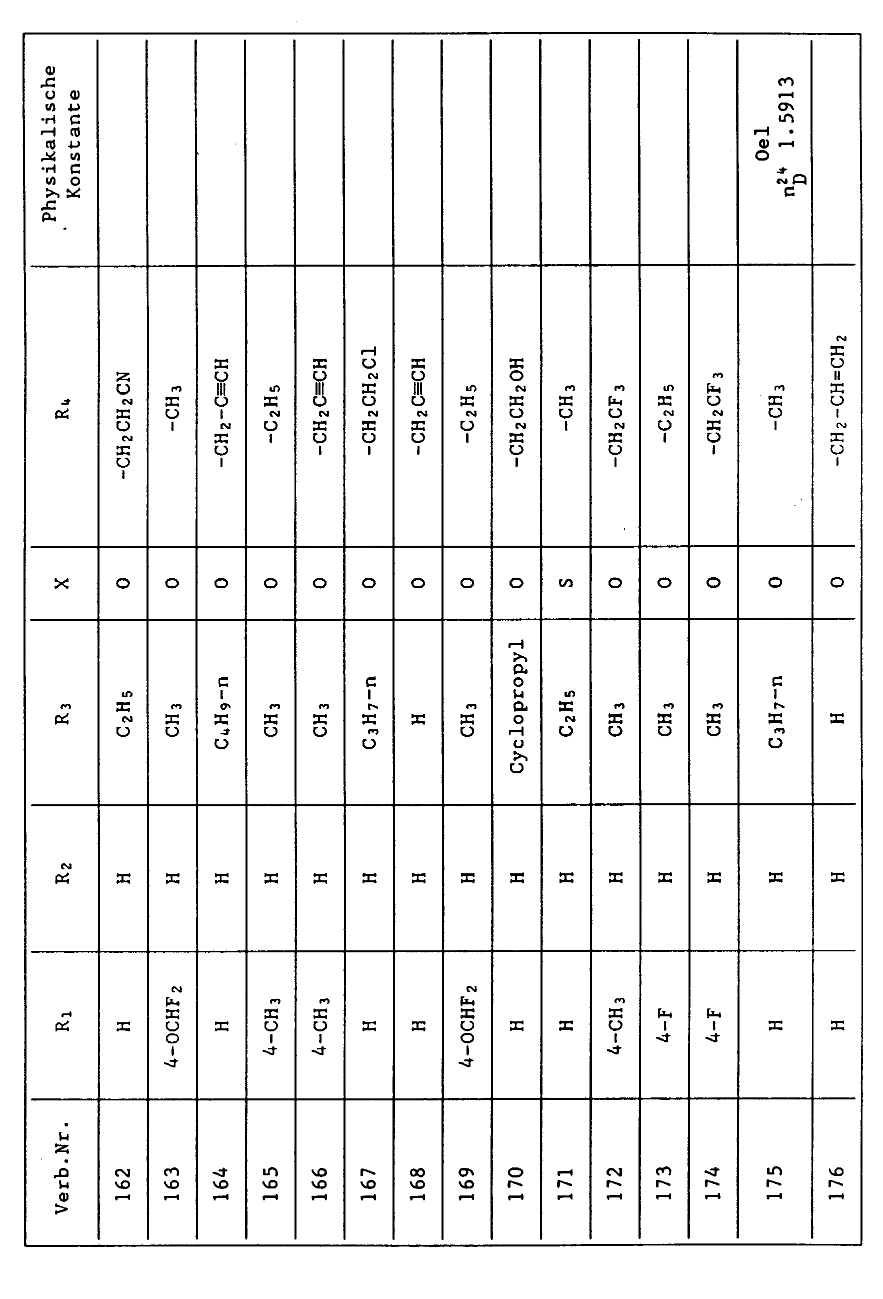

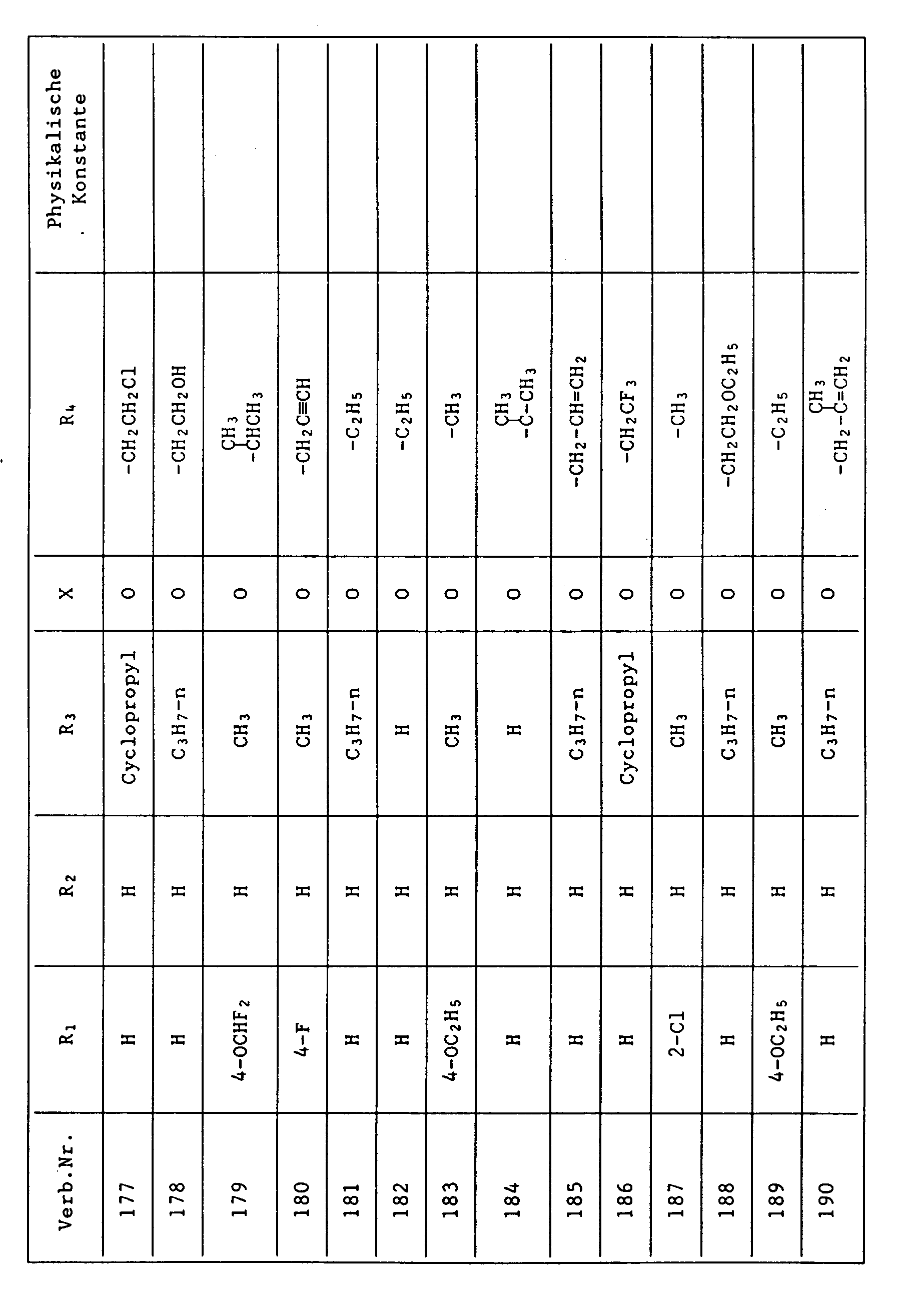

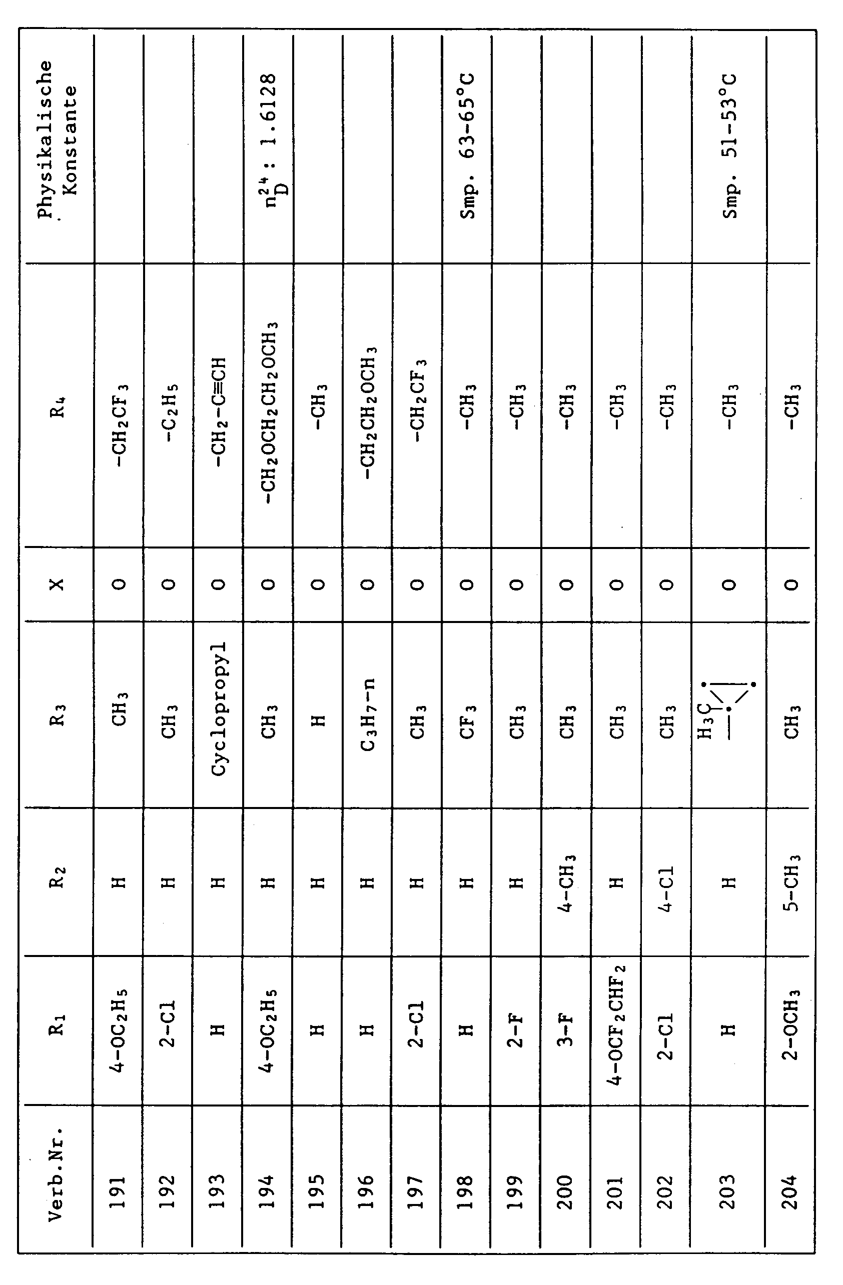

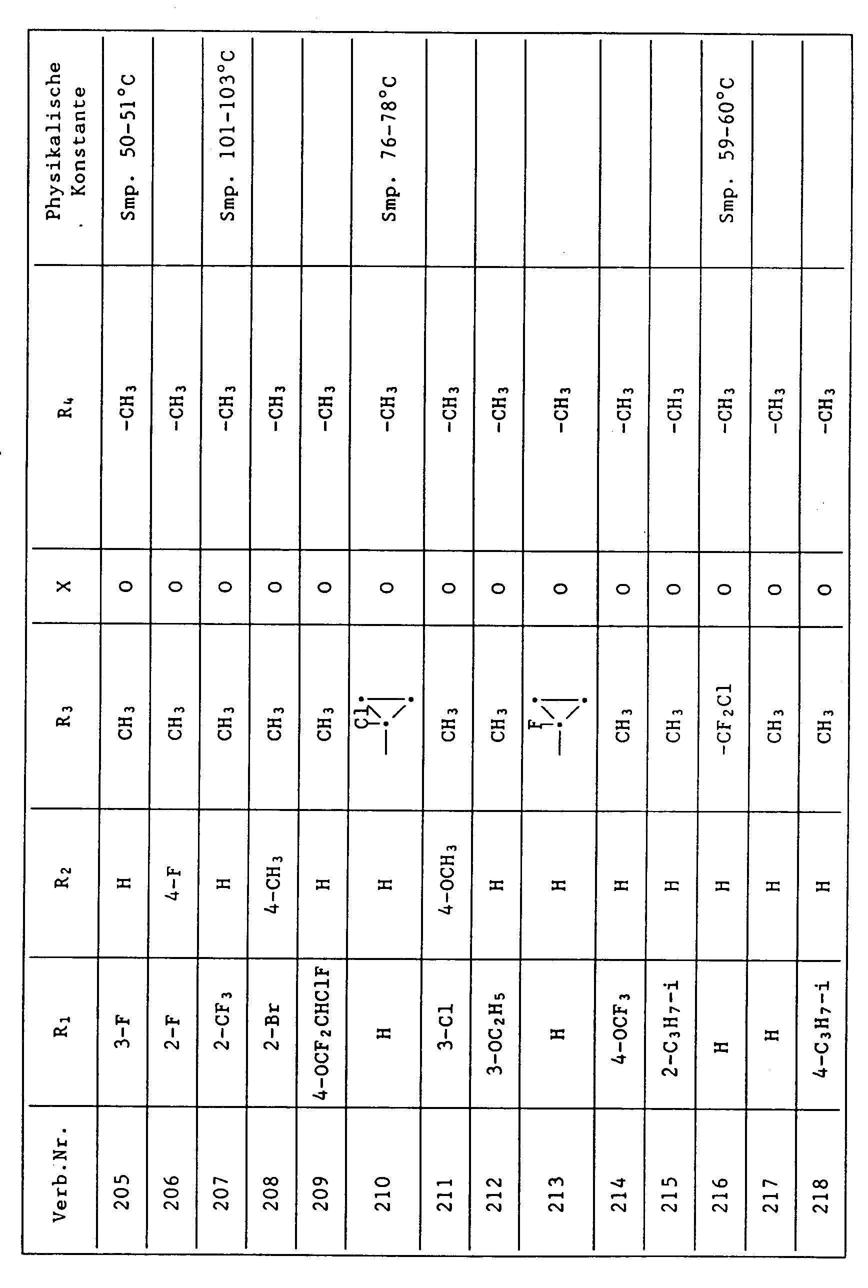

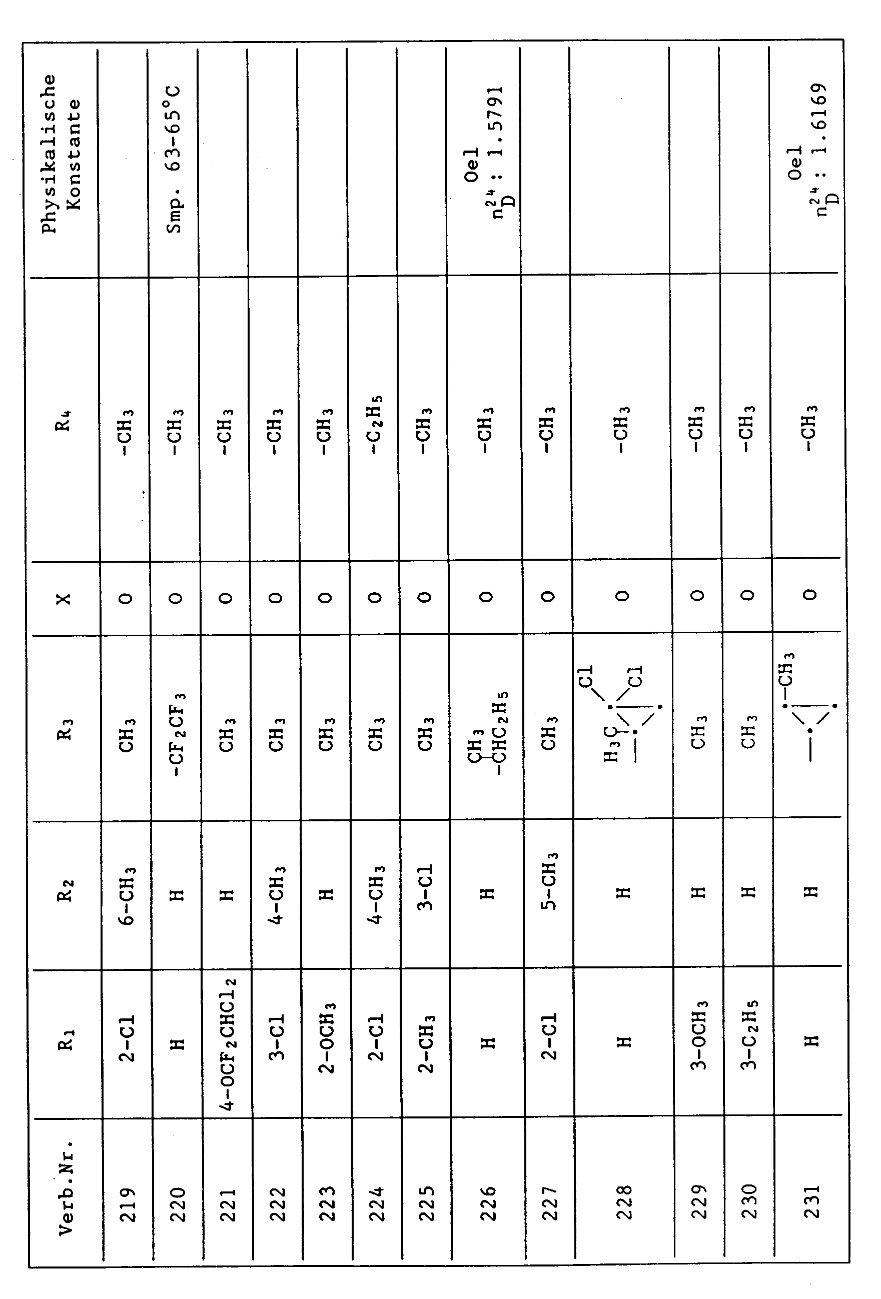

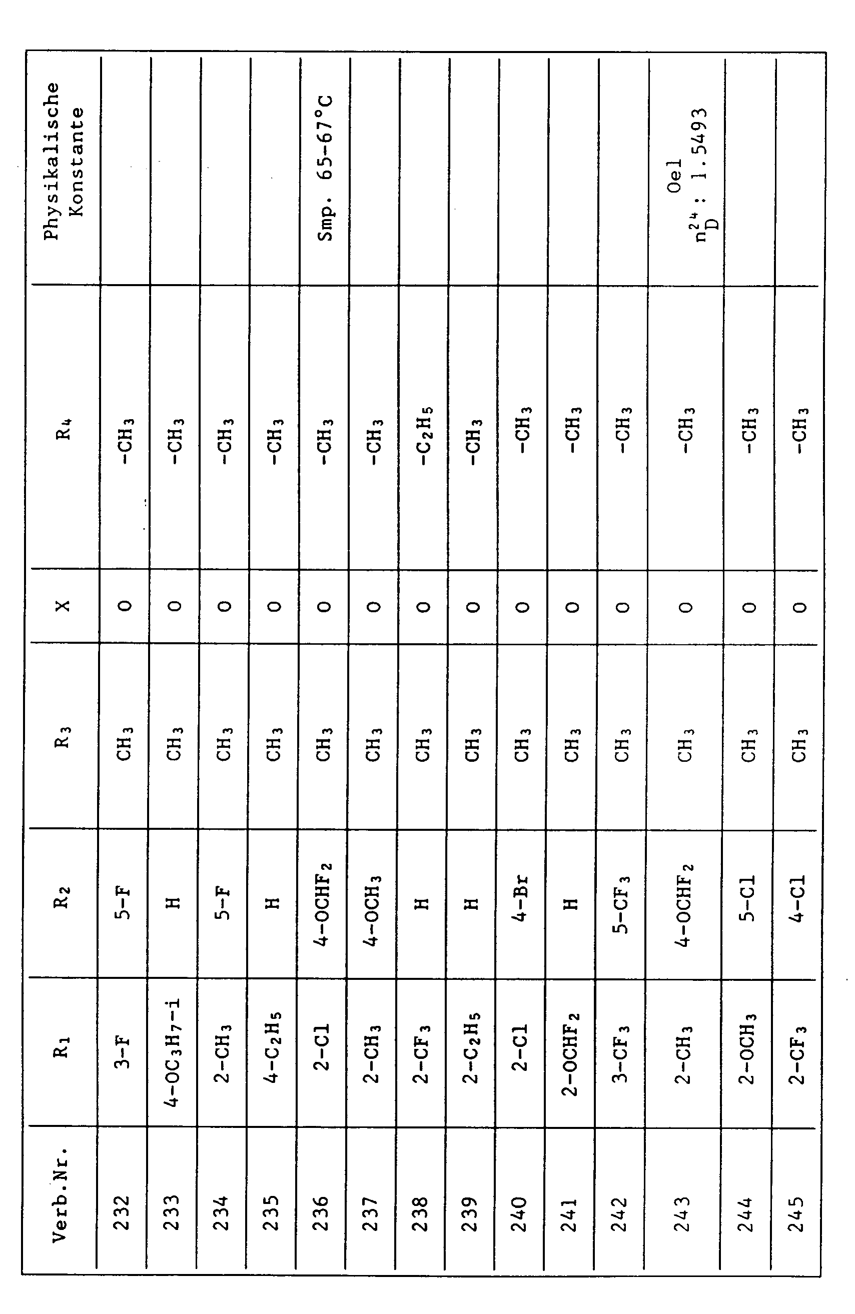

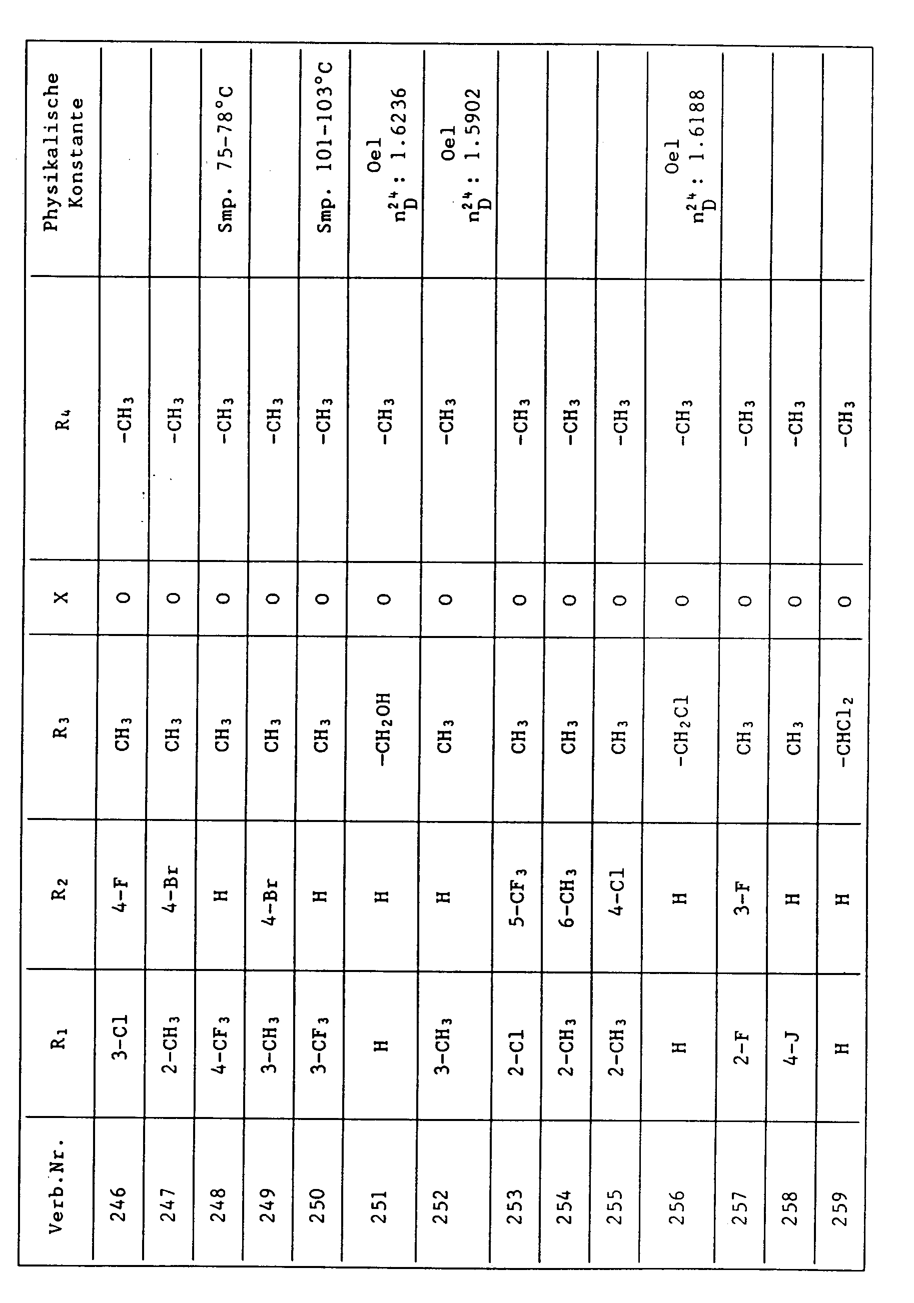

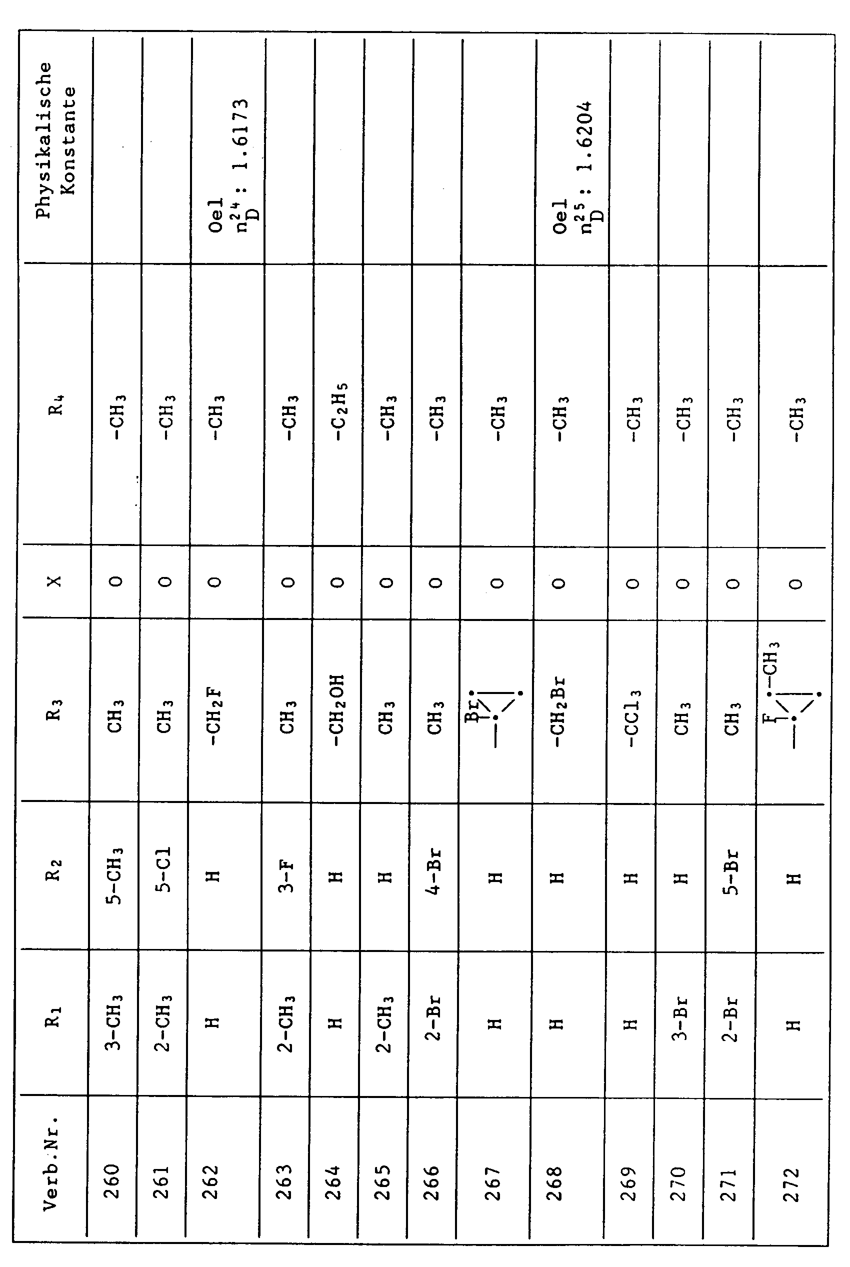

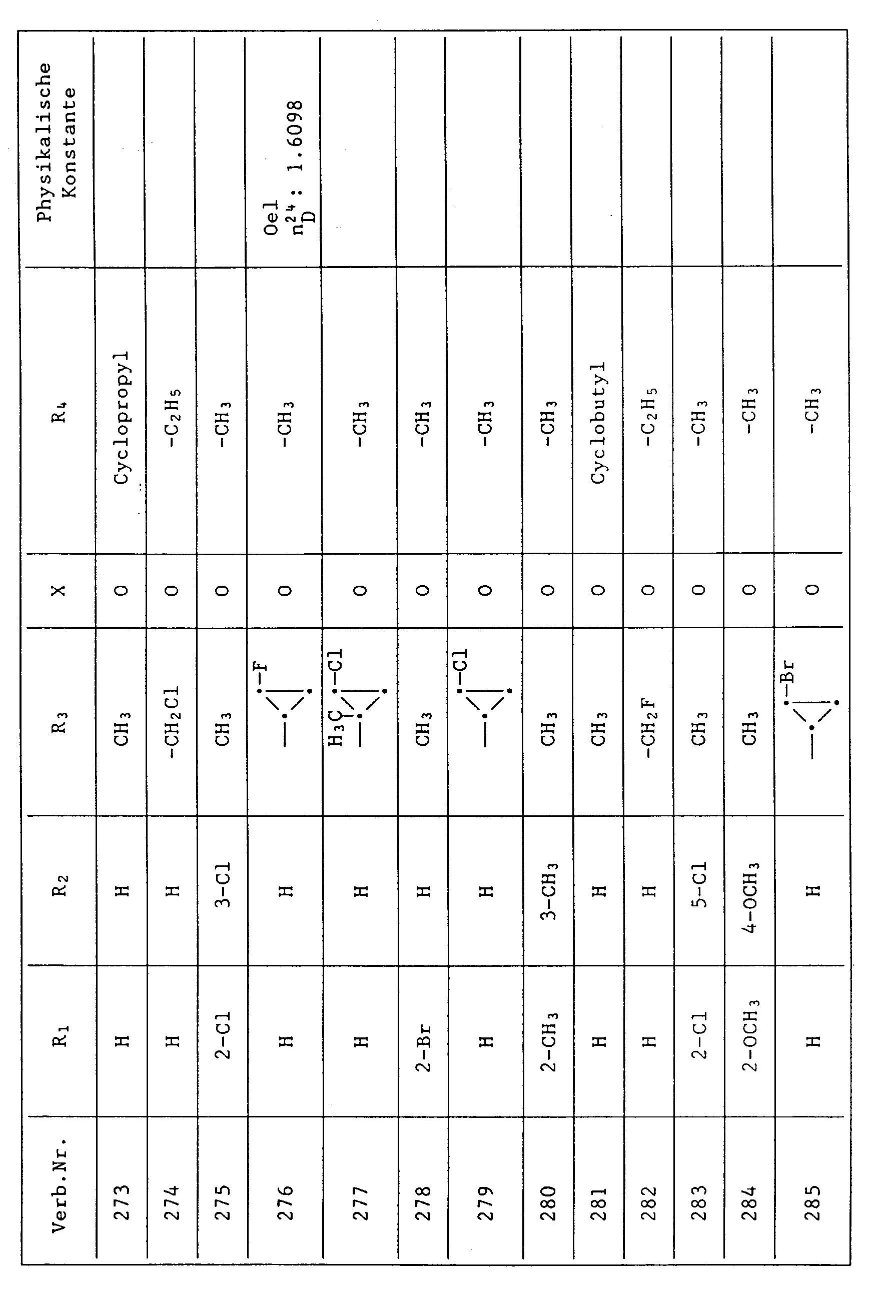

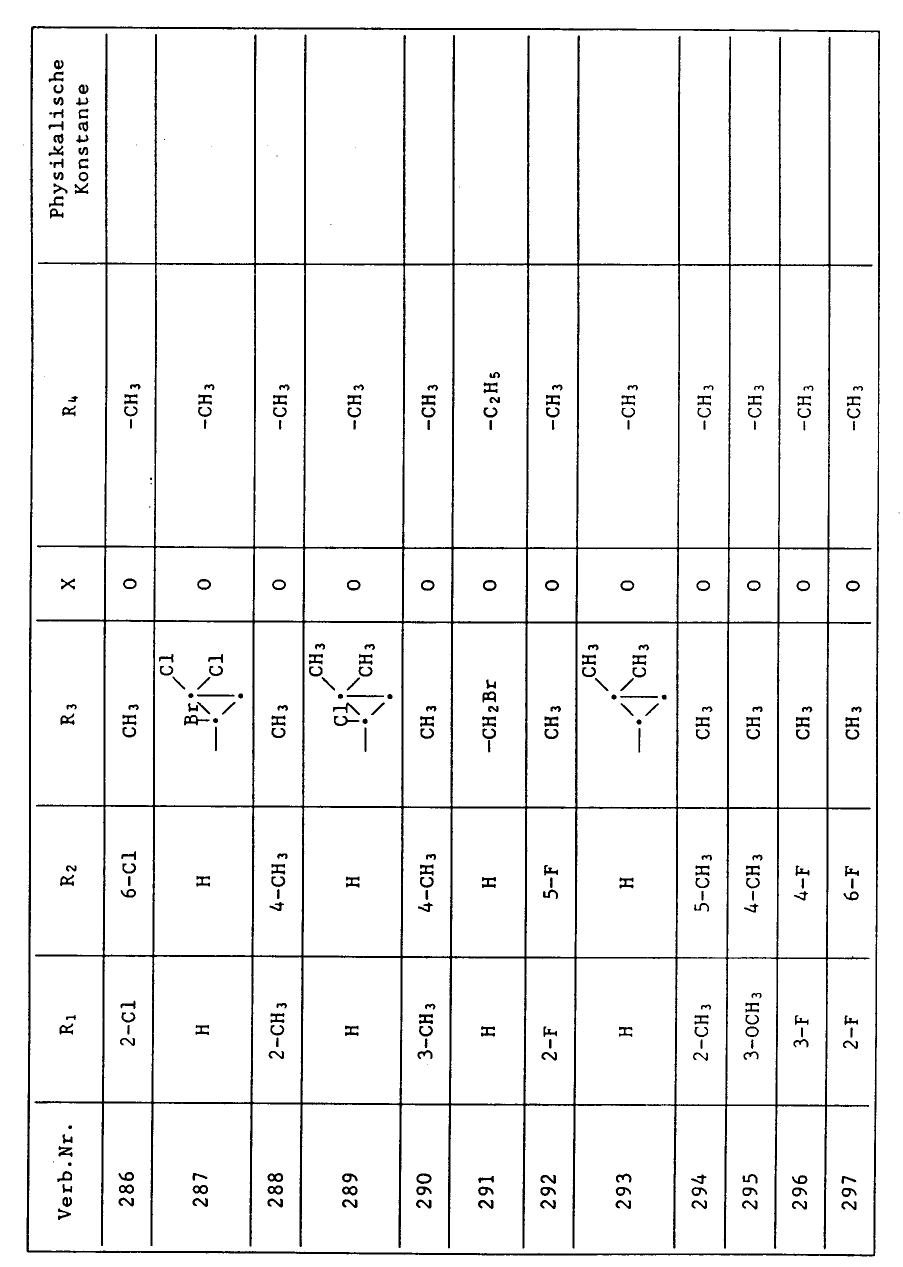

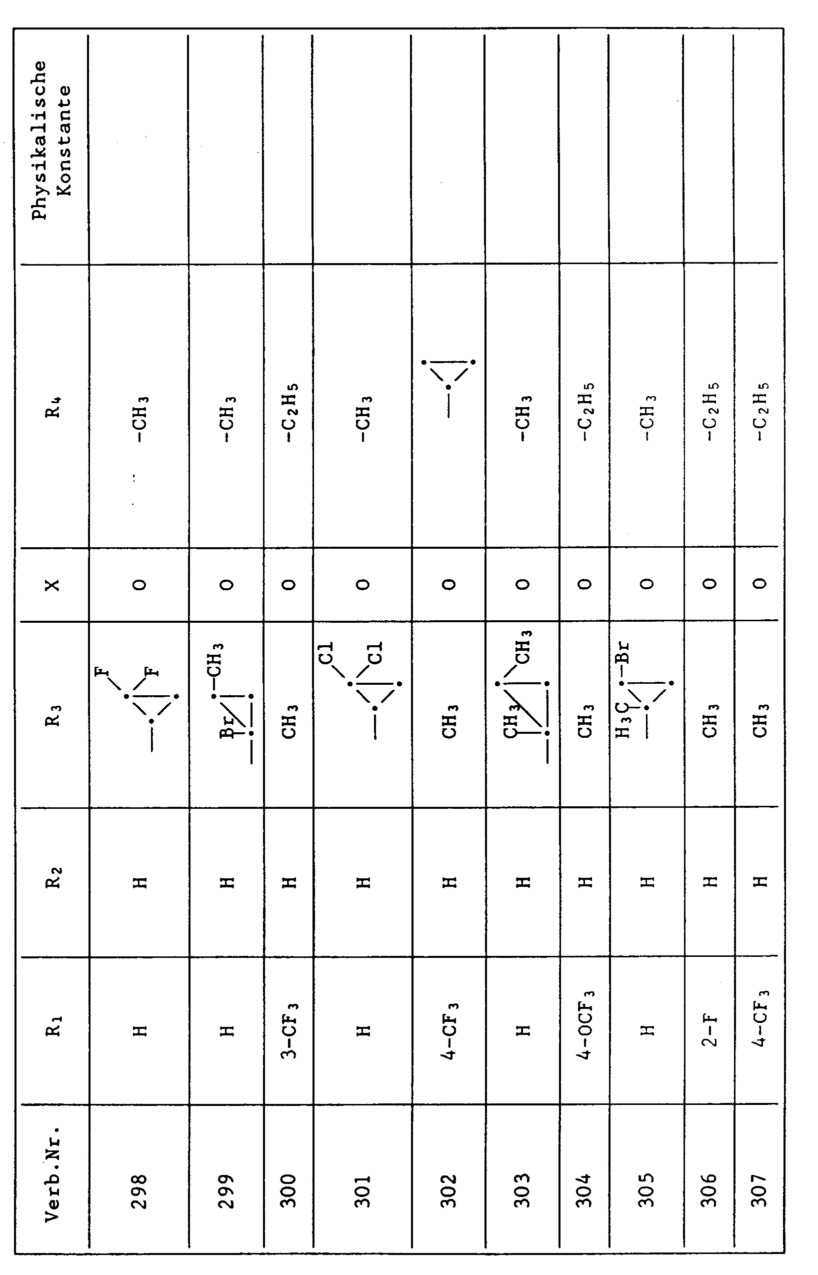

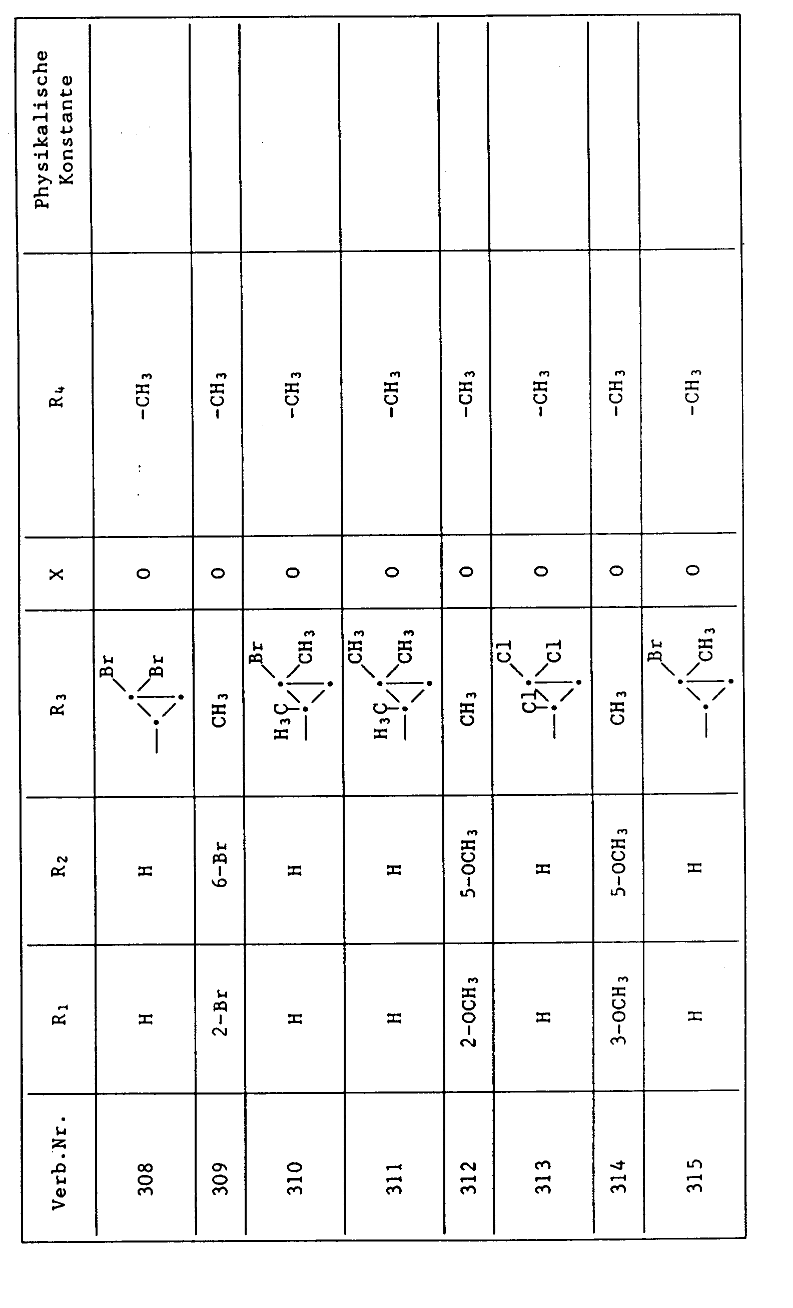

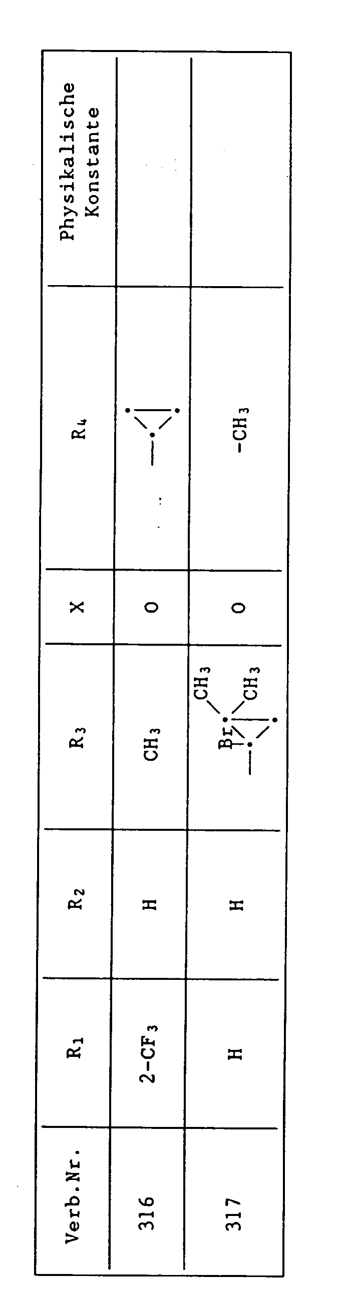

### 2. Formulierungsbeispiele für flüssige Wirkstoffe der Formel I % = Gewichtsprozent)

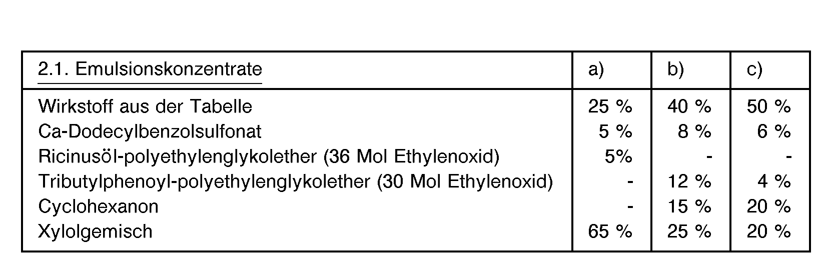

Aus solchen Konzentraten können durch Verdünnen mit Wasser Emulsionen jeder gewümschten Konzentration hergestellt werden.

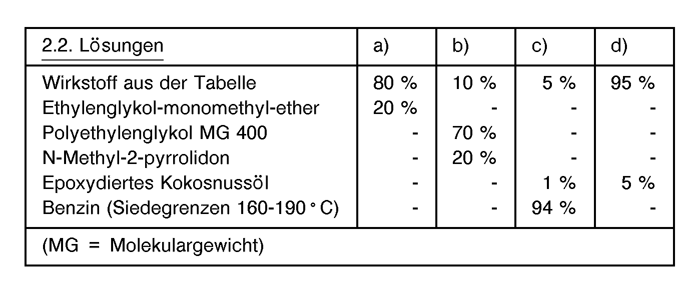

Die Lösungen sind zur Anwendung in Form kleinster Tropfen geeignet.

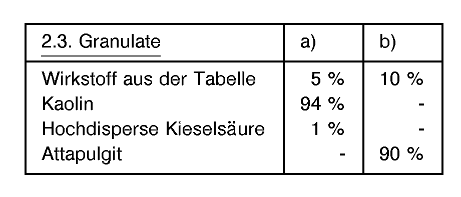

Der Wirkstoff wird in Methylenchlorid gelöst, auf den Träger aufgesprüht und das Lösungsmittel anschliessend im Vakuum abgedampft.

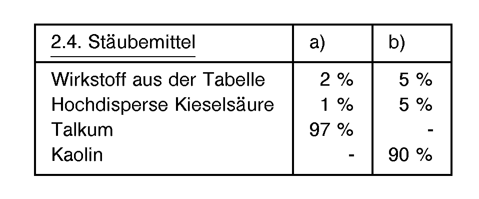

Durch inniges Vermischen auf Trägerstoffe mit dem Wirkstoff erhält man gebrauchsfertige Stäubemittel.

### Formulierungsbeispiele für feste Wirkstoffe der Formel 1 (% = Gewichtsprozent)

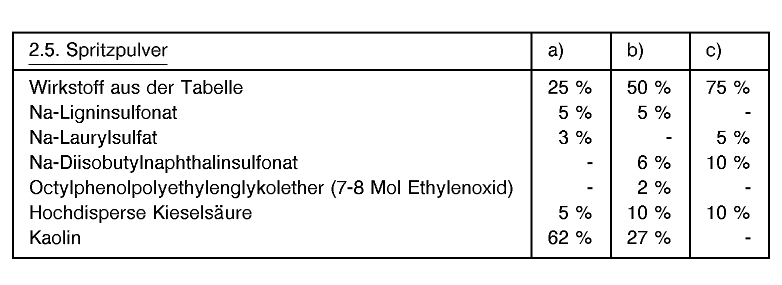

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

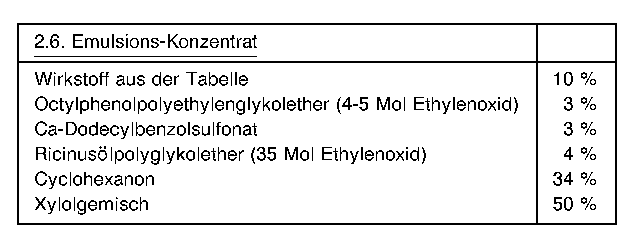

Aus diesem Konzentrat können durch Verdünnen mit Wasser Emulsionen jeder gewünschten Konzentration hergestellt werden.

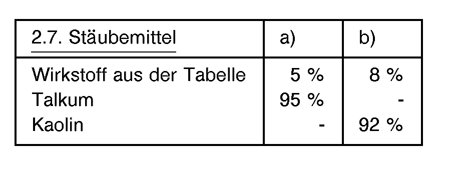

Man erhält anwendungsfertige Stäubemittel, indem der Wirkstoff mit dem Träger vermischt auf einer geeigneten Mühle vermahlen wird.

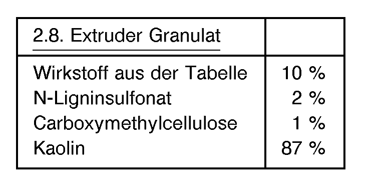

Der Wirkstoff wird mit den Zusatzstoffen vermischt, vermahlen und mit Wasser angefeuchtet. Dieses Gemisch wird extrudiert und anschliessend im Luftstrom getrocknet.

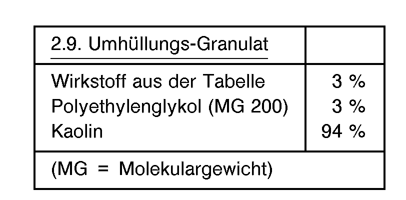

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

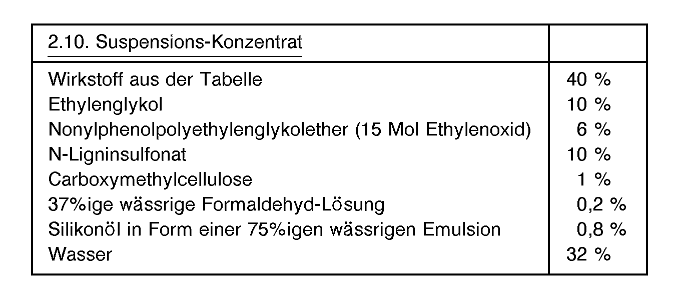

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### 3. Biologische Beispiele

### Beispiel 3.1: Wirkung gegen Puccinia graminis auf Weizen

### a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 _{°} C werden die infizierten Pflanzen in einem Gewächshaus bei ca. 22 _{°} C aufgestellt. Die Beurteilung der Rostpustelnentwicklung erfolgt 12 Tage nach der Infektion.

### b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 ° C werden die infizierten Pflanzen in einem Gewächshaus bei ca. 22 ° C aufgestellt. Die Beurteilung der Rostpustelnentwicklung erfolgt 12 Tage nach der Infektion.

Verbindungen aus der Tabelle zeigen gegen Puccinia gute Wirksamkeit (Befall: weniger als 20 %). Unbehandelte aber infizierte Kontrollpflanzen weisen dagegen einen Puccinia-Befall von 100 % auf.

### Beispiel 3.2: Wirkung gegen Phytophthora auf Tomatenpflanzen

### a) Residual-protektive Wirkung

Tomatenpflanzen wurden nach dreiwöchiger Anzucht mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

### b) Residual-kurative Wirkung

Tomatenpflanzen wurden nach dreiwöchiger Anzucht mit einer SporangienSuspension des Pilzes infiziert. Nach einer Inkubation von 22 Stunden in einer Feuchtkammer bei 90-100 % relativer Luftfeuchtigkeit und 20 _{°} C wurden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 5 Tage nach der Infektion.

### c) Systemische Wirkung

Zu Tomatenpflanzen wurde nach dreiwöchiger Anzucht eine aus Spritzpulver des Wirkstoffes hergestellte Spritzbrühe (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 _{°} C.

Verbindungen aus der Tabelle zeigen gegen Phytophthora gute Wirksamkeit (Befall: weniger als 20 %). Unbehandelte aber infizierte Kontrollpflanzen weisen dagegen einen Phytophthora-Befall von 100 % auf.

### Beispiel 3.3: Wirkung gegen Plasmopara viticola auf Reben

### Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20 _{°} C wird der Pilzbefall beurteilt.

Verbindungen aus der Tabelle zeigen gegen Plasmopara gute Wirksamkeit. Unbehandelte aber infizierte Kontrollpflanzen weisen dagegen einen Plasmopara-Befall von 100 % auf.

### Beispiel 3.4: Wirkung gegen Cercospora arachidicola auf Erdnusspflanzen

### Residual-protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21 _{°} C und hoher Luftfeuchtigkeit inkubiert und anschliessend bis zum Auftreten des typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Verbindungen aus der Tabelle zeigen gegen Cercospora gute Wirksamkeit (Befall: weniger als 20 %). Unbehandelte aber infizierte Kontrollpflanzen weisen dagegen einen Cercospora-Befall von 100 % auf.

### Beispiel 3.5: Wirkung gegen Venturia inaequalis auf Apfeltrieben

### Residual-protektive Wirkung

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24 _{°} C aufgestellt. Der Schorfbefall wird 15 Tage nach der Infektion beurteilt.

Verbindungen aus der Tabelle zeigen gegen Venturia gute Wirksamkeit; so reduzierten z.B. die Verbindungen Nr. 1, 10, 38, 59, 231, 262, 276 den Venturia-Befall auf weniger als 10 %. Unbehandelte aber infizierte Kontrollpflanzen weisen dagegen einen Venturia-Befall von 100 % auf.

### Beispiel 3.6: Wirkung gegen Botrytis cinerea an Apfelfrüchten

### Residual-protektive Wirkung

Künstlich verletzte Aepfel werden behandelt, indem eine aus Spritzpulver der Wirksubstanz hergestellte Spritzbrühe (0,002 % Aktivsubstanz) auf die Verletzungsstellen aufgepfropft wird. Die behandelten Früchte werden anschliessend mit einer Sporensuspension des Pilzes inokuliert und während einer Woche bei hoher Luftfeuchtigkeit und ca. 20 _{°} C inkubiert. Bei der Auswertung werden die angefaulten Verletzungsstellen gezählt und daraus die fungizide Wirkung der Testsubstanz abgeleitet.

Verbindungen aus der Tabelle zeigen gegen Botrytis gute Wirksamkeit (Befall: weniger als 20 %). So reduzierten z.B. die Verbindungen Nr. 1, 3, 7, 10, 38, 59, 231, 256, 262, 268, 276 den Botrytis-Befall auf 0 bis 5 %. Unbehandelte aber infizierte Kontrollpflanzen weisen dagegen einen Botrytis-Befall von 100 % auf.

### Beispiel 3.7: Wirkung gegen Erysiphae graminis auf Gerste

### Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22 _{°} C aufgestellt und der Pilzbefall nach 10 Tagen beurteilt.

Verbindungen aus der Tabelle zeigen gegen Erysiphae gute Wirksamkeit; so reduzierten z.B. die Verbindungen Nr. 1, 5, 6, 10, 38, 59, 135, 175, 231, 262 den Erysiphae-Befall auf weniger als 20 %. Unbehandelte aber infizierte Kontrollpflanzen weisen dagegen einen Erysiphae-Befall von 100 % auf.

### Beispiel 3.8: Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Die Verbindungen der Tabelle verhindern den Pilzbefall weitgehend (0 bis 10 %).

### Beispiel 3.9: Wirkung gegen Fusarium nivale

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen.

Bei den Körnern, die mit einem Spritzpulver behandelt worden sind, das als Wirkstoff eine Verbindung der Tabelle enthält, wurde die Entwicklung der Pilzkolonien fast vollständig unterdrückt (0 bis 5 %).

### Beispiel 3.10: Wirkung gegen Tilletia caries

Gerstenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Die Verbindungen der Tabelle verhindern den Pilzbefall weitgehend (0 bis 10 %).

### Beispiel 3.11: Wirkung gegen Colletotrichum lagenarium auf Gurken (Cucumis sativus L.)

Gurkenpflanzen werden nach 2-wöchiger Anzucht mit einer aus Spritzpulver des Wirkstoffs hergestellten Spritzbrühe (Konzentration 0,02 %) besprüht. Nach 2 Tagen werden die Pflanzen mit einer Sporensuspension (1,5 x 10⁵ Sporen/ml) des Pilzes infiziert und für 36 Stunden im Dunkeln bei 23 °C und hoher Luftfeuchtigkeit inkubiert. Die Inkubation wird dann bei normaler Luftfeuchtigkeit und ca. 22-23 _{°} C weitergeführt. Der eingetretene Pilzbefall wird 8 Tage nach der Infektion beurteilt. Unbehandelte aber infizierte Kontrollpflanzen weisen einen Pilzbefall von 100 % auf.

Verbindungen aus der Tabelle zeigen gute Wirksamkeit und verhindern die Ausbreitung des Krankheitsbefalls. Der Pilzbefall wird auf 20 % oder weniger zurückgedrängt.

### Beispiel 3.12: Wirkung gegen Septoria nodorum auf Weizen

### Residual-protektive Wirkung

7 Tage alte Weizenpflanzen werden mit einer aus der formulierten Substanz hergestellten Spritzbrühe (Konzentration 0,02 %) besprüht und 2 Tage später mit einer Konidiensuspension (400'000 Konidien/ml mit Zusatz von 0,1 % Tween 20 als Netzmittel) von Septoria nodorum infiziert. Nach einer 2-tägigen Inkubationsphase in einer Gewächshauskabine bei 20 ° C und 95-100 % rel. Luftfeuchtigkeit werden die Testpflanzen bis zum Versuchsende unbedeckt in einer Gewächshauskabine bei 21 _{°}C und 60 % rel. Luftfeuchtigkeit plaziert. Die Bonitierung des Pilzbefalls erfolgt 7 bis 10 Tage nach der Infektion.

Während unbehandelte, aber infizierte Kontrollpflanzen vollständig befallen sind, weisen Pflanzen, die mit Verbindungen aus der Tabelle behandelt werden, einen Pilzbefall von weniger als 20 % auf.

### Beispiel 3.13: Wirkung gegen Alternaria solani auf Tomaten

### Residual-protektive Wirkung

3 Wochen alte Tomatenpflanzen werden mit einer aus der formulierten Prüfsubstanz hergestellten Spritzbrühe (Konzentration 0,02 %) besprüht und 2 Tage später beidseitig mit einer Konidiensuspension (20'000 Konidien/ml) von Alternaria solani infiziert. Um ein Abwaschen des feintropfigen Sprühbelages zu verhindern, werden die infizierten Pflanzen in einer ersten 3-tägigen Inkubationsphase in einer Gewächshauskabine bei 20 _{°} C und Kulimatbefeuchtung mit engmaschigen Netzen abgedeckt. Anschliessend kommen die Pflanzen bis zum Versuchsende in eine Gewächshauskabine bei 24 °C. Die Bonitierung des Pilzbefalls erfolgt 4 Tage nach der Infektion.

Während unbehandelte, aber infizierte Kontrollpflanzen vollständig befallen sind, weisen Pflanzen, die mit Verbindungen aus der Tabelle behandelt werden, einen Pilzbefall von weniger als 20 % auf.

### Beispiel 3.14:

### a) Insektizide Kontakt-Wirkung gegen Nephotettix cincticeps und Nilaparvata lugens (Nymphen)

Der Test wird an wachsenden Reis-Pflanzen durchgeführt. Dazu werden jeweils 4 Pflanzen (14-20 Tage alt) mit einer Höhe von ca. 15 cm in Töpfe (Durchmesser 5,5 cm) eingepflanzt.

Die Pflanzen werden auf einem Drehteller mit 100 ml einer wässrigen Emulsions-Zubereitung enthaltend 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des Adultstadiums über 6 Tage an der behandelten Pflanze gehalten. Die Auswertung auf prozentuale Mortalität erfolgt 6 Tage nach der Besiedlung. Der Versuch wird bei etwa 27 °C, 60 % rel. Luftfeuchtigkeit und einer Beleuchtungsperiode von 16 Stunden durchgeführt.

### b) Systemische Insektizid-Wirkung gegen Nilaparvata lugens (in Wasser)

Etwa 10 Tage alte Reispflanzen (ca. 10 cm hoch) werden in einen Plastik-Becher eingestellt, der 150 ml einer wässrigen EmulsionsZubereitung des zu prüfenden Wirkstoffes in einer Konzentration von 100 ppm enthält und mit einem mehrfach durchlöcherten Plastikdeckel abgeschlossen ist. Die Wurzel der Reispflanze wird durch ein Loch in dem Plastikdeckel in die wässrige Test-Zubereitung geschoben. Dann wird die Reispflanze mit 20 Nymphen von Nilaparvata lugens im N 2 bis N 3 Stadium besiedelt und mit einem Plastikzylinder abgedeckt. Der Versuch wird bei ca. 26 ° C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen aus der Tabelle zeigen sowohl beim Kontaktversuch wie beim systemischen Versuch mehr als 80 % Wirkung gegen die genannten Reisschädlinge.

### Beschreibung für die Vertragsstaaten: CH, DE, FR, GB, IT, LI, NL

Die vorliegende Erfindung betrifft neue 2-Anilino-pyrimidin-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von Schädlingen, vor allem schädlichen Insekten und pflanzenschädigenden Mikroorganismen, vorzugsweise Pilzen.

Die erfindungsgemässen Verbindungen entsprechen der allgemeinen Formel I 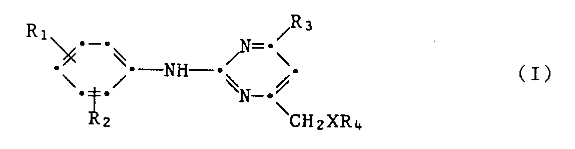 in welcher bedeuten:
R₁ und R₂ unabhängig voneinander Wasserstoff, Halogen, C1-C3-Alkyl, C1-C2-Halogenalkyl, C₁-C₃-Alkoxy oder C₁ -C₃ Halogenalkoxy;
R₃ Wasserstoff, C1-C4-Alkyl; durch Halogen oder Hydroxy substituiertes C1-C2-Alkyl, Cyclopropyl oder durch Halogen und/oder Methyl substituiertes Cyclopropyl;
R₄ C₁-C₈-Alkyl; durch Halogen, Hydroxy, Cyano, C₁-C₄-Alkoxy, C₃-C₄-Alkoxyalkoxy oder C₁-C₃-Alkylthio substituiertes C₂-C₆-Alkyl; C₃-C₆-Alkenyl; C₃-C₆-Alkinyl; C₃-C₇-Cycloalkyl oder durch Methyl substituiertes C₃-C₇-Cycloalkyl;
X Sauerstoff oder Schwefel; wobei, wenn R₁ Wasserstoff, R₂ Wasserstoff, Halogen, C₁-C₃-Alkyl, Cₗ-C₃-Alkoxy oder C₁-C₂-Halogenalkyl und
R₃ Halogen, C₁-C₄-Alkyl oder C₁-C₂-Halogenalkyl sind, R₄ im Falle X = Schwefel von Alkyl verschieden ist, und R₄ im Falle X = Sauerstoff von Alkyl, Alkenyl und Alkinyl verschieden ist..

Unter Alkyl selbst oder als Bestandteil eines anderen Substituenten, wie Halogenalkyl, Alkoxy oder Halogenalkoxy, sind je nach Anzahl der benannten Kohlenstoffatome beispielsweise zu verstehen: Methyl, Ethyl, Propyl, Butyl, Pentyl, Hexyl, Heptyl, Octyl sowie ihre Isomeren, wie z.B. Isopropyl, Isobutyl, tert.-Butyl, Isopentyl usw.. Halogen steht für Fluor, Chlor, Brom oder Jod. Halogenalkyl und Halogenalkoxy bezeichnen einfach bis perhalogenierte Reste, wie z.B. CHCI₂, CH₂F, CC1₃, CH₂CI, CHF₂, CH₂CH₂Br, C₂Cl₅, CHBr, CHBrCI usw., vorzugsweise CF₃. Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl. Cycloalkyl steht je nach Zahl der genannten Kohlenstoffatome z.B. für Cyclopropyl, Cyclobutyl, Cyclopentyl, Cyclohexyl oder Cycloheptyl.

Verbindungen mit einer N-Pyrimidylanilinstruktur sind bereits bekannt. So sind in der publizierten japanischen Patentanmeldung Sho 56-65804 sowie in der DDR-Patentschrift 151404 und in Chemical Abstracts, Bd. 103, Nr. 18, Juli 1985, 6301 r Verbindungen, die diese Grundstruktur besitzen, als wirksam gegen Pflanzenschädlinge beschrieben. Des weiteren sind aus EP-A-264 348, EP-A-135 472 und aus EP-A-172 786 2-Nitroanilinpyrimidinebekannt. Die bekannten Verbindungen konnten jedoch bisher die in der Praxis an sie gestellten Forderungen nicht in vollem Masse befriedigen. Die erfindungsgemässen Verbindungen der Formel I unterscheiden sich in charakteristischer Weise von den bekannten Verbindungen durch die Einführung spezifischer Substituenten und deren Kombination in die Anilinopyrimidin-Struktur, wodurch bei den neuen Verbindungen eine unerwartet hohe fungizide Wirksamkeit und insektizide Wirkung erreicht wird.

Für die eingangs benannten Vertragsstaaten ist die EP-B-270 111 im Sinne von Art. 54, Abs. 2 bis 4 , von Bedeutung. Darin werden als Pflanzenfungizide unter anderem 2-Anilinopyrimidine mit einer Struktur vorgeschlagen, wie sie durch den Disclaimer zur Formel I hierin gekennzeichnet ist.

Die Verbindungen der Formel 1 sind bei Raumtemperatur stabile Oele, Harze oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen.

Die erfindungsgemässen Wirkstoffe der Formel I zeichnen sich bei niedrigen Anwendungskonzentrationen nicht nur durch hervorragende insektizide und fungizide Wirkung, sondern auch durch besonders gute Pflanzenverträglichkeit aus.

Eine wichtige Gruppe von Pflanzenfungiziden sind jene der Formel I, bei denen R₁ und R₂ Wasserstoff bedeuten.

Eine besondere Gruppe von Anilino-pyrimidin-Derivaten stellen Verbindungen der Formel l dar, in denen
R₁ und R₂ unabhängig voneinander Wasserstoff, Halogen, C₁-C₃-Alkyl, C₁-C₂-Halogenalkyl, C₁-C₃-Alkoxy oder C₁ -C₃ Halogenalkoxy;
R₃ Wasserstoff, C₁-C₄-Alkyl oder Cyclopropyl; R₄ C₁-C₈-Alkyl; durch Halogen, Hydroxy, Cyano, C₁-C₄-Alkoxy, C3-C4-Alkoxyalkoxy, C₁-C₃-Alkylthio substituiertes C₂-C₆-Alkyl; C₃-C₆-Alkenyl; C₃-C₆-Alkinyl; C₅-C₇-Cycloalkyl oder durch Methyl substituiertes C₅-C₇-Cycloalkyl; und
X Sauerstoff oder Schwefel bedeuten.

Folgende Wirkstoffgruppen sind aufgrund ihrer ausgeprägten bioziden, insbesondere pflanzenfungiziden, Aktivität bevorzugt:

Gruppe 1 a: Verbindungen der Formel I, worin bedeuten:
R₁ und R₂ unabhängig voneinander Wasserstoff, Fluor, Chlor, Brom, C₁-C₃-Alkyl, C₁-C₂-Halogenalkyl, Ci-C₃-Alkoxy oder C₁-C₂-Halogenalkoxy;
R₃ Wasserstoff, C₁-C₃-Alkyl oder Cyclopropyl;
R₄ C₁-C₆-Alkyl; durch Halogen, Hydroxy, Cyano, C₁-C₃-Alkoxy, C₃-C₄-Alkoxyalkoxy oder C₁-C₂-Alkylthio substituiertes C₂-C₄-Alkyl; C₃-Alkenyl; C₃-Alkinyl oder unsubstituiertes oder durch Methyl substituiertes C₆-C₇-Cycloalkyl;
X Sauerstoff oder Schwefel.

Unter den vorstehend genannten Verbindungen stellen jene eine besonders bevorzugte Gruppe dar, bei denen R₁ = R₂ = Wasserstoff ist
(= Gruppe 1aa).

Gruppe 1 b: Verbindungen der Formel I, worin bedeuten:
R₁ und R₂ unabhängig voneinander Wasserstoff, Fluor, Chlor, Brom, C₁-C₃-Alkyl, C₁-C₃-Alkoxy, Trifluormethyl, -OCHF₂, -OCF₂CHF₂, -OCF₂CHCIF, -OCF₂CHCI₂ oder -OCF₂CCI₂F;
R₃ Wasserstoff, Methyl, Ethyl oder n-Propyl;
R₄ C₁-C₄-Alkyl; durch Halogen, Hydroxy, Cyano, C₁-C₂-Alkoxy oder C₁-C₂-Alkylthio substituiertes C₂-C₃-Alkyl; C₃-C₆-Alkenyl; C₃-C₆-Alkinyl oder Cyclohexyl;
X Sauerstoff oder Schwefel.

Unter den vorstehend genannten Verbindungen stellen jene eine besonders bevorzugte Gruppe dar, bei denen R₁ = R₂ = Wasserstoff ist (= Gruppe 1bb).

Gruppe 1 c: Verbindungen der Formel I, worin bedeuten:
R₁ und R₂ unabhängig voneinander Wasserstoff, Fluor, Chlor, Brom, C₁-C₂-Alkyl, C₁-C₂-Alkoxy, Trifluormethyl, -OCHF₂, -OCF₃, -OCF₂CHF₂ oder -OCF₂CHCIF;
R₃ Wasserstoff oder C₁-C₂-Alkyl;
R₄ C₁-C₃-Alkyl; durch Halogen, Hydroxy, Cyano, C₁-C₂-Alkoxy oder C₁-C₂-Alkylthio substituiertes C₂-Alkyl; C₃-C₄-Alkenyl; C₃-C₄-Alkinyl;
X Sauerstoff oder Schwefel.

Unter den vorstehend genannten Verbindungen stellen jene eine besonders bevorzugte Gruppe dar, bei denen R₁ = R₂ = Wasserstoff ist und X Sauerstoff bedeutet (= Gruppe 1cc).

Gruppe 1 d: Verbindungen der Formel I, worin bedeuten:
R₁ und R₂ unabhängig voneinander Wasserstoff, Fluor, Chlor, Brom, C₁-C₂-Alkyl, C₁-C₂-Alkoxy, Trifluormethyl oder -OCHF₂;
R₃ Methyl;
R₄ C₁-C₂-Alkyl; durch Fluor, Chlor, Brom, Cyano, C₁-C₂ -Alkoxy oder Methylthio substituiertes C₂-Alkyl; Allyl oder Propargyl;
X Sauerstoff oder Schwefel.

Unter den vorstehend genannten Verbindungen stellen jene eine besonders bevorzugte Gruppe dar, bei denen R₁ = R₂ = Wasserstoff ist und X Sauerstoff bedeutet (= Gruppe 1dd).

Gruppe 2a: Verbindungen der Formel I, worin bedeuten:
R₁ und R₂ unabhängig voneinander Wasserstoff, Halogen, C₁-C₂-Alkyl, Halogenmethyl, C₁-C₂-Alkoxy oder C₁ -C₂-Halogenalkoxy;
R₃ Wasserstoff, C₁-C₃-Alkyl; durch Halogen oder Hydroxy substituiertes C₁-C₂-Alkyl; Cyclopropyl oder durch Halogen und/oder Methyl ein- bis dreifach gleich oder verschieden substituiertes Cyclopropyl;
R₄ C₁-C₄-Alkyl; durch Halogen, Cyano, C1-C2-Alkoxy oder C₁-C₂-Alkylthio substituiertes C₂-C₃-Alkyl; C₃-C₆-Alkenyl; C₃-C₆-Alkinyl oder C₃-C₇-Cycloalkyl;
X Sauerstoff oder Schwefel.

Unter den vorstehend genannten Verbindungen stellen jene eine besonders bevorzugte Gruppe dar, bei denen R₁ = R₂ = Wasserstoff ist
(= Gruppe 2aa).

Gruppe 2b: Verbindungen der Formel I, worin bedeuten:
R₁ und R₂ unabhängig voneinander Wasserstoff, Fluor, Chlor, Brom, Methyl, Trifluormethyl, Methoxy oder Difluormethoxy;
R₃ Wasserstoff, C₁-C₃-Alkyl; durch Halogen oder Hydroxy substituiertes C₁-C₂-Alkyl; Cyclopropyl, oder durch Halogen und/oder Methyl ein- bis dreifach gleich oder verschieden substituiertes Cyclopropyl;
R₄ C₁-C₃-Alkyl; durch Fluor, Chlor oder C₁-C₂-Alkoxy substituiertes C₂-C₃-Alkyl; C₃-C₆-Alkenyl; C₃-C₆-Alkinyl oder C₃-C₆-Cycloalkyl;
X Sauerstoff oder Schwefel.

Unter den vorstehend genannten Verbindungen stellen jene eine besonders bevorzugte Gruppe dar, bei denen R₁ = R₂ = Wasserstoff ist
(= Gruppe 2bb).

Gruppe 2c: Verbindungen der Formel I, worin bedeuten:
R₁ und R₂ unabhängig voneinander Wasserstoff, Fluor, Chlor, Methyl, Trifluormethyl, Methoxy oder Difluormethoxy;
R₃ C₁-C₃-Alkyl; durch Halogen oder Hydroxy substituiertes Methyl; Cyclopropyl oder durch Halogen oder Methyl ein- bis dreifach substituiertes Cyclopropyl;
R₄ C₁-C₃-Alkyl; durch Fluor, Chlor oder Methoxy substituiertes C₂-C₃-Alkyl; C₃-C₄-Alkenyl; C₃-C₄-Alkinyl; Cyclopropyl; Cyclohexyl;
X Sauerstoff.

Unter den vorstehend genannten Verbindungen stellen jene eine besonders bevorzugte Gruppe dar, bei denen R₁ = R₂ = Wasserstoff ist
(= Gruppe 2cc).

Gruppe 2d: Verbindungen der Formel I, worin bedeuten:
R₁ und R₂ Wasserstoff;
R₃ C₁-C₃-Alkyl; durch Fluor, Chlor oder Brom substituiertes Methyl; Cyclopropyl oder durch Chlor oder Methyl substituiertes Cyclopropyl;
R₄ C₁-C₃-Alkyl, 2-Chlorethyl, 2,2,2-Trifluorethyl, Allyl oder Propargyl;
X Sauerstoff.

Eine bevorzugte Einzelsubstanz ist z.B.:
2-Phenylamino-4-cyclopropyl-6-methoxymethyl-pyrimidin (Verb.Nr.10).

Die Verbindungen der Formel I werden hergestellt, indem man
1. ein Phenylguanidinsalz der Formel Ila 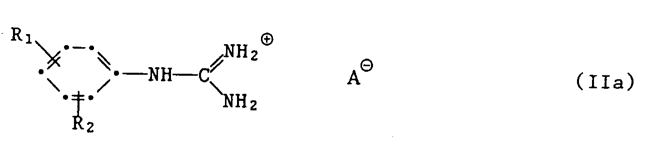 oder das zugrundeliegende Guanidin der Formel Ilb 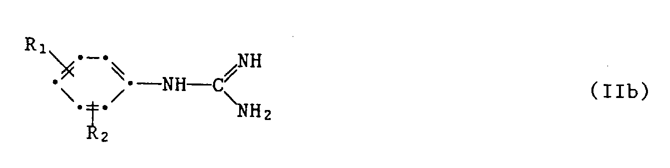 mit einem Diketon der Formel lll 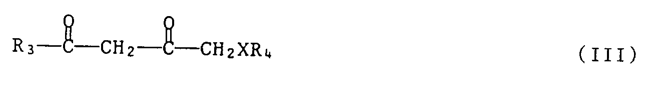 ohne Lösungsmittel oder in einem aprotischen, bevorzugt aber in einem protischen Lösungsmittel bei Temperaturen von 60 ° C bis 160 ° C, bevorzugt 60 ° C bis 110 ° C, umsetzt oder
2. in einem mehrstufigen Verfahren:
   2.1 Harnstoff der Formel IV 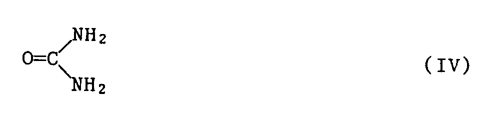 mit einem Keton der Formel lll 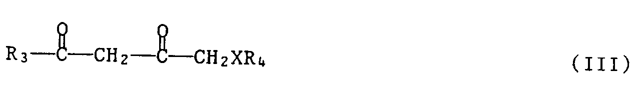 in Gegenwart einer Säure in einem inerten Lösungsmittel bei Temperaturen von 20 ° C bis 140 ° C, bevorzugt 40 ° C bis 100 ° C, zur Reaktion bringt und danach bei Rückflusstemperatur des verwendeten Lösungsmittels zu einer Pyrimidinverbindung der Formel V 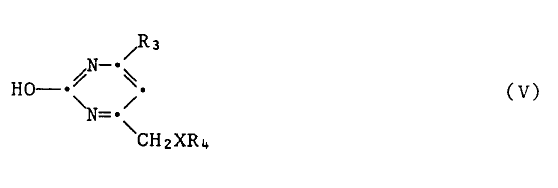 cyclisiert und
   2.2 die erhaltene Verbindung der Formel V weiter mit überschüssigem POCl₃ ohne Lösungsmittel oder in einem gegen POCl3 inerten Lösungsmittel bei Temperaturen von 50 ° C bis 110 ° C, bevorzugt bei der Rückflusstemperatur des POC!₃, zu der Verbindung der Formel VI 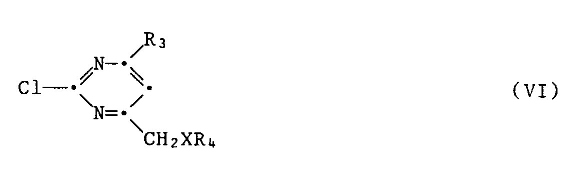 umsetzt und
   2.3 die erhaltene Verbindung der Formel VI weiter mit einer Anilinverbindung der Formel VII 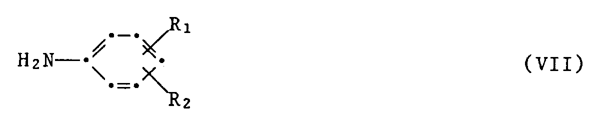 je nach Verfahrensbedingungen entweder
      a) in Gegenwart eines Protonenakzeptors, wie der überschüssigen Anilinverbindung der Formel VII oder einer anorganischen Base, ohne Lösungsmittel oder in einem protischen oder aprotischen Lösungsmittel oder
      b) in Gegenwart einer Säure in einem inerten Lösungsmittel bei jeweils Temperaturen von 60 ° C bis 120°C, bevorzugt 80 ° C bis 100 ° C, zur Reaktion bringt; oder
3. in einem zwei-stufigen Verfahren:
   3.1 ein Guanidiniumsalz der Formel VIII 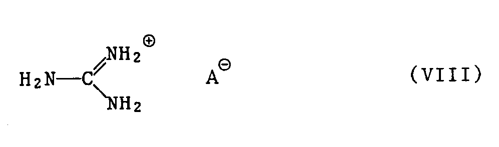 mit einem Diketon der Formel lll 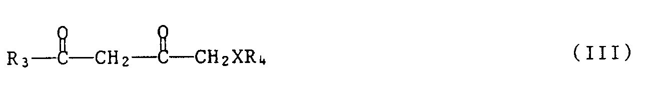
      a) ohne Lösungsmittel bei Temperaturen von 100 ° C bis 160 ° C, bevorzugt 120-150 ° C, oder
      b) in einem protischen oder aprotischen Lösungsmittel oder einem Gemisch aus protischen und aprotischen Lösungsmitteln bei Temperaturen von 30 ° C bis 140 ° C, bevorzugt 60 ° C bis 120 ° C, zu einer Pyrimidinverbindung der Formel IX 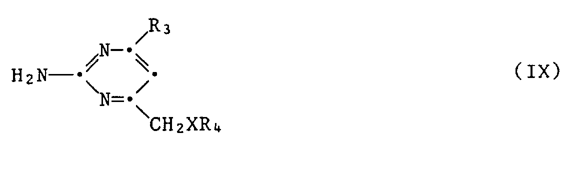 cyclisiert und
   3.2 die erhaltene Verbindung der Formel IX mit einer Verbindung der Formel X 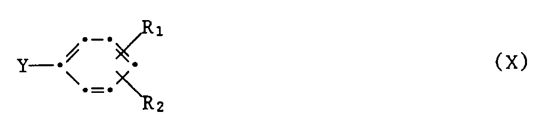 unter Abspaltung von HY in Gegenwart eines Protonenakzeptors in aprotischen Lösungsmitteln bei Temperaturen von 30 ° C bis 140 ° C, bevorzugt 60 ° C bis 120 ° C, umsetzt, wobei in den Formeln ll bis X die Substituenten R₁ bis R₄ und X die unter Formel I angegebenen Bedeutungen besitzen, A^{S} ein Säureanion und Y Halogen darstellen.

In den vorstehend beschriebenen Verfahren kommen bei den Verbindungen der Formeln Ila und VIII mit dem Säureanion A^{S} beispielsweise folgende Salze in Betracht: Carbonat, Hydrogencarbonat, Nitrat, Halogenid, Sulfat oder Hydrogensulfat. Unter Halogenid sind Fluorid, Chlorid, Bromid oder Jodid, bevorzugt Chlorid oder Bromid zu verstehen.

Als Säuren finden vornehmlich anorganische Säuren, wie z.B. Halogenwasserstoffsäuren, beispielsweise Fluorwasserstoffsäure, Chlorwasserstoffsäure oder Bromwasserstoffsäure, sowie Schwefelsäure, Phosphorsaure oder Salpetersäure Verwendung; jedoch können auch geeignete organische Säuren angewendet werden.

Als Protonenakzepto ren dienen z.B. anorganische Basen, wie beispielsweise Alkali- oder Erdalkaliverbindungen, z.B. die Hydroxide, Oxide oder Karbonate von Lithium, Natrium, Kalium, Magnesium, Calcium, Strontium und Barium oder auch Hydride, wie z.B. Natriumhydrid.

In den vorstehend beschriebenen Verfahren können in Anpassung an die jeweiligen Reaktionsbedingungen beispielsweise folgende Lösungsmittel verwendet werden:
Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Chlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Dichlorbenzol, Dibrombenzol, Chlortoluol, Trichlortoluol; Ether, wie Ethylpropylether, Methyl-tert.-butylether, n-Butylethylether, Di-n-butylether, Di-isobutylether, Diisoamylether, Diisopropylether, Anisol, Cyclohexylmethylether, Diethylether, Ethylenglykoldimethylether, Tetrahydrofuran, Dioxan, Thioanisol, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, wie Heptan, Hexan, Octan, Nonan, Cymol, Benzinfraktionen innerhalb eines Siedepunktintervalles von 70 _{°} C bis 190°C, Cyclohexan, Methylcyclohexan, Dekalin, Petrolether, Ligroin, Trimethylpentan wie 2,3,3-Trimethylpentan; Ester wie Ethylacetat, Acetessigester, Isobutylacetat; Amide, z.B. Formamid, Methylformamid, Dimethylformamid; Ketone, wie Aceton, Methylethylketon; Alkohole, insbesondere niedere aliphatische Alkohole, wie z.B. Methanol, Ethanol, n-Propanol, iso-Propanol sowie die Butanole; gegebenenfalls auch Wasser. Auch Gemische der genannten Lösungs- und Verdünnungsmittel kommen in Betracht.

Zu den vorstehend beschriebenen Herstellungsverfahren analoge Synthesemethoden sind in der Literatur an folgenden Stellen publiziert:
Verfahren 1: A.Kreutzberger und J.Gillessen, J.Heterocyclic Chem.22, 101 (1985).
Verfahren 2, Stufe 2.1: O. Stark, Ber.Dtsch.Chem.Ges.42, 699 (1909); J. Hale, J.Am.Chem.Soc.36, 104 (1914); G.M. Kosolapoff, J.Org.Chem.26, 1895 (1961). Stufe 2.2: St. Angerstein, Ber.Dtsch.Chem.Ges.34, 3956 (1901); G.M. Kosolapoff, J.Org.Chem.26, 1895 (1961). Stufe 2.3: M.P.V. Boarland and J.F.W. McOmie, J.Chem.Soc. 1951, 1218; T.Matsukawa und K. Shirakuwa, J.Pharm.Soc.Japan 71, 933 (1951); Chem.Abstr.46, 4549 (1952).
Verfahren 3: Ä. Combes und C. Combes, Bull.Soc. Chem.(3), 7, 791 (1892); W.J. Hale und F.C. Vibrans, J.Am.Chem.Soc.40, 1046 (1918).

Die beschriebenen Herstellungsverfahren einschliesslich aller Teilschritte sind ein Bestandteil der vorliegenden Erfindung.

Ueberraschenderweise wurde nun gefunden, dass Verbindungen der Formel I ein für praktische Bedürfnisse sehr günstiges biozides Spektrum zur Bekämpfung von Insekten und phytopathogenen Mikroorganismen, insbesondere Fungi aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel 1 sind z.B. gegen die den folgenden Klassen angehörenden phytopathogenen Pilze wirksam: Fungi imperfecti (insbesondere Botrytis, Helminthosporium, Fusarium, Septoria, Cercospora und Alternaria); Basidiomyceten (z.B. die Gattungen Hemileia, Rhizoctonia, Puccinia); und Ascomyceten (z.B. Venturia, Podosphaera, Erysiphe, Monilinia, Uncinula). Sie wirken auch gegen Oomyceten (Perenosporales, Phytophthora, Plasmopara, Pythium). Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Verbindungen der Formel 1 sind darüberhinaus gegen Schadinsekten wirksam, z.B. gegen Schädlinge an Getreide wie Reis.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel 1 bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Hirn- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen-und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel 1 bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha, bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen C₈ bis Cl 2, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden. Besonders vorteilhafte applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, die man beispielsweise aus Sojabohnen gewinnen kann.

Als oberflächenaktive Verbindungen kommen je nach Art des zu formulierenden Wirkstoffs der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren (C₁₀-C₂₂), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdlalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsauren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind in wasserlöslichen 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheiten 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele 1 und 2 dienen zur näheren Erläuterung des Herstellungsverfahrens, ohne dass die gewonnenen Verbindungen vom Schutzumfang der Ansprüche erfasst werden.

### Beispiel 1: Herstellung von 2-Phenylamino-4-methyl-6-methoxymethylpyrimidin

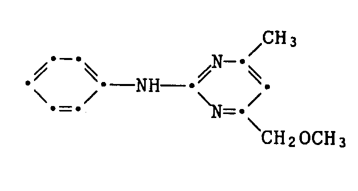

Ein Gemisch von 7,5 g Phenylguanidin-hydrogencarbonat und 7,4 g Methoxyacetylaceton werden unter Rühren vier Stunden auf 100 ° C erwärmt, wobei die einsetzende Kohlendioxidentwicklung mit fortschreitender Reaktionsdauer nachlässt. Nach dem Abkühlen auf Raumtemperatur wird die braune Emulsion mit 60 ml Diethylether versetzt, dreimal mit je 20 ml Wasser gewaschen, über Natriumsulfat getrocknet, filtriert und das Lösungsmittel verdampft. Die 10,5 g öliger Rückstand werden in 140 ml Diethylether gelöst und unter Rühren mit 4,4 g 65 %iger Salpetersäure versetzt. Die Suspension des ausgefallenen Nitratsalzes wird noch 20 Minuten nachgerührt, filtriert und mit 100 ml Diethylether nachgewaschen. Ein Gemisch, bestehend aus 11 g des Nitratsalzes, 80 ml Diethylether und 60 ml Wasser wird unter Rühren mit 6 g 30 %iger Natronlauge versetzt, die organische Phase abgetrennt, zweimal mit je 40 ml Wasser gewaschen, über Natriumsulfat getrocknet, filtriert und das Lösungsmittel verdampft. Die 8,7 g des hellbraunen öligen Rückstandes werden mit 100 ml Petrolether (Sdp. 50-70 ° C) zur Kristallisation gebracht, filtriert und getrocknet. Die 8 g des hellen, beige-farbenen Kristallpulvers schmelzen bei 59-60 ° C; Ausbeute: 92 % d.Th. bezogen auf Phenylguanidin-hydrogencarbonat.

### Beispiel 2: Herstellung von 2-(p-Chlorphenylamino)-4-methyl-6-methoxymethyl-pyrimidin

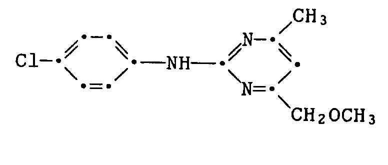

Eine Lösung von 6,4 g 4-Chloranilin und 8,6 g 2-Chlor-4-methyl-6-methoxymethyl-pyrimidin wird unter Rühren mit 5 ml konz. Salzsäure auf pH 1 gebracht und anschliessend 20 Stunden unter Rückfluss erhitzt. Nach dem Abkühlen auf Raumtemperatur wird die braune Emulsion mit 12 ml 30%igem Ammoniak alkalisch gestellt, auf 100 ml Eiswasser gegossen und dreimal mit je 50 ml Ethylacetat extrahiert. Die vereinigten Extrakte werden mit 50 ml Wasser gewaschen, über Natriumsulfat getrocknet, filtriert und das Lösungsmittel verdampft. Die 11,8 g rotes Oel werden über eine 30 cm lange Kieselgelsäule mittels Dichlormethan/Diethylether (3:2) chromatographiert. Nach Verdampfen des Laufmittels wird der zunächst ölige Rückstand durch Anreiben mit Petrolether zur Kristallisation gebracht. Nach dem Umkristallisieren aus Diisopropylether/Petrolether (50-70 ° C) werden 9,8 g beige-farbenes Kristallpulver erhalten, das bei 57-59 ° C schmilzt; Ausbeute: 74 % d. Th.

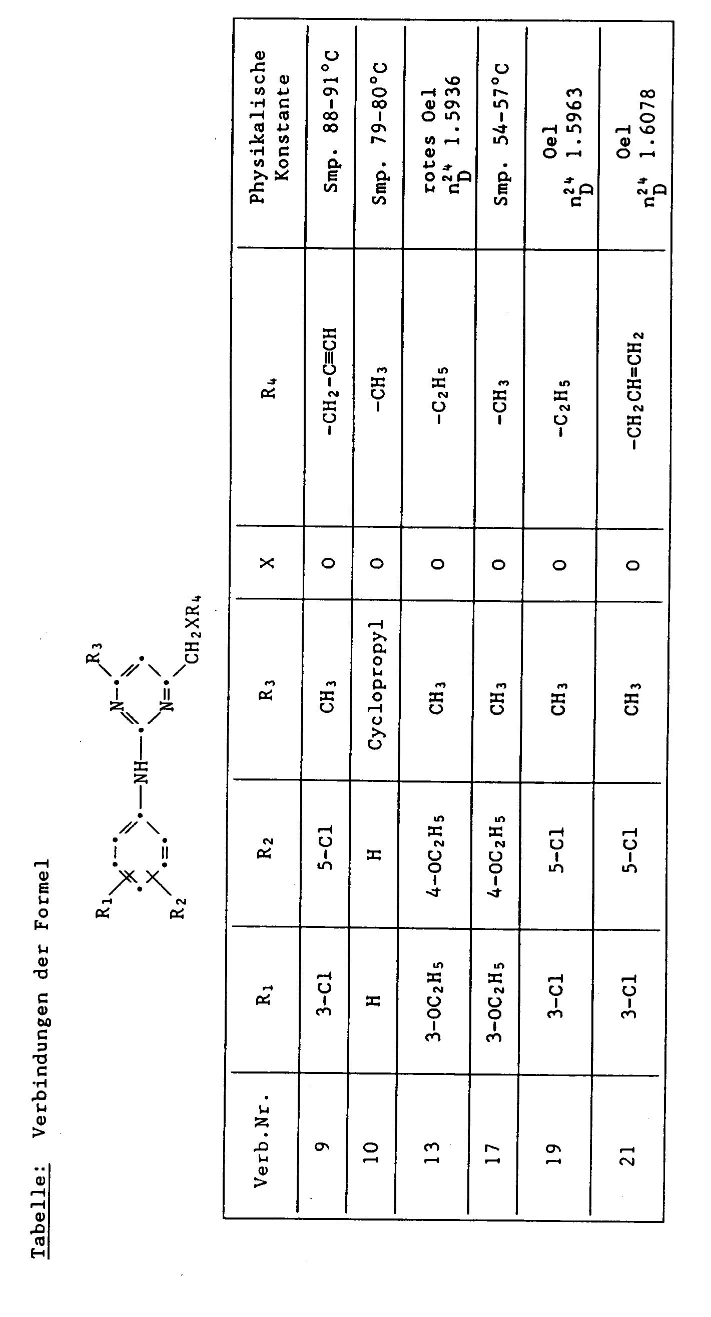

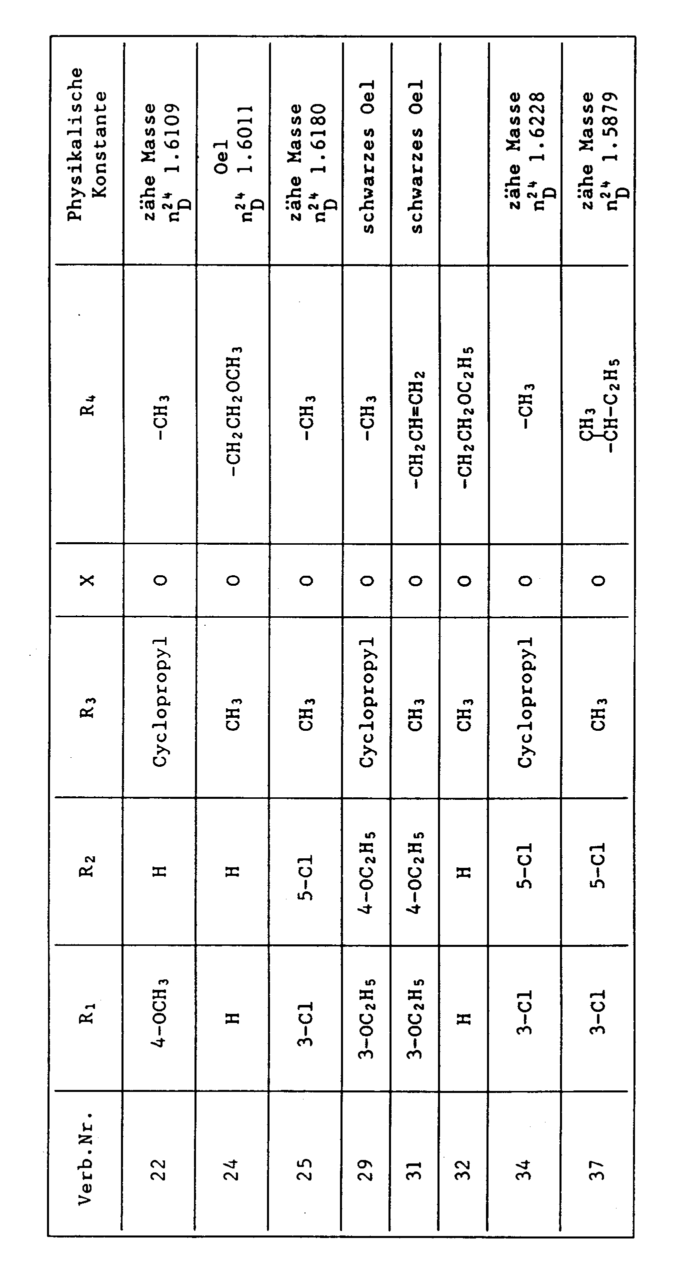

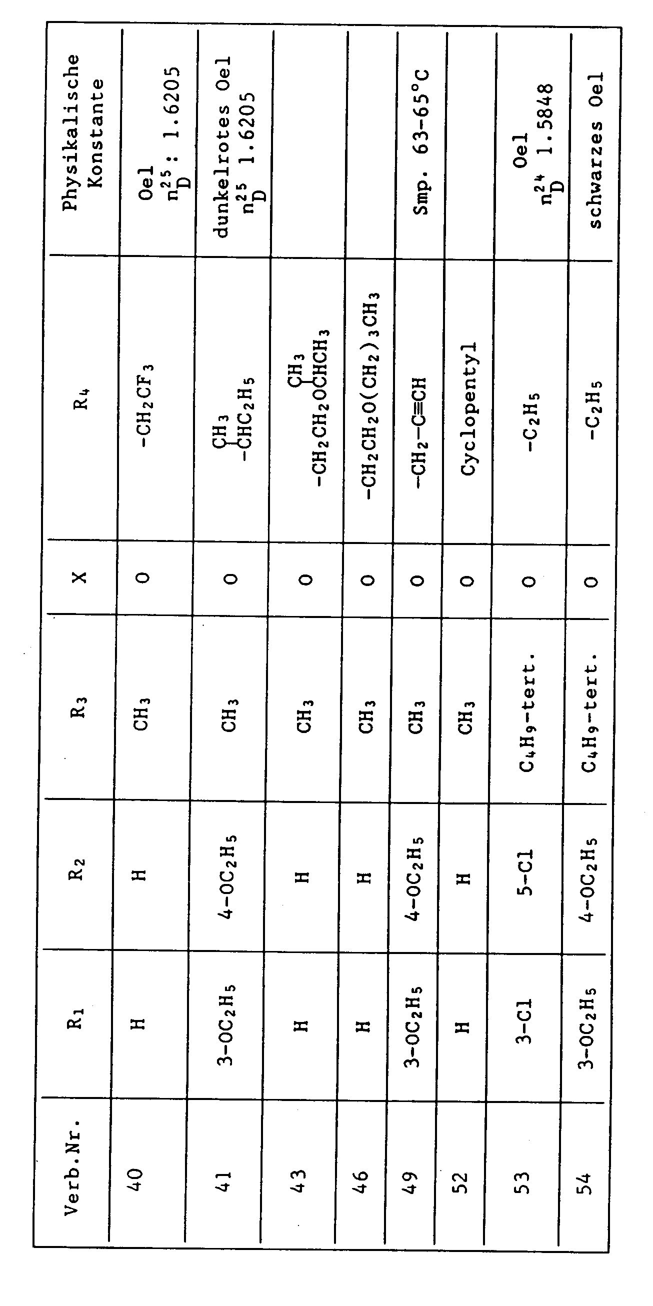

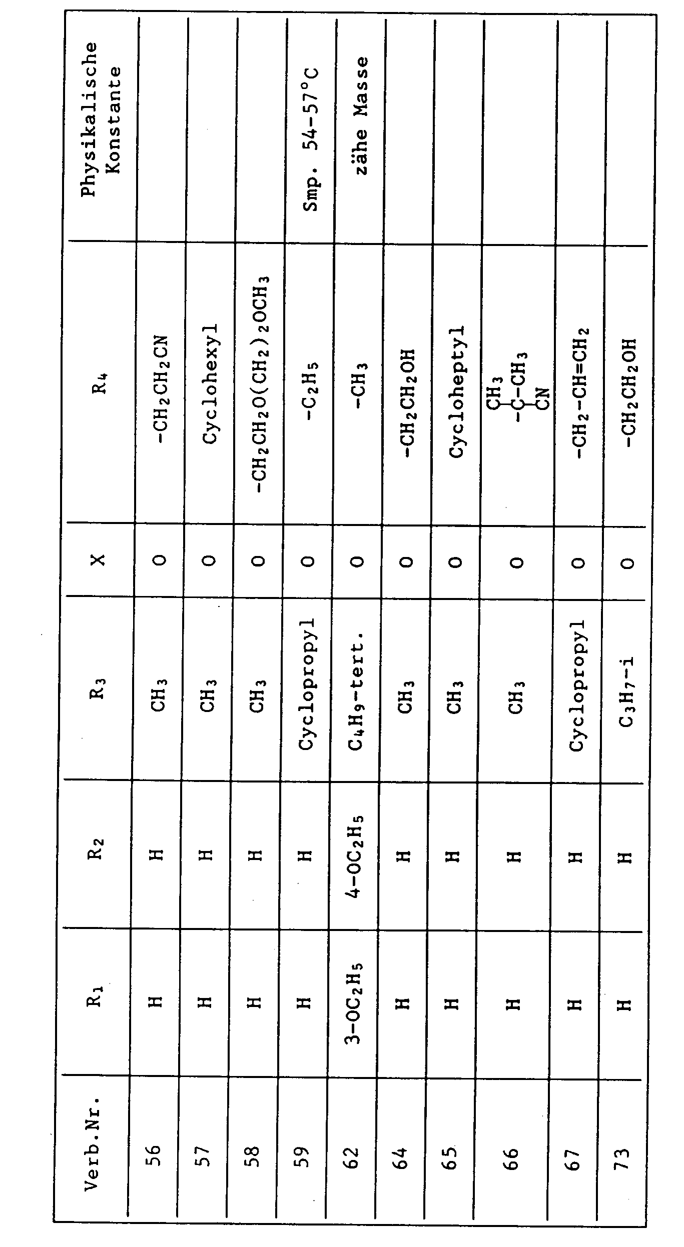

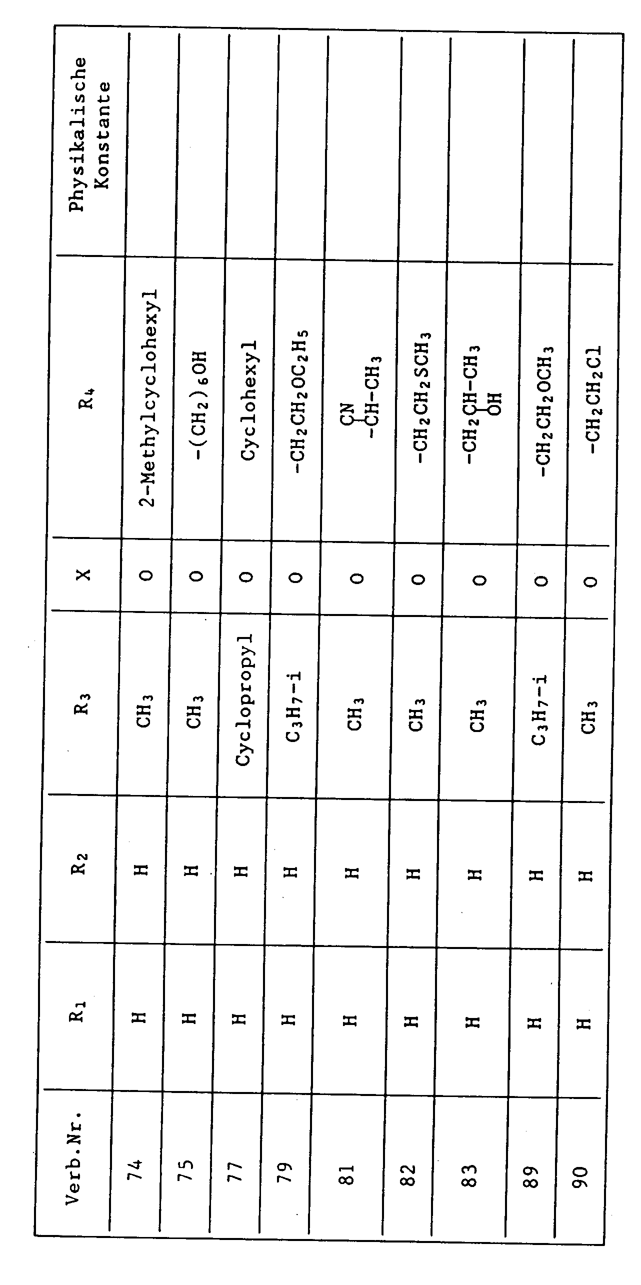

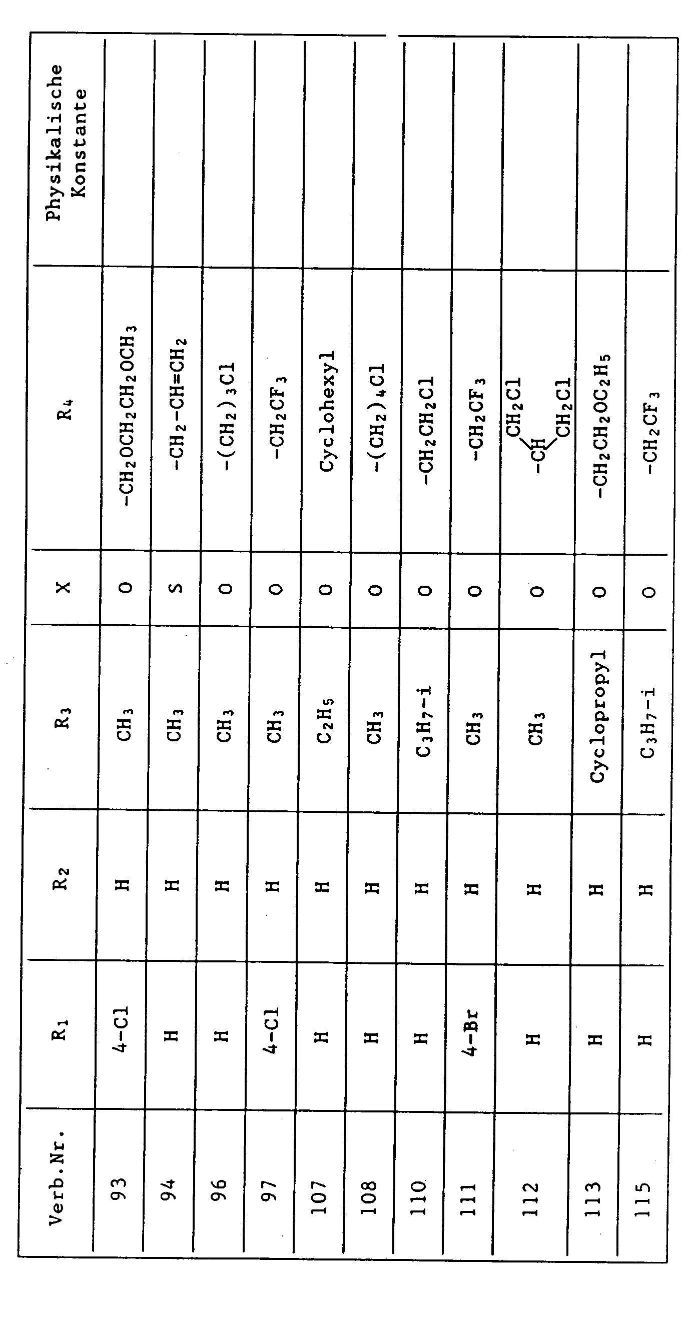

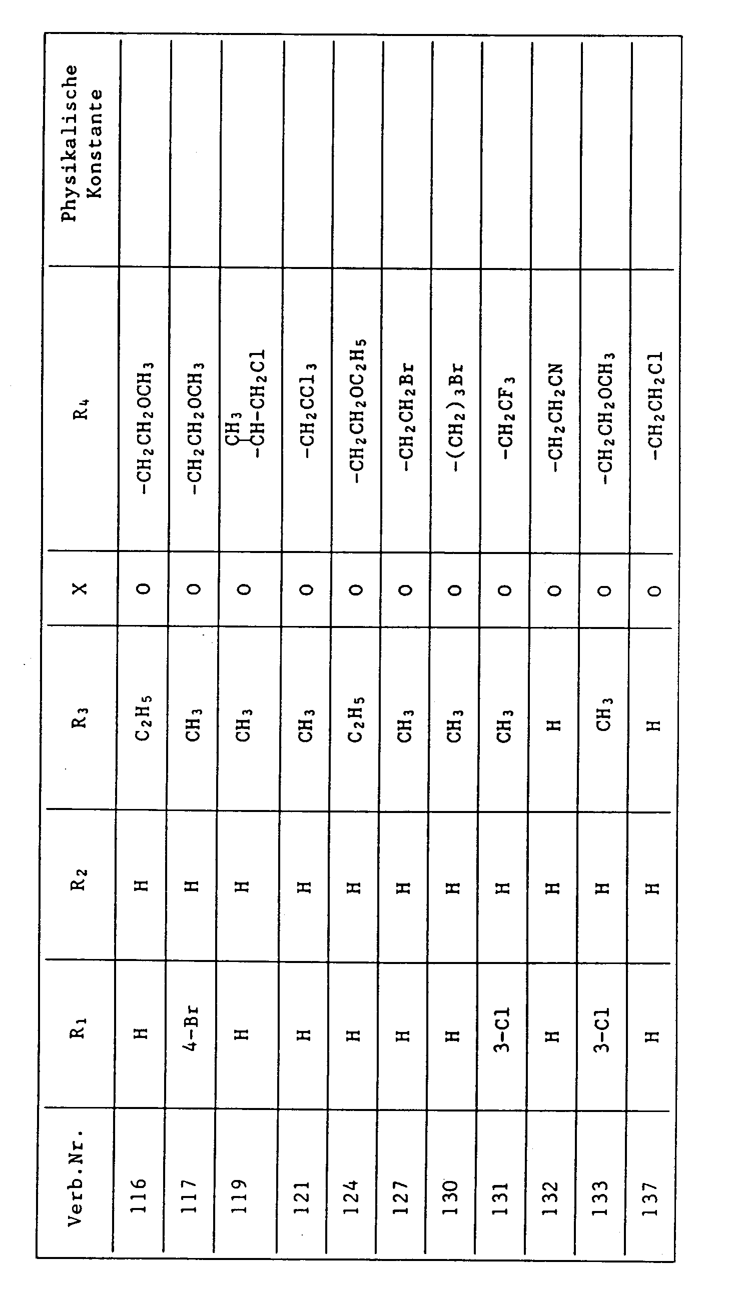

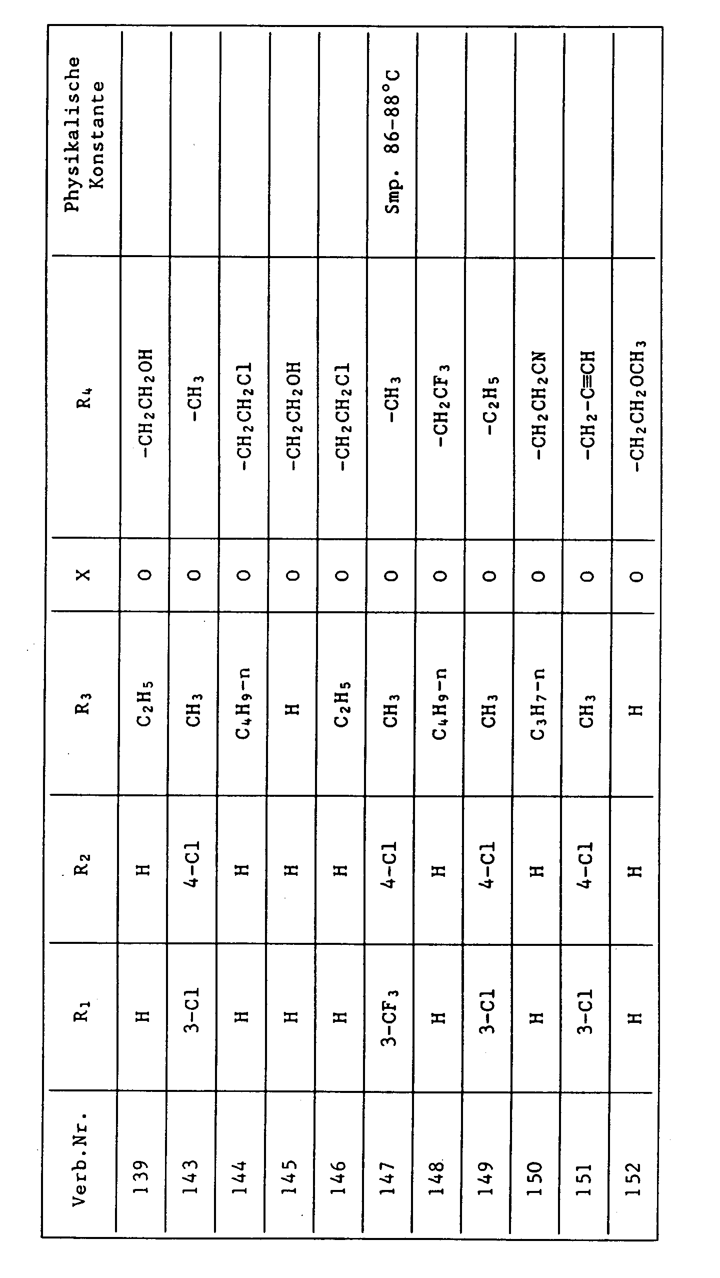

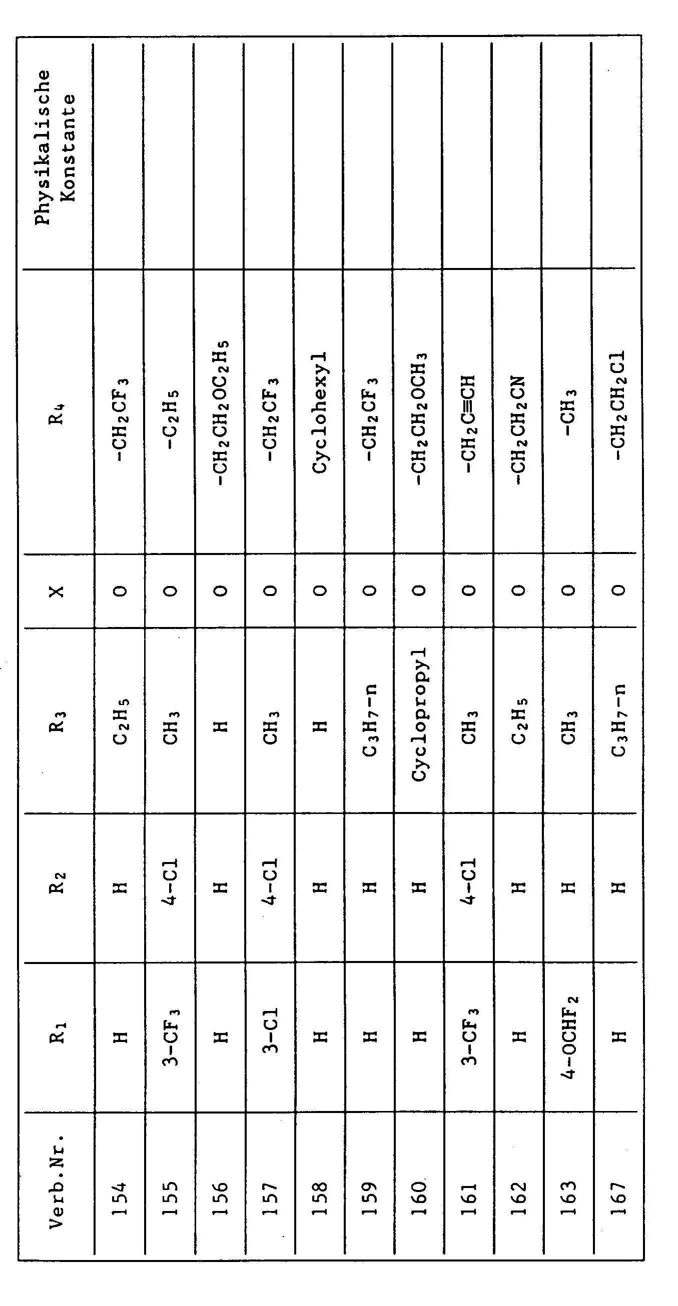

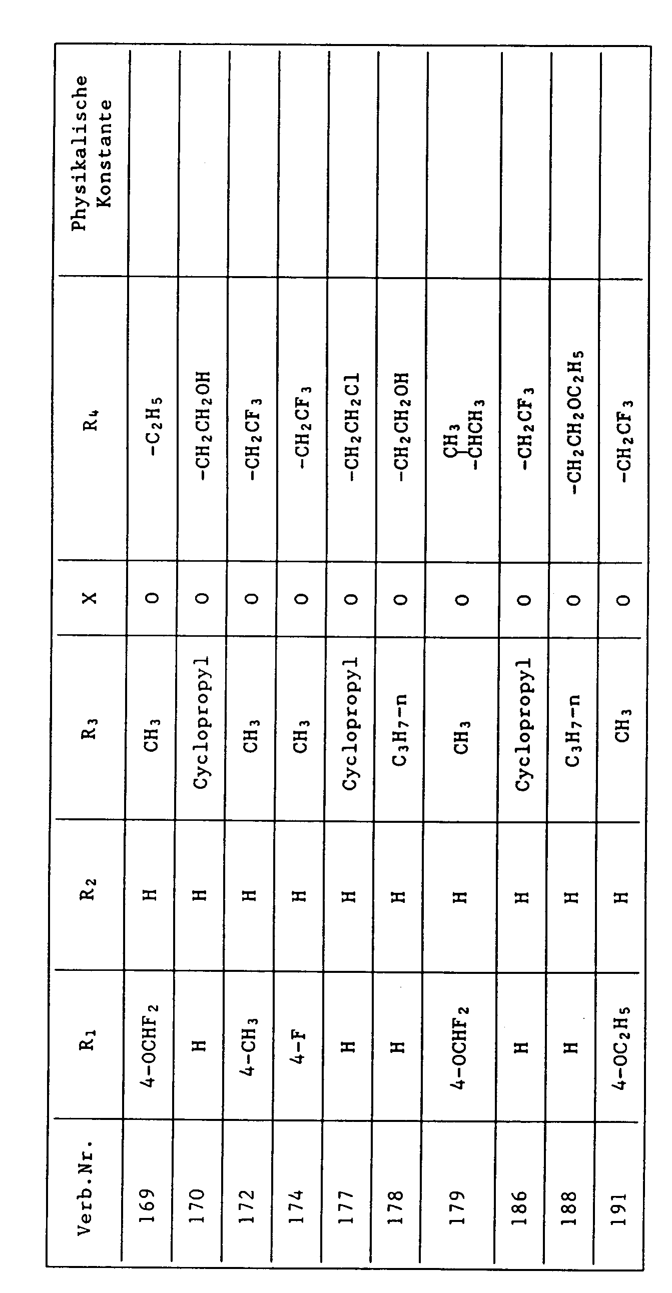

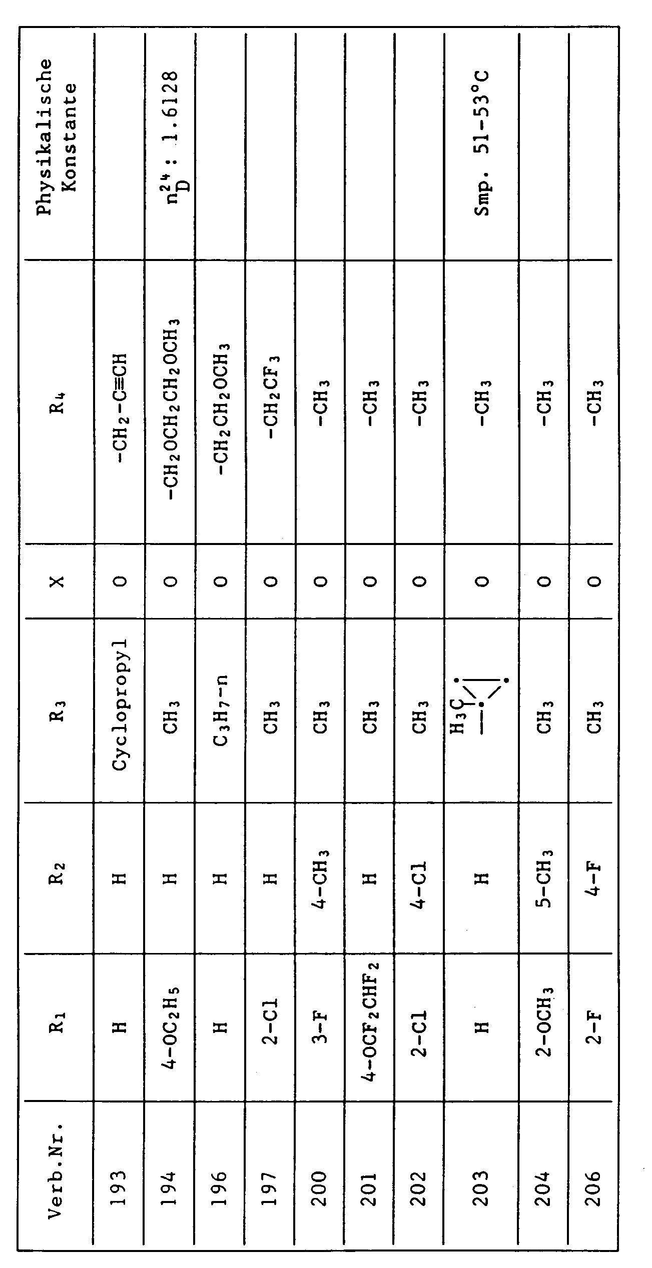

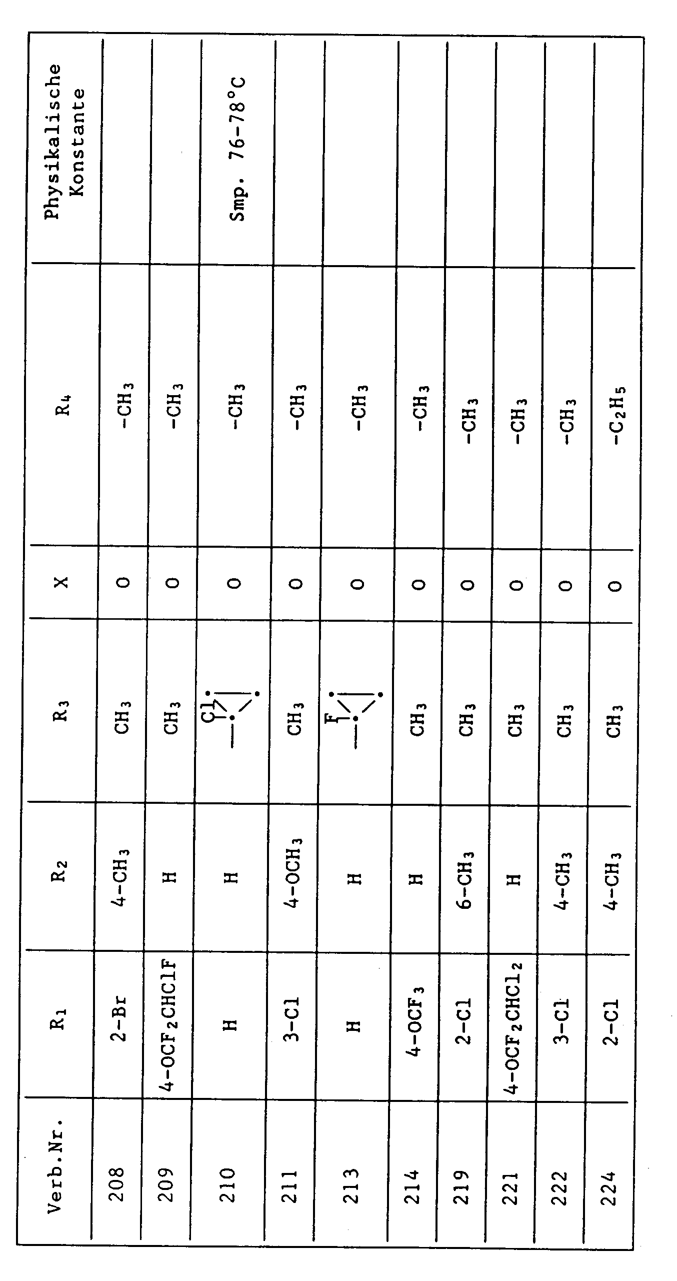

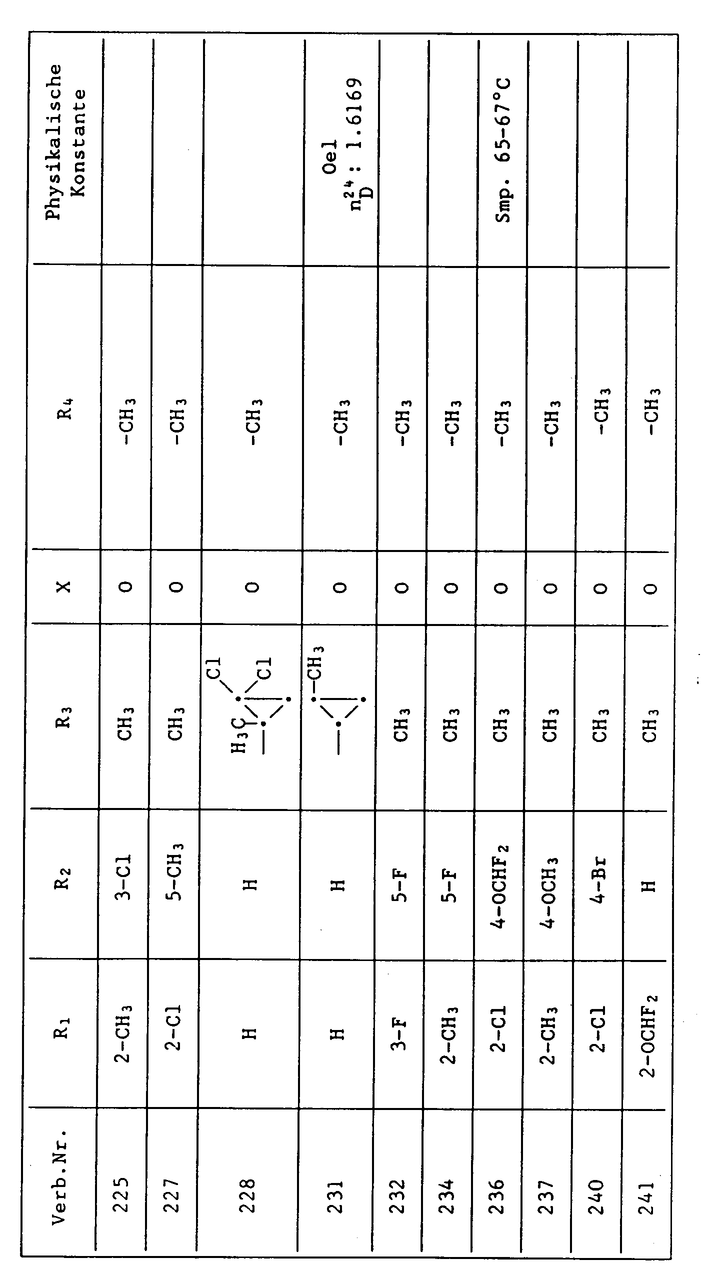

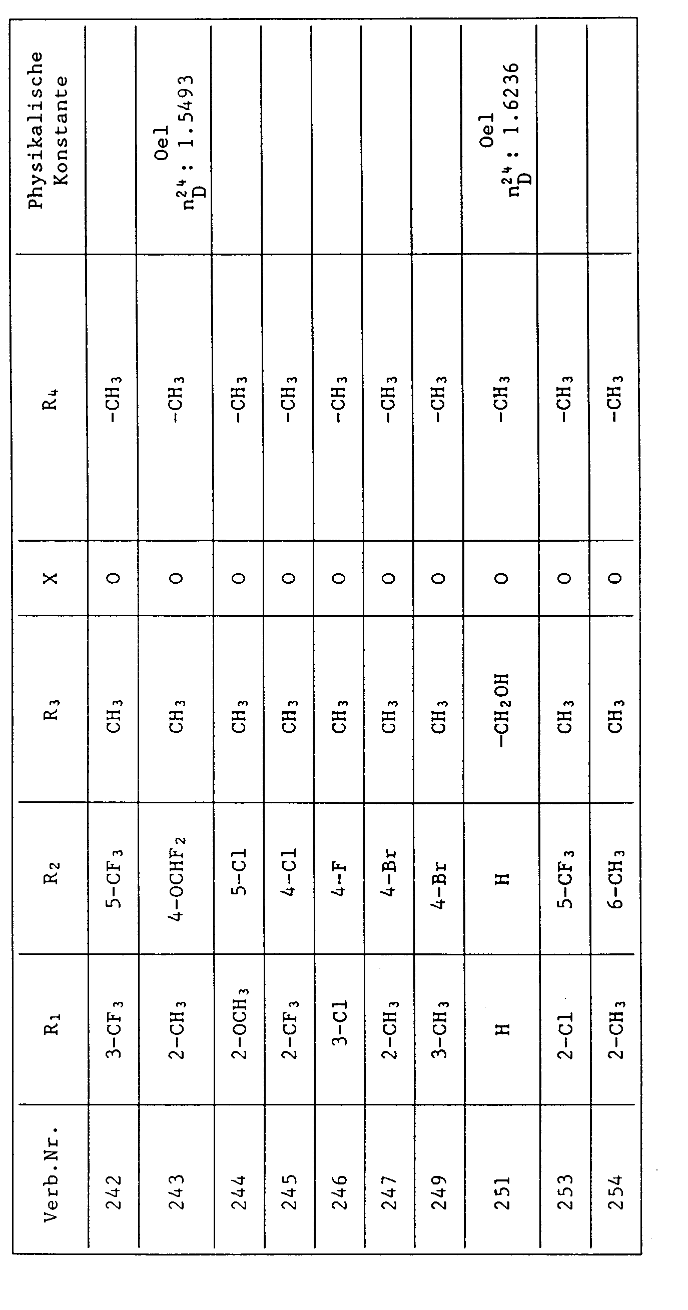

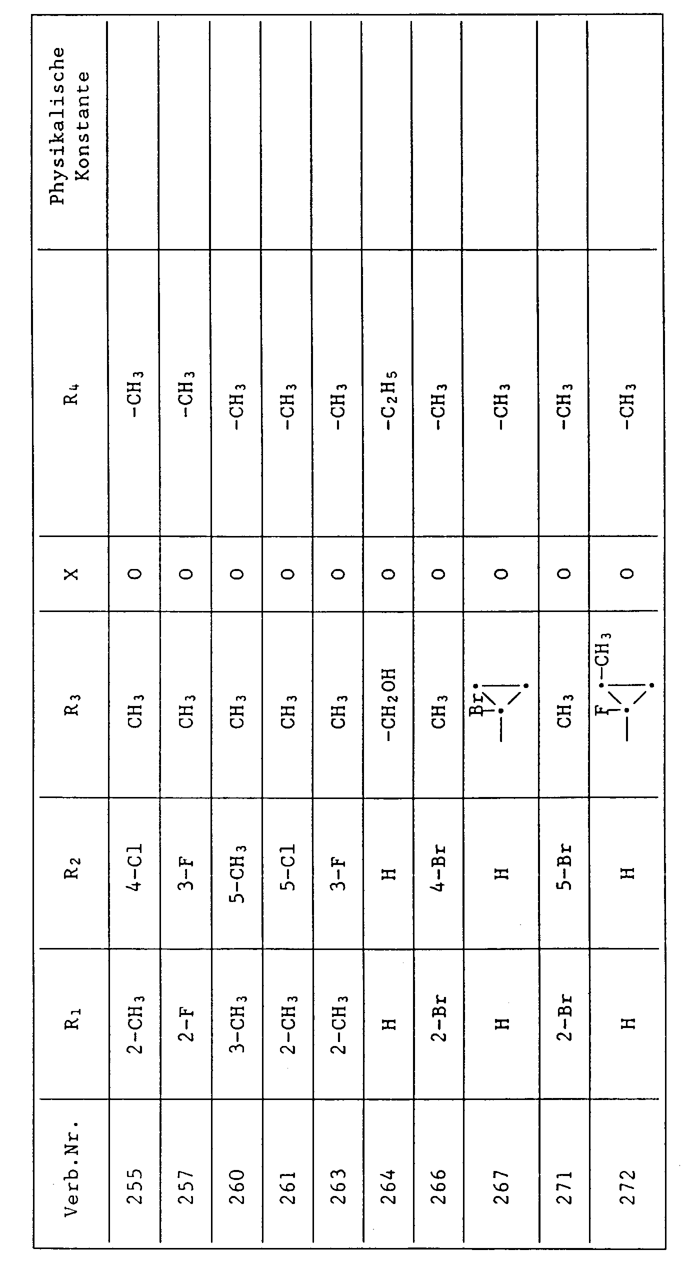

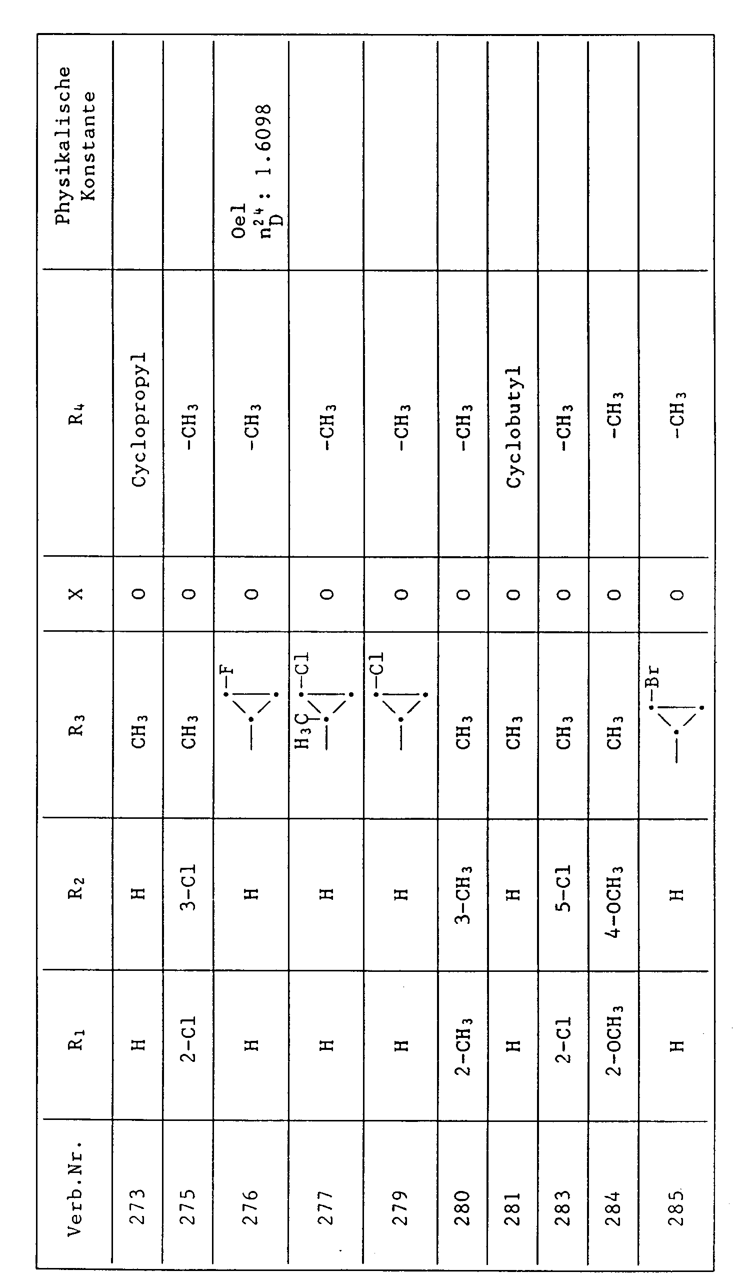

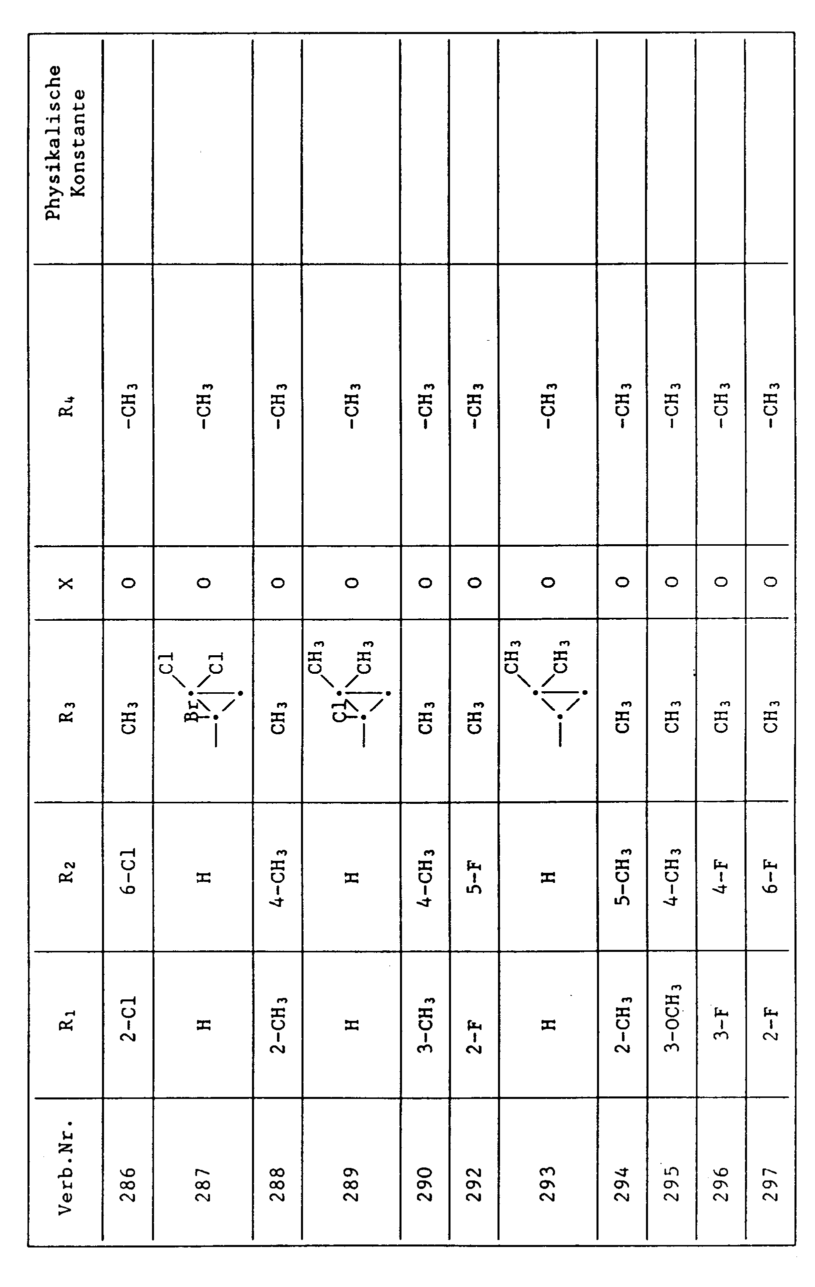

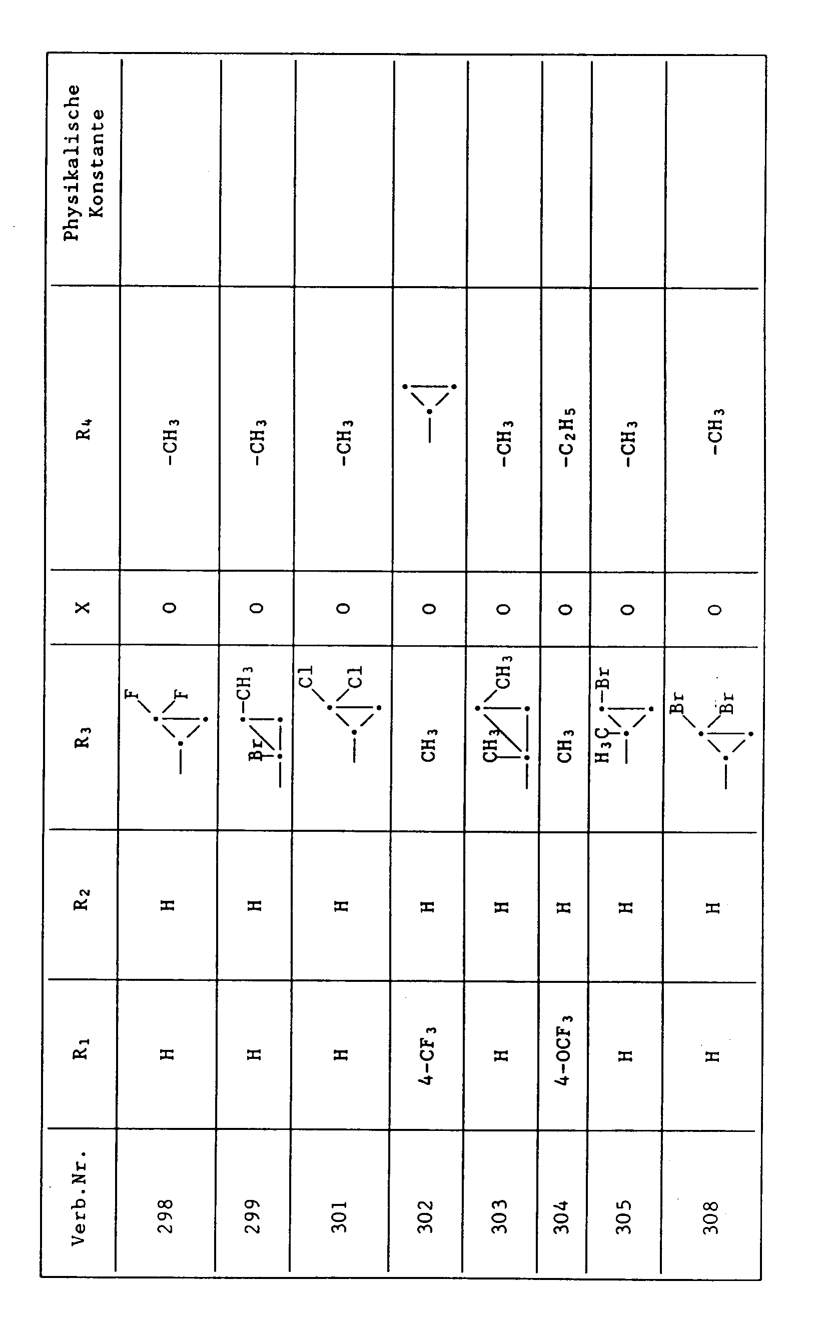

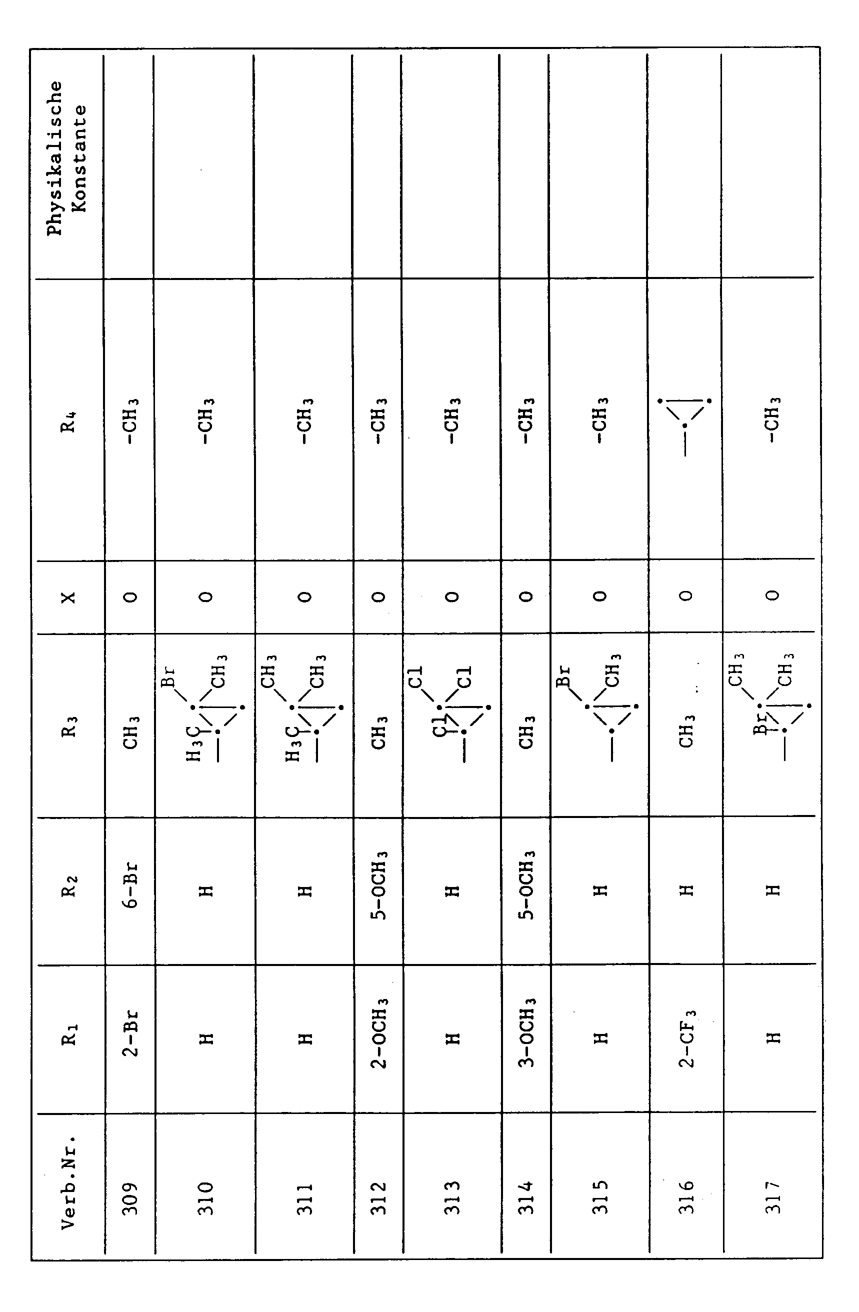

### 2. Formulierungsbeispiele für flüssige Wirkstoffe der Formel I % = Gewichtsprozent)

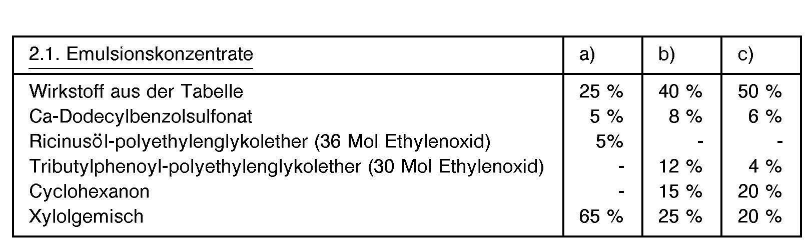

Aus solchen Konzentraten können durch Verdünnen mit Wasser Emulsionen jeder gewümschten Konzentration hergestellt werden.

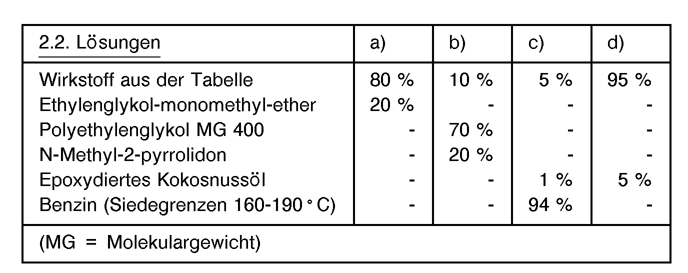

Die Lösungen sind zur Anwendung in Form kleinster Tropfen geeignet.

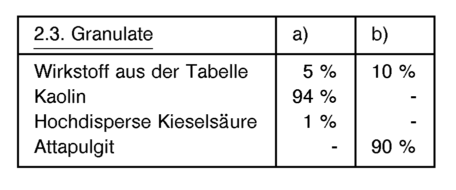

Der Wirkstoff wird in Methylenchlorid gelöst, auf den Träger aufgesprüht und das Lösungsmittel anschliessend im Vakuum abgedampft.

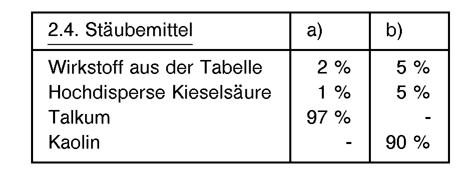

Durch inniges Vermischen auf Trägerstoffe mit dem Wirkstoff erhält man gebrauchsfertige Stäubemittel.

### Formulierungsbeispiele für feste Wirkstoffe der Formel 1 (% = Gewichtsprozent)

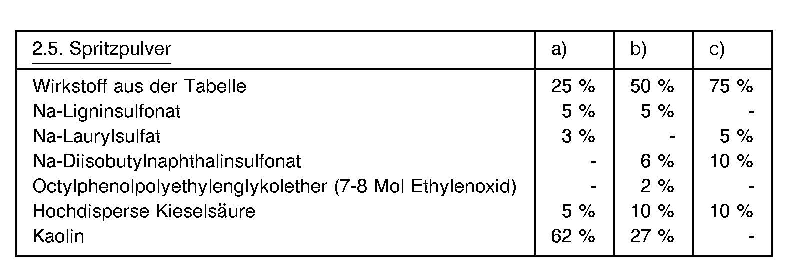

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

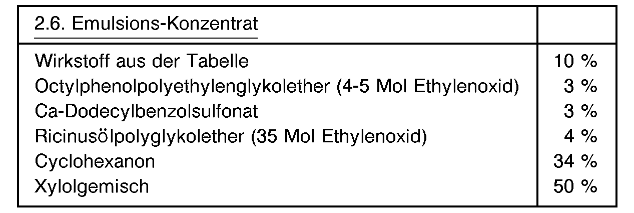

Aus diesem Konzentrat können durch Verdünnen mit Wasser Emulsionen jeder gewünschten Konzentration hergestellt werden.

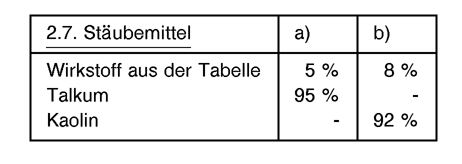

Man erhält anwendungsfertige Stäubemittel, indem der Wirkstoff mit dem Träger vermischt auf einer geeigneten Mühle vermahlen wird.

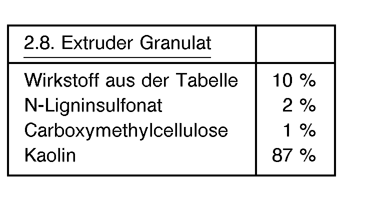

Der Wirkstoff wird mit den Zusatzstoffen vermischt, vermahlen und mit Wasser angefeuchtet. Dieses Gemisch wird extrudiert und anschliessend im Luftstrom getrocknet.

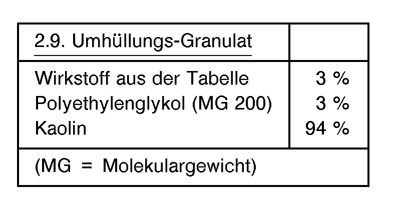

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

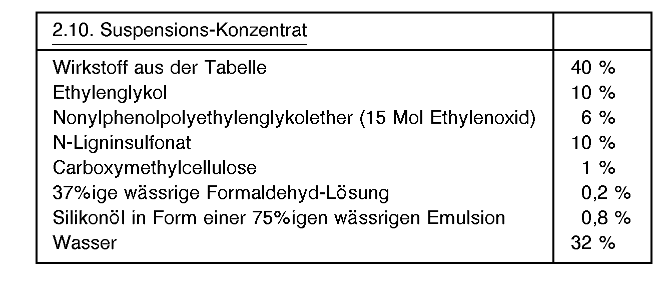

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### 3. Biologische Beispiele

### Beispiel 3.1: Wirkung gegen Puccinia graminis auf Weizen

### a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 _{°} C werden die infizierten Pflanzen in einem Gewächshaus bei ca. 22 _{°} C aufgestellt. Die Beurteilung der Rostpustelnentwicklung erfolgt 12 Tage nach der Infektion.

### b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 ° C werden die infizierten Pflanzen in einem Gewächshaus bei ca. 22 ° C aufgestellt. Die Beurteilung der Rostpustelnentwicklung erfolgt 12 Tage nach der Infektion.

Verbindungen aus der Tabelle zeigen gegen Puccinia gute Wirksamkeit (Befall: weniger als 20 %). Unbehandelte aber infizierte Kontrollpflanzen weisen dagegen einen Puccinia-Befall von 100 % auf.

### Beispiel 3.2: Wirkung gegen Phytophthora auf Tomatenpflanzen

### a) Residual-protektive Wirkung

Tomatenpflanzen wurden nach dreiwöchiger Anzucht mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

### b) Residual-kurative Wirkung

Tomatenpflanzen wurden nach dreiwöchiger Anzucht mit einer Sporangiensuspension des Pilzes infiziert. Nach einer Inkubation von 22 Stunden irt einer Feuchtkammer bei 90-100 % relativer Luftfeuchtigkeit und 20 _{°} C wurden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 5 Tage nach der Infektion.

### c) Systemische Wirkung

Zu Tomatenpflanzen wurde nach dreiwöchiger Anzucht eine aus Spritzpulver des Wirkstoffes hergestellte Spritzbrühe (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 _{°} C.

Verbindungen aus der Tabelle zeigen gegen Phytophthora gute Wirksamkeit (Befall: weniger als 20 %). Unbehandelte aber infizierte Kontrollpflanzen weisen dagegen einen Phytophthora-Befall von 100 % auf.

### Beispiel 3.3: Wirkung gegen Plasmopara viticola auf Reben

### Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20 _{°} C wird der Pilzbefall beurteilt.

Verbindungen aus der Tabelle zeigen gegen Plasmopara gute Wirksamkeit. Unbehandelte aber infizierte Kontrollpflanzen weisen dagegen einen Plasmopara-Befall von 100 % auf.

### Beispiel 3.4: Wirkung gegen Cercospora arachidicola auf Erdnusspflanzen

### Residual-protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21 _{°} C und hoher Luftfeuchtigkeit inkubiert und anschliessend bis zum Auftreten des typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Verbindungen aus der Tabelle zeigen gegen Cercospora gute Wirksamkeit (Befall: weniger als 20 %). Unbehandelte aber infizierte Kontrollpflanzen weisen dagegen einen Cercospora-Befall von 100 % auf.

### Beispiel 3.5: Wirkung gegen Venturia inaequalis auf Apfeltrieben

### Residual-protektive Wirkung

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24 ° C aufgestellt. Der Schorfbefall wird 15 Tage nach der Infektion beurteilt.

Verbindungen aus der Tabelle zeigen gegen Venturia gute Wirksamkeit; so reduzierten z.B. die Verbindungen Nr. 10, 59, 231, 276 den Venturia-Befall auf weniger als 10 %. Unbehandelte aber infizierte Kontrollpflanzen weisen dagegen einen Venturia-Befall von 100 % auf.

### Beispiel 3.6: Wirkung gegen Botrvtis cinerea an Apfelfrüchten

### Residual-protektive Wirkung

Künstlich verletzte Aepfel werden behandelt, indem eine aus Spritzpulver der Wirksubstanz hergestellte Spritzbrühe (0,002 % Aktivsubstanz) auf die Verletzungsstellen aufgepfropft wird. Die behandelten Früchte werden anschliessend mit einer Sporensuspension des Pilzes inokuliert und während einer Woche bei hoher Luftfeuchtigkeit und ca. 20 _{°} C inkubiert. Bei der Auswertung werden die angefaulten Verletzungsstellen gezählt und daraus die fungizide Wirkung der Testsubstanz abgeleitet.

Verbindungen aus der Tabelle zeigen gegen Botrytis gute Wirksamkeit (Befall: weniger als 20 %). So reduzierten z.B. die Verbindungen Nr. 10, 59, 231, 276 den Botrytis-Befall auf 0 bis 5 %. Unbehandelte aber infizierte Kontrollpflanzen weisen dagegen einen Botrytis-Befall von 100 % auf.

### Beispiel 3.7: Wirkung gegen Erysiphae graminis auf Gerste

### Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22 _{°} C aufgestellt und der Pilzbefall nach 10 Tagen beurteilt.

Verbindungen aus der Tabelle zeigen gegen Erysiphae gute Wirksamkeit; so reduzierten z.B. die Verbindungen Nr. 10, 59, 231 den Erysiphae-Befall auf weniger als 20 %. Unbehandelte aber infizierte Kontrollpflanzen weisen dagegen einen Erysiphae-Befall von 100 % auf.

### Beispiel 3.8: Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Die Verbindungen der Tabelle verhindern den Pilzbefall weitgehend (0 bis 10 %).

### Beispiel 3.9: Wirkung gegen Fusarium nivale

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen.

Bei den Körnern, die mit einem Spritzpulver behandelt worden sind, das als Wirkstoff eine Verbindung der Tabelle enthält, wurde die Entwicklung der Pilzkolonien fast vollständig unterdrückt (0 bis 5 %).

### Beispiel 3.10: Wirkung gegen Tilletia caries

Gerstenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Die Verbindungen der Tabelle verhindern den Pilzbefall weitgehend (0 bis 10 %).

### Beispiel 3.11: Wirkung gegen Colletotrichum lagenarium auf Gurken (Cucumis sativus L.)

Gurkenpflanzen werden nach 2-wöchiger Anzucht mit einer aus Spritzpulver des Wirkstoffs hergestellten Spritzbrühe (Konzentration 0,02 %) besprüht. Nach 2 Tagen werden die Pflanzen mit einer Sporensuspension (1,5 x 10⁵ Sporen/ml) des Pilzes infiziert und für 36 Stunden im Dunkeln bei 23 °C und hoher Luftfeuchtigkeit inkubiert. Die Inkubation wird dann bei normaler Luftfeuchtigkeit und ca. 22-23 _{°} C weitergeführt. Der eingetretene Pilzbefall wird 8 Tage nach der Infektion beurteilt. Unbehandelte aber infizierte Kontrollpflanzen weisen einen Pilzbefall von 100 % auf.

Verbindungen aus der Tabelle zeigen gute Wirksamkeit und verhindern die Ausbreitung des Krankheitsbefalls. Der Pilzbefall wird auf 20 % oder weniger zurückgedrängt.

### Beispiel 3.12: Wirkung gegen Septoria nodorum auf Weizen

### Residual-protektive Wirkung

7 Tage alte Weizenpflanzen werden mit einer aus der formulierten Substanz hergestellten Spritzbrühe (Konzentration 0,02 %) besprüht und 2 Tage später mit einer Konidiensuspension (400'000 Konidien/ml mit Zusatz von 0,1 % Tween 20 als Netzmittel) von Septoria nodorum infiziert. Nach einer 2-tägigen Inkubationsphase in einer Gewächshauskabine bei 20 ° C und 95-100 % rel. Luftfeuchtigkeit werden die Testpflanzen bis zum Versuchsende unbedeckt in einer Gewächshauskabine bei 21 _{°}C und 60 % rel. Luftfeuchtigkeit plaziert. Die Bonitierung des Pilzbefalls erfolgt 7 bis 10 Tage nach der Infektion.

Während unbehandelte, aber infizierte Kontrollpflanzen vollständig befallen sind, weisen Pflanzen, die mit Verbindungen aus der Tabelle behandelt werden, einen Pilzbefall von weniger als 20 % auf.

### Beispiel 3.13: Wirkung gegen Alternaria solani auf Tomaten

### Residual-protektive Wirkung

3 Wochen alte Tomatenpflanzen werden mit einer aus der formulierten Prüfsubstanz hergestellten Spritzbrühe (Konzentration 0,02 %) besprüht und 2 Tage später beidseitig mit einer Konidiensuspension (20'000 Konidien/ml) von Alternaria solani infiziert. Um ein Abwaschen des feintropfigen Sprühbelages zu verhindern, werden die infizierten Pflanzen in einer ersten 3-tägigen Inkubationsphase in einer Gewächshauskabine bei 20 _{°} C und Kulimatbefeuchtung mit engmaschigen Netzen abgedeckt. Anschliessend kommen die Pflanzen bis zum Versuchsende in eine Gewächshauskabine bei 24 °C. Die Bonitierung des Pilzbefalls erfolgt 4 Tage nach der Infektion.

Während unbehandelte, aber infizierte Kontrollpflanzen vollständig befallen sind, weisen Pflanzen, die mit Verbindungen aus der Tabelle behandelt werden, einen Pilzbefall von weniger als 20 % auf.

### Beispiel 3.14:

### a) Insektizide Kontakt-Wirkung gegen Nephotettix cincticeps und Nilaparvata lugens (Nymphen)

Der Test wird an wachsenden Reis-Pflanzen durchgeführt. Dazu werden jeweils 4 Pflanzen (14-20 Tage alt) mit einer Höhe von ca. 15 cm in Töpfe (Durchmesser 5,5 cm) eingepflanzt.

Die Pflanzen werden auf einem Drehteller mit 100 ml einer wässrigen Emulsions-Zubereitung enthaltend 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des Adultstadiums über 6 Tage an der behandelten Pflanze gehalten. Die Auswertung auf prozentuale Mortalität erfolgt 6 Tage nach der Besiedlung. Der Versuch wird bei etwa 27 °C, 60 % rel. Luftfeuchtigkeit und einer Beleuchtungsperiode von 16 Stunden durchgeführt.

### b) Systemische Insektizid-Wirkung gegen Nilaparvata lugens (in Wasser)

Etwa 10 Tage alte Reispflanzen (ca. 10 cm hoch) werden in einen Plastik-Becher eingestellt, der 150 ml einer wässrigen Emulsions-Zubereitung des zu prüfenden Wirkstoffes in einer Konzentration von 100 ppm enthält und mit einem mehrfach durchlöcherten Plastikdeckel abgeschlossen ist. Die Wurzel der Reispflanze wird durch ein Loch in dem Plastikdeckel in die wässrige Test-Zubereitung geschoben. Dann wird die Reispflanze mit 20 Nymphen von Nilaparvata lugens im N 2 bis N 3 Stadium besiedelt und mit einem Plastikzylinder abgedeckt. Der Versuch wird bei ca. 26 ° C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen aus der Tabelle zeigen sowohl beim Kontaktversuch wie beim systemischen Versuch mehr als 80 % Wirkung gegen die genannten Reisschädlinge.

## Patentansprüche (Patentansprüche für folgende(n) Vertragsstaat(en) : AT, BE, GR, LU, SE)

1. Verbindungen der Formel I 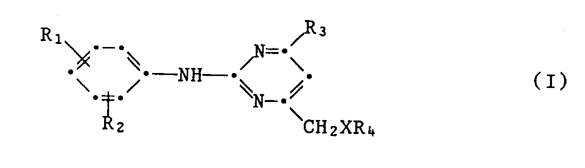 in welcher bedeuten:
R₁ und R₂ unabhängig voneinander Wasserstoff, Halogen, C₁-C₃-Alkyl, C₁-C₂-Halogenalkyl, C₁-C₃-Alkoxy oder C₁ -C₃ Halogenalkoxy;
R₃ Wasserstoff, C₁-C₄-Alkyl, durch Halogen oder Hydroxy substituiertes C₁-C₂-Alkyl, Cyclopropyl oder durch Halogen und/oder Methyl substituiertes Cyclopropyl;
R₄ C₁-C₈-Alkyl; durch Halogen, Hydroxy, Cyano, C₁-C₄-Alkoxy, C₃-C₄-Alkoxyalkoxy oder C₁-C₃-Alkylthio substituiertes C₂-C₆-Alkyl; C₃-C₆-Alkenyl; C₃-C₆-Alkinyl; C₃-C₇-Cycloalkyl oder durch Methyl substituiertes C₃-C₇-Cycloalkyl;
X Sauerstoff oder Schwefel.

2. Verbindungen der Formel I gemäss Anspruch 1, worin R₃, R₄ und X die genannte Bedeutung haben und R₁ und R₂ Wasserstoff darstellen.

3. Verbindungen der Formel I gemäss Anspruch 1, in welcher bedeuten:
R₁ und R₂ unabhängig voneinander Wasserstoff, Halogen, C₁-C₃-Alkyl, C₁-C₂-Halogenalkyl, Ci-C₃-Alkoxy oder C₁ -C₃ Halogenalkoxy;
R₃ Wasserstoff, C₁-C₄-Alkyl oder Cyclopropyl;
R₄ C₁-C₈-Alkyl; durch Halogen, Hydroxy, Cyano, C₁-C₄-Alkoxy, C₃-C₄-Alkoxyalkoxy oder C₁-C₃-Alkylthio substituiertes C₂-C₆-Alkyl; C₃-C₆-Alkenyl; C₃-C₆-Alkinyl; C₅-C₇-Cycloalkyl oder durch Methyl substituiertes C₅-C₇-Cycloalkyl;
X Sauerstoff oder Schwefel.

4. Verbindungen der Formel 1 nach Anspruch 3, in welcher bedeuten:
R₁ und R₂ unabhängig voneinander Wasserstoff, Fluor, Chlor, Brom, C₁-C₃-Alkyl, C₁-C₂-Halogenalkyl, C₁-C₃-Alkoxy oder C₁-C₂-Halogenalkoxy;
R₃ Wasserstoff, C₁-C₃-Alkyl oder Cyclopropyl;
R₄ C₁-C₆-Alkyl; durch Halogen, Hydroxy, Cyano, C₁-C₃-Alkoxy, C₃-C₄-Alkoxyalkoxy oder C₁-C₂-Alkylthio substituiertes C₂-C₄-Alkyl;
C₃-Alkenyl; C₃-Alkinyl; oder unsubstituiertes oder durch Methyl substituiertes C₆-C₇-Cycloalkyl;
X Sauerstoff oder Schwefel.

5. Verbindungen der Formel 1 nach Anspruch 3, in welcher bedeuten:
R₁ und R₂ unabhängig voneinander Wasserstoff, Fluor, Chlor, Brom, C₁-C₃-Alkyl, C₁-C₃-Alkoxy, Trifluormethyl, -OCHF₂, -OCF₂CHF₂, -OCF₂CHCIF, -OCF₂CHCI₂ oder -OCF₂CCI₂F;
R₃ Wasserstoff, Methyl, Ethyl oder n-Propyl;
R₄ C₃-C₄-Alkyl; durch Halogen, Hydroxy, Cyano, C₁-C₂-Alkoxy oder C₁-C₂-Alkylthio substituiertes C₂-C₃-Alkyl; C₃-C₆-Alkenyl; C₃-C₆-Alkinyl oder Cyclohexyl;
X Sauerstoff oder Schwefel.

6. Verbindungen der Formel 1 nach Anspruch 4, in welcher bedeuten:
R₁ und R₂ unabhängig voneinander Wasserstoff, Fluor, Chlor, Brom, C₁-C₂-Alkyl, C₁-C₂-Alkoxy, Trifluormethyl, -OCHF₂, -OCF₃, -OCF₂CHF₂ oder -OCF₂CHCIF;
R₃ Wasserstoff oder C₁-C₂-Alkyl;
R₄ C₁-C₃-Alkyl; durch Halogen, Hydroxy, Cyano, C₁-C₂-Alkoxy oder C₁-C₂-Alkylthio substituiertes C₂-Alkyl; C₃-C₄-Alkenyl; C₃-C₄-Alkinyl;
X Sauerstoff oder Schwefel.

7. Verbindungen der Formel 1 nach Anspruch 6, in welcher bedeuten:
R₁ und R₂ unabhängig voneinander Wasserstoff, Fluor, Chlor, Brom, C₁-C₂-Alkyl, C₁-C₂-Alkoxy, Trifluormethyl oder -OCHF₂;
R₃ Methyl;
R₄ C₁-C₂-Alkyl; durch Fluor, Chlor, Brom, Cyano, C₁-C₂-Alkoxy oder Methylthio substituiertes C₂-Alkyl;
Allyl oder Propargyl;
X Sauerstoff oder Schwefel.

8. Verbindungen der Formel 1 nach Anspruch 1, in welcher bedeuten:
R₁ und R₂ unabhängig voneinander Wasserstoff, Halogen, C₁-C₂-Alkyl, Halogenmethyl, C₁-C₂-Alkoxy oder C₁-C₂-Halogenalkoxy;
R₃ Wasserstoff, C₁-C₃-Alkyl; durch Halogen oder Hydroxy substituiertes C₁-C₂-Alkyl; Cyclopropyl oder durch Halogen und/oder Methyl ein- bis dreifach gleich oder verschieden substituiertes Cyclopropyl;
R₄ C₁-C₄-Alkyl; durch Halogen, Cyano, C₁-C₂-Alkoxy oder C₁-C₂-Alkylthio substituiertes C₂-C₃-Alkyl;
C₃-C₆-Alkenyl; C₃-C₆-Alkinyl oder C₃-C₇-Cycloalkyl;
X Sauerstoff oder Schwefel.

9. Verbindungen der Formel 1 nach Anspruch 8, in welcher bedeuten:
R₁ und R₂ unabhängig voneinander Wasserstoff, Fluor, Chlor, Brom, Methyl, Trifluormethyl, Methoxy oder Difluormethoxy;
R₃ Wasserstoff, C₁-C₃-Alkyl; durch Halogen oder Hydroxy substituiertes C₁-C₂-Alkyl; Cyclopropyl, oder durch Halogen und/oder Methyl ein- bis dreifach gleich oder verschieden substituiertes Cyclopropyl;
R₄ C₁-C₃-Alkyl; durch Fluor, Chlor oder C₁-C₂-Alkoxy substituiertes C₂-C₃-Alkyl; C₃-C₆-Alkenyl; C₃-C₆-Alkinyl oder C₃-C₆-Cycloalkyl;
X Sauerstoff oder Schwefel.

10. Verbindungen der Formel l nach Anspruch 9, in welcher bedeuten:
R₁ und R₂ unabhängig voneinander Wasserstoff, Fluor, Chlor, Methyl, Trifluormethyl, Methoxy oder Difluormethoxy;
R₃ C₁-C₃-Alkyl; durch Halogen oder Hydroxy substituiertes Methyl; Cyclopropyl oder durch Halogen oder Methyl ein- bis dreifach substituiertes Cyclopropyl;
R₄ C₁-C₃-Alkyl; durch Fluor, Chlor oder Methoxy substituiertes C₂-C₃-Alkyl; C₃-C₄-Alkenyl; C₃-C₄-Alkinyl; Cyclopropyl; Cyclohexyl;
X Sauerstoff.

11. Verbindungen der Formel nach Anspruch 10, in welcher bedeuten:
R₁ und R₂ Wasserstoff;
R₃ C₁-C₃-Alkyl; durch Fluor, Chlor oder Brom substituiertes Methyl; Cyclopropyl oder durch Chlor oder Methyl substituiertes Cyclopropyl;
R₄ C₁-C₃-Alkyl; 2-Chlorethyl, 2,2,2-Trifluorethyl; Allyl oder Propargyl;
X Sauerstoff.

12. Eine Verbindung der Formel l nach Anspruch 3, ausgewählt aus
2-Phenylamino-4-methyl-6-methoxymethyl-pyrimidin;
2-Phenylamino-4-cyclopropyl-6-methoxymethyl-pyrimidin.

13. Verfahren zur Herstellung von Verbindungen der Formel l nach Anspruch 1, dadurch gekennzeichnet, dass man
1. ein Phenylguanidinsalz der Formellla 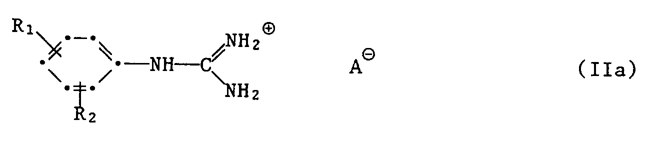 oder das Zugrundeliegende Guanidin der Formel Ilb 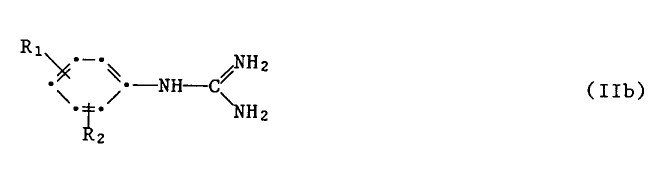 mit einem Diketon der Formel lll 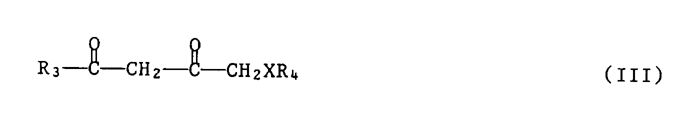 ohne Lösungsmittel oder in einem aprotischen Lösungsmittel, bevorzugt aber in einem protischen Lösungsmittel bei Temperaturen von 60 ° C bis 160 ° C umsetzt oder
2. in einem mehrstufigen Verfahren:
2.1 Harnstoff mit einem Keton der Formel lll 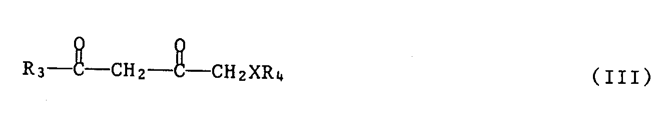 in Gegenwart einer Säure in einem inerten Lösungsmittel bei Temperaturen von 20 ° C bis 140 ° C zur Reaktion bringt und danach bei Rückflusstemperatur des verwendeten Lösungsmittels zu einer Pyrimidinverbindung der Formel V 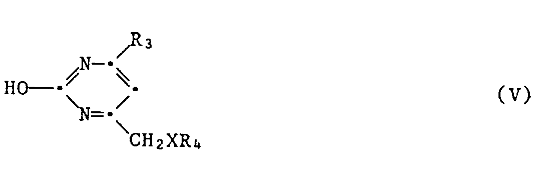 cyclisiert und
2.2 die erhaltene Verbindung der Formel V weiter mit überschüssigem POCl₃ ohne Lösungsmittel oder in einem gegen POCl3 inerten Lösungsmittel bei Temperaturen von 50 ° C bis 110 ° C zu der Verbindung der Formel VI 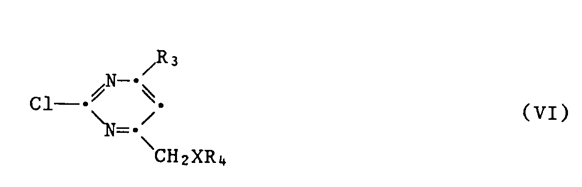 umsetzt und
2.3 die erhaltene Verbindung der Formel VI weiter mit einer Anilinverbindung der Formel VII 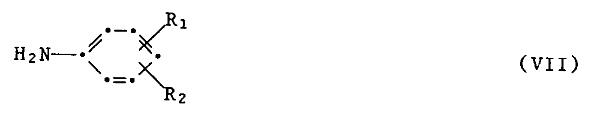 je nach Verfahrensbedingungen entweder
a) in Gegenwart eines Protonenakzeptors ohne Lösungsmittel oder in einem protischen oder aprotischen Lösungsmittel oder
b) in Gegenwart einer Säure in einem inerten Lösungsmittel bei jeweils Temperaturen von 60 ° C bis 120°C zur Reaktion bringt, oder
3. in einem zwei-stufigen Verfahren:
3.1 ein Guanidinsalz der Formel VIII 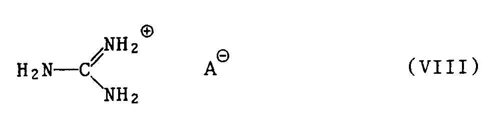 mit einem Diketon der Formel lll 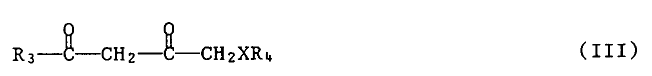
a) ohne Lösungsmittel bei Temperaturen von 100 ° C bis 160 ° C oder
b) in einem protischen oder aprotischen Lösungsmittel oder einem Gemisch aus protischen und aprotischen Lösungsmitteln bei Temperaturen von 30 ° C bis 140 ° C
zu einer Pyrimidinverbindung der Formel IX 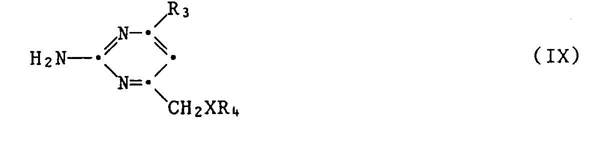 cyclisiert und
3.2 die erhaltene Verbindung der Formel IX mit einer Verbindung der Formel X 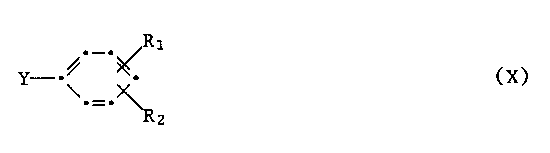 unter Abspaltung von HY in Gegenwart eines Protonenakzeptors in aprotischen Lösungsmitteln bei Temperaturen von 30 ° C bis 140 ° C umsetzt, wobei in den Formeln ll bis X die Substituenten R₁ bis R₄ und X die unter Formel I angegebenen Bedeutungen besitzen, A^{S} ein Säureanion und Y Halogen darstellen.

14. Mittel zur Bekämpfung oder Verhütung eines Befalls durch schädliche Insekten oder Mikroorganismen, dadurch gekennzeichnet, dass es als mindestens eine aktive Komponente eine Verbindung der Formel I gemäss Anspruch 1 enthält.

15. Mittel gemäss Anspruch 14, dadurch gekennzeichnet, dass es als mindestens eine aktive Komponente eine Verbindung der Formel I gemäss Anspruch 2 enthält.

16. Mittel gemäss Anspruch 14, dadurch gekennzeichnet, dass es als mindestens eine aktive Komponente eine Verbindung der Formel I gemäss Anspruch 3 enthält.

17. Mittel gemäss Anspruch 14, dadurch gekennzeichnet, dass es als mindestens eine aktive Komponente eine Verbindung der Formel I gemäss einem der Ansprüche 4 bis 12 enthält.

18. Mittel nach Anspruch 14, dadurch gekennzeichnet, dass es 0,1 bis 99 Gew.-% eines Wirkstoffs der Formel I, 99,9 bis 1 Gew.-% eines festen oder flüssigen Zusatzstoffes und 0 bis 25 Gew.-% eines Tensides enthält.

19. Verfahren zur Herstellung eines wie in Anspruch 14 definierten agrochemischen Mittels, dadurch gekennzeichnet, dass man mindestens eine gemäss Anspruch 1 definierte Verbindung der Formel I mit geeigneten festen oder flüssigen Zusatzstoffen und Tensiden innig vermischt.

20. Verfahren zur Bekämpfung oder Verhütung eines Befalls von Kulturpflanzen durch Schadinsekten oder phytopathogene Mikroorganismen, dadurch gekennzeichnet, dass man eine gemäss Anspruch 1 definierte Verbindung der Formel I auf die Pflanze oder deren Standort appliziert.

21. Verwendung von Verbindungen der Formel I gemäss Anspruch 1 zur Bekämpfung und/oder präventiven Verhütung eines Befalls von schädlichen Mikroorganismen.

22. Verwendung gemäss Anspruch 21, dadurch gekennzeichnet, dass es sich bei den Mikroorganismen um phytopathogene Pilze handelt.

23. Verwendung gemäss Anspruch 22 gegen Pilze aus der Klasse der Fungi imperfecti.

24. Verwendung gemäss Anspruch 23 gegen Botrytis-Befall.

## Patentansprüche (Patentansprüche für folgende(n) Vertragsstaat(en) : ES)

1. Verfahren zur Herstellung von Verbindungen der Formel I 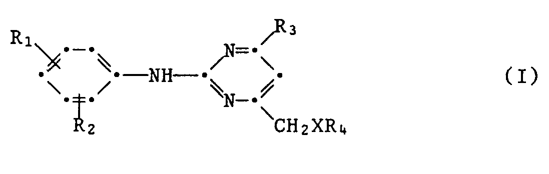 in welcher bedeuten:
R₁ und R₂ unabhängig voneinander Wasserstoff, Halogen, C1-C3-Alkyl, C1-C2-Halogenalkyl, C₁-C₃-Alkoxy oder C₁ -C₃ Halogenalkoxy;
R₃ Wasserstoff, C₁-C₄-Alkyl, durch Halogen oder Hydroxy substituiertes C₁-C₂-Alkyl, Cyclopropyl oder durch Halogen und/oder Methyl substituiertes Cyclopropyl;
R₄ C₁-C₈-Alkyl; durch Halogen, Hydroxy, Cyano, C1-C4-Alkoxy, C₃-C₄-Alkoxyalkoxy oder C₁-C₃-Alkylthio substituiertes C₂-C₆-Alkyl; C₃-C₆-Alkenyl; C₃-C₆-Alkinyl; C₃-C₇-Cycloalkyl oder durch Methyl substituiertes C₃-C₇-Cycloalkyl;
X Sauerstoff oder Schwefel,
dadurch gekennzeichnet, dass man
1. ein Phenylguanidinsalz der Formellla 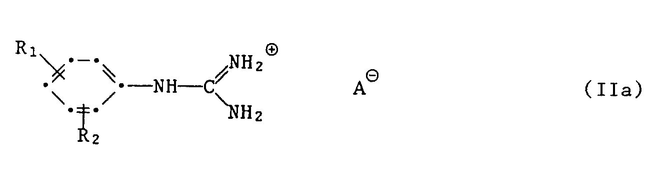 oder das zugrundeliegende Guanidin der Formel Ilb 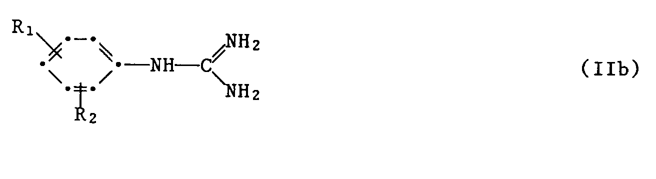 mit einem Diketon der Formel lll 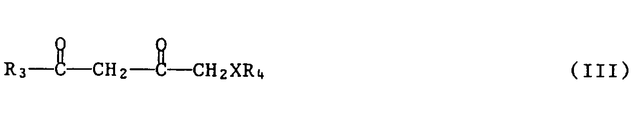 ohne Lösungsmittel oder in einem aprotischen Lösungsmittel, bevorzugt aber in einem protischen Lösungsmittel bei Temperaturen von 60 ° C bis 160 ° C umsetzt oder
2. in einem mehrstufigen Verfahren:
2.1 Harnstoff mit einem Keton der Formel lll 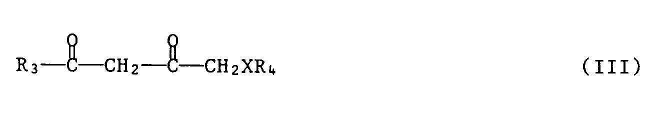 in Gegenwart einer Säure in einem inerten Lösungsmittel bei Temperaturen von 20 ° C bis 140 ° C zur Reaktion bringt und danach bei Rückflusstemperatur des verwendeten Lösungsmittels zu einer Pyrimidinverbindung der Formel V 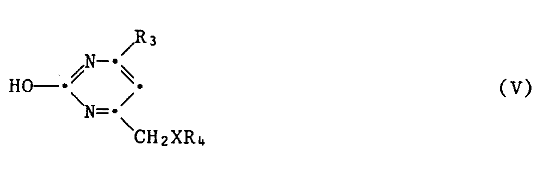 cyclisiert und
2.2 die erhaltene Verbindung der Formel V weiter mit überschüssigem POCl₃ ohne Lösungsmittel oder in einem gegen POCl3 inerten Lösungsmittel bei Temperaturen von 50 ° C bis 110 ° C zu der Verbindung der Formel VI 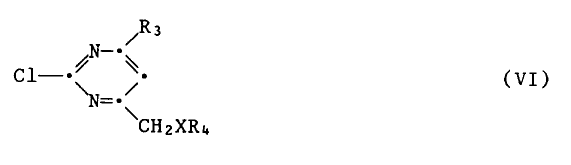 umsetzt und
2.3 die erhaltene Verbindung der Formel VI weiter mit einer Anilinverbindung der Formel VII 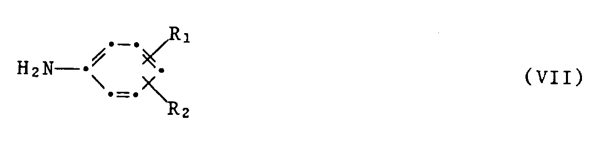 je nach Verfahrensbedingungen entweder
a) in Gegenwart eines PrXtonenakzeptors ohne Lösungsmittel oder in einem protischen oder aprotischen Lösungsmittel oder
b) in Gegenwart einer Säure in einem inerten Lösungsmittel bei jeweils Temperaturen von 60 ° C bis 120°C zur Reaktion bringt, oder
3. in einem zweistufigen Verfahren:
3.1 ein Guanidinsalz der Formel VIII 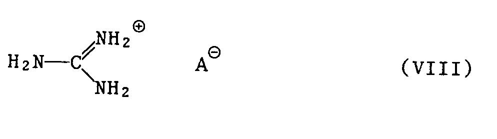 mit einem Diketon der Formel lll 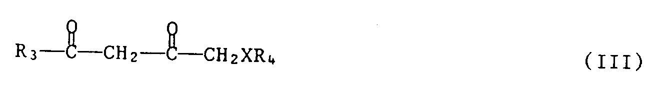
a) ohne Lösungsmittel bei Temperaturen von 100 ° C bis 160 ° C oder
b) in einem protischen oder aprotischen Lösungsmittel oder einem Gemisch aus protischen und aprotischen Lösungsmitteln bei Temperaturen von 30 ° C bis 140 ° C
zu einer Pyrimidinverbindung der Formel IX 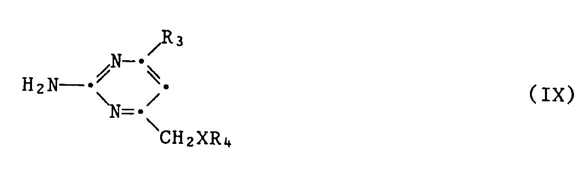 cyclisiert und
3.2 die erhaltene Verbindung der Formel IX mit einer Verbindung der Formel X 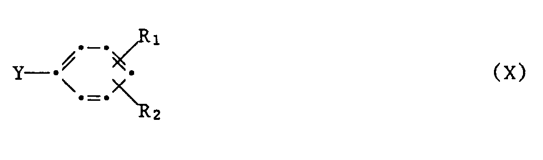 unter Abspaltung von HY in Gegenwart eines Protonenakzeptors in aprotischen Lösungsmitteln bei Temperaturen von 30 ° C bis 140 ° C umsetzt, wobei in den Formeln ll bis X die Substituenten R₁ bis R₄ und X die unter Formel I angegebenen Bedeutungen besitzen, A^{S} ein Säureanion und Y Halogen darstellen.

2. Verfahren gemäss Anspruch 1 zur Herstellung der Verbindungen der Formel I, worin R₃, R₄ und X die genannten Bedeutungen haben und R₁ und R₂ Wasserstoff darstellen.

3. Verfahren gemäss Anspruch 1 zur Herstellung der Verbindungen der Formel I, worin bedeuten:
R₁ und R₂ unabhängig voneinander Wasserstoff, Halogen, C₁-C₃-Alkyl, C₁-C₂-Halogenalkyl, C₁-C₃-Alkoxy oder C₁ -C₃ Halogenalkoxy;
R₃ Wasserstoff, C₁-C₄-Alkyl oder Cyclopropyl;
R₄ C₁-C₈-Alkyl; durch Halogen, Hydroxy, Cyano, C₁-C₄-Alkoxy, C₃-C₄-Alkoxyalkoxy oder C₁-C₃-Alkylthio substituiertes C₂-C₆-Alkyl; C₃-C₆-Alkenyl; C₃-C₆-Alkinyl; C₅-C₇-Cycloalkyl oder durch Methyl substituiertes C₅-C₇-Cycloalkyl;
X Sauerstoff oder Schwefel.

4. Verfahren gemäss Anspruch 1 zur Herstellung der Verbindungen der Formel I, worin bedeuten:
R₁ und R₂ unabhängig voneinander Wasserstoff, Fluor, Chlor, Brom, C₁-C₃-Alkyl, C₁-C₂-Halogenalkyl, C₁-C₃-Alkoxy oder C₁-C₂ -Halogenalkoxy;
R₃ Wasserstoff, C₁-C₃-Alkyl oder Cyclopropyl;
R₄ C₁-C₆-Alkyl; durch Halogen, Hydroxy, Cyano, C₁-C₃-Alkoxy, C₃-C₄-Alkoxyalkoxy oder Ci-C₂-Alkylthio substituiertes C₂-C₄-Alkyl; C₃-Alkenyl; C₃-Alkinyl; oder unsubstituiertes oder durch Methyl substituiertes C₆-C₇-Cycloalkyl;
X Sauerstoff oder Schwefel.

5. Verfahren gemäss Anspruch 1 zur Herstellung der Verbindungen der Formel I, worin bedeuten:
R₁ und R₂ unabhängig voneinander Wasserstoff, Fluor, Chlor, Brom, C₁-C₃-Alkyl, C₁-C₃-Alkoxy, Trifluormethyl, -OCHF₂, -OCF₂CHF₂, -OCF₂CHCIF, -OCF₂CHCI₂ oder -OCF₂CCI₂F;
R₃ Wasserstoff, Methyl, Ethyl oder n-Propyl;
R₄ C₁-C₄-Alkyl; durch Halogen, Hydroxy, Cyano, C₁-C₂-Alkoxy oder C₁-C₂-Alkylthio substituiertes C₂-C₃-Alkyl; C₃-C₆-Alkenyl; C₃-C₆-Alkinyl oder Cyclohexyl;
X Sauerstoff oder Schwefel.

6. Verfahren gemäss Anspruch 1 zur Herstellung der Verbindungen der Formel I, worin bedeuten:
R₁ und R₂ unabhängig voneinander Wasserstoff, Fluor, Chlor, Brom, C₁-C₂-Alkyl, C₁-C₂-Alkoxy, Trifluormethyl, -OCHF₂, -OCF₃, -OCF₂CHF₂ oder -OCF₂CHCIF;
R₃ Wasserstoff oder C₁-C₂-Alkyl;
R₄ C₁-C₃-Alkyl; durch Halogen, Hydroxy, Cyano, C₁-C₂-Alkoxy oder C₁-C₂-Alkylthio substituiertes C₂-Alkyl; C₃-C₄-Alkenyl; C₃-C₄-Alkinyl;
X Sauerstoff oder Schwefel.

7. Verfahren gemäss Anspruch 1 zur Herstellung der Verbindungen der Formel I, worin bedeuten:
R₁ und R₂ unabhängig voneinander Wasserstoff, Fluor, Chlor, Brom, C₁-C₂-Alkyl, C₁-C₂-Alkoxy, Trifluormethyl oder -OCHF₂;
R₃ Methyl;
R₄ C₁-C₂-Alkyl; durch Fluor, Chlor, Brom, Cyano, C₁-C₂-Alkoxy oder Methylthio substituiertes C₂-Alkyl; Allyl oder Propargyl;
X Sauerstoff oder Schwefel.

8. Verfahren gemäss Anspruch 1 zur Herstellung der Verbindungen der Formel I, worin bedeuten:
R₁ und R₂ unabhängig voneinander Wasserstoff, Halogen, C₁-C₂-Alkyl, Halogenmethyl, C₁-C₂-Alkoxy oder C₁-C₂-Halogenalkoxy;
R₃ Wasserstoff, C₁-C₃-Alkyl; durch Halogen oder Hydroxy substituiertes C₁-C₂-Alkyl; Cyclopropyl oder durch Halogen und/oder Methyl ein- bis dreifach gleich oder verschieden substituiertes Cyclopropyl;
R₄ C₁-C₄-Alkyl; durch Halogen, Cyano, C₁-C₂-Alkoxy oder C₁-C₂-Alkylthio substituiertes C₂-C₃-Alkyl;
C₃-C₆-Alkenyl; C₃-C₆-Alkinyl oder C₃-C₇-Cycloalkyl;
X Sauerstoff oder Schwefel.

9. Verfahren gemäss Anspruch 1 zur Herstellung der Verbindungen der Formel I, worin bedeuten:
R₁ und R₂ unabhängig voneinander Wasserstoff, Fluor, Chlor, Brom, Methyl, Trifluormethyl, Methoxy oder Difluormethoxy;
R₃ Wasserstoff, C₁-C₃-Alkyl; durch Halogen oder Hydroxy substituiertes C₁-C₂-Alkyl; Cyclopropyl, oder durch Halogen und/oder Methyl ein- bis dreifach gleich oder verschieden substituiertes Cyclopropyl;
R₄ C₁-C₃-Alkyl; durch Fluor, Chlor oder C₁-C₂-Alkoxy substituiertes C₂-C₃-Alkyl; C3-C6-Alkenyl; C₃-C₆-Alkinyl oder C₃-C₆-Cycloalkyl;
X Sauerstoff oder Schwefel.

10. Verfahren gemäss Anspruch 1 zur Herstellung der Verbindungen der Formel I, worin bedeuten:
R₁ und R₅ unabhängig voneinander Wasserstoff, Fluor, Chlor, Methyl, Trifluormethyl, Methoxy oder Difluormethoxy;
R₃ C₁-C₃-Alkyl; durch Halogen oder Hydroxy substituiertes Methyl; Cyclopropyl oder durch Halogen oder Methyl ein- bis dreifach substituiertes Cyclopropyl;
R₄ C₁-C₃-Alkyl; durch Fluor, Chlor oder Methoxy substituiertes C₂-C₃-Alkyl; C₃-C₄-Alkenyl; C₃-C₄-Alkinyl; Cyclopropyl; Cyclohexyl;
X Sauerstoff.

11. Verfahren gemäss Anspruch 1 zur Herstellung der Verbindungen der Formel 1, worin bedeuten:
R₁ und R₂ Wasserstoff;
R₃ C₁-C₃-Alkyl; durch Fluor, Chlor oder Brom substituiertes Methyl; Cyclopropyl oder durch Chlor oder Methyl substituiertes Cyclopropyl;
R₄ C₁-C₃-Alkyl; 2-Chlorethyl, 2,2,2-Trifluorethyl; Allyl oder Propargyl;
X Sauerstoff.

12. Verfahren gemäss Anspruch 1 zur Herstellung einer Verbindung der Formel I, ausgewählt aus
2-Phenylamino-4-methyl-6-methoxymethyl-pyrimidin;
2-Phenylamino-4-cyclopropyl-6-methoxymethyl-pyrimidin.

## Patentansprüche (Patentansprüche für folgende(n) Vertragsstaat(en) : CH, DE, FR, GB, IT, LI, NL)

1. Verbindungen der Formel I 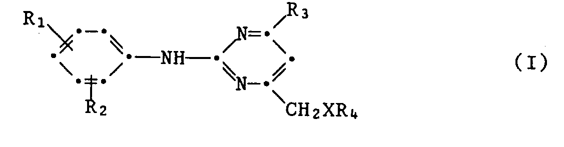 in welcher bedeuten:
R₁ und R₂ unabhängig voneinander Wasserstoff, Halogen, C₁-C₃-Alkyl, C₁-C₂-Halogenalkyl, C₁-C₃-Alkoxy oder C₁ -C₃ Halogenalkoxy;
R₃ Wasserstoff, C₁-C₄-Alkyl, durch Halogen oder Hydroxy substituiertes C₁-C₂-Alkyl, Cyclopropyl oder durch Halogen und/oder Methyl substituiertes Cyclopropyl;
R₄ C₁-C₈-Alkyl; durch Halogen, Hydroxy, Cyano, C₁-C₄-Alkoxy, C₃-C₄-Alkoxyalkoxy oder C₁-C₃-Alkylthio substituiertes C₂-C₆-Alkyl; C₃-C₆-Alkenyl; C₃-C₆-Alkinyl; C₃-C₇-Cycloalkyl oder durch Methyl substituiertes C₃-C₇-Cycloalkyl;
X Sauerstoff oder Schwefel;
wobei, wenn R₁ Wasserstoff, R₂ Wasserstoff, Halogen, C₁-C₃-Alkyl, C₁-C₃-Alkoxy oder C₁-C₂-Halogenalkyl und R₃ Halogen, C₁-C₄-Alkyl oder C₁-C₂-Halogenalkyl ist, R₄ im Falle X = Schwefel von Alkyl verschieden ist, und R₄ im Falle X = Sauerstoff von Alkyl, Alkenyl und Alkinyl verschieden ist.

2. Verbindungen der Formel I gemäss Anspruch 1, worin R₃, R₄ und X die genannte Bedeutung haben und R₁ und R₂ Wasserstoff darstellen.

3. Verbindungen der Formel I gemäss Anspruch 1, in welcher bedeuten:
R₁ und R₂ unabhängig voneinander Wasserstoff, Halogen, C₁-C₃-Alkyl, C₁-C₂-Halogenalkyl, Ci-C₃-Alkoxy oder C₁ -C₃ Halogenalkoxy;
R₃ Wasserstoff, C₁-C₄-Alkyl oder Cyclopropyl;
R₄ C₁-C₈-Alkyl; durch Halogen, Hydroxy, Cyano, C₁-C₄-Alkoxy, C₃-C₄-Alkoxyalkoxy oder C₁-C₃-Alkylthio substituiertes C₂-C₆-Alkyl; C₃-C₆-Alkenyl; C₃-C₆-Alkinyl; C₅-C₇-Cycloalkyl oder durch Methyl substituiertes C₅-C₇-Cycloalkyl;
X Sauerstoff oder Schwefel.

4. Verbindungen der Formel 1 nach Anspruch 3, in welcher bedeuten:
R₁ und R₂ unabhängig voneinander Wasserstoff, Fluor, Chlor, Brom, C₁-C₃-Alkyl, C₁-C₂-Halogenalkyl, C₁-C₃-Alkoxy oder C₁-C₂-Halogenalkoxy;
R₃ Wasserstoff, C₁-C₃-Alkyl oder Cyclopropyl;
R₄ C₁-C₆-Alkyl; durch Halogen, Hydroxy, Cyano, C₁-C₃-Alkoxy, C₃-C₄-Alkoxyalkoxy oder C₁-C₂-Alkylthio substituiertes C₂-C₄-Alkyl; C₃-Alkenyl; C₃-Alkinyl; oder unsubstituiertes oder durch Methyl substituiertes C₆-C₇-Cycloalkyl;
X Sauerstoff oder Schwefel.

5. Verbindungen der Formel 1 nach Anspruch 3, in welcher bedeuten:
R₁ und R₂ unabhängig voneinander Wasserstoff, Fluor, Chlor, Brom, C₁-C₃-Alkyl, C₁-C₃-Alkoxy, Trifluormethyl, -OCHF₂, -OCF₂CHF₂, -OCF₂CHCIF, -OCF₂CHCI₂ oder -OCF₂CCI₂F;
R₃ Wasserstoff, Methyl, Ethyl oder n-Propyl;
R₄ C₁-C₄-Alkyl; durch Halogen, Hydroxy, Cyano, C₁-C₂-Alkoxy oder C₁-C₂-Alkylthio substituiertes C₂-C₃-Alkyl; C₃-C₆-Alkenyl; C₃-C₆-Alkinyl oder Cyclohexyl;
X Sauerstoff oder Schwefel.

6. Verbindungen der Formel 1 nach Anspruch 4, in welcher bedeuten:
R₁ und R₂ unabhängig voneinander Wasserstoff, Fluor, Chlor, Brom, C₁-C₂-Alkyl, C₁-C₂-Alkoxy, Trifluormethyl, -OCHF₂, -OCF₃, -OCF₂CHF₂ oder -OCF₂CHCIF;
R₃ Wasserstoff oder C₁-C₂-Alkyl;
R₄ C₁-C₃-Alkyl; durch Halogen, Hydroxy, Cyano, C₁-C₂-Alkoxy oder C₁-C₂-Alkylthio substituiertes C₂-Alkyl; C₃-C₄-Alkenyl; C₃-C₄-Alkinyl;
X Sauerstoff oder Schwefel.

7. Verbindungen der Formel I nach Anspruch 6, in welcher bedeuten:
R₁ und R₂ unabhängig voneinander Wasserstoff, Fluor, Chlor, Brom, C₁-C₂-Alkyl, C₁-C₂-Alkoxy, Trifluormethyl oder -OCHF₂;
R₃ Methyl;
R₄ C₁-C₂-Alkyl; durch Fluor, Chlor, Brom, Cyano, C₁-C₂-Alkoxy oder Methylthio substituiertes C₂-Alkyl; Allyl oder Propargyl;
X Sauerstoff oder Schwefel.

8. Verbindungen der Formel I nach Anspruch 1, in welcher bedeuten:
R₁ und R₂ unabhängig voneinander Wasserstoff, Halogen, C₁-C₂-Alkyl, Halogenmethyl, C₁-C₂-Alkoxy oder C₁-C₂-Halogenalkoxy;
R₃ Wasserstoff, C₁-C₃-Alkyl; durch Halogen oder Hydroxy substituiertes C₁-C₂-Alkyl; Cyclopropyl oder durch Halogen und/oder Methyl ein- bis dreifach gleich oder verschieden substituiertes Cyclopropyl;
R₄ C₁-C₄-Alkyl; durch Halogen, Cyano, C₁-C₂-Alkoxy oder C₁-C₂-Alkylthio substituiertes C₂-C₃-Alkyl;
C₃-C₆-Alkenyl; C₃-C₆-Alkinyl oder C₃-C₇-Cycloalkyl;
X Sauerstoff oder Schwefel.

9. Verbindungen der Formel I nach Anspruch 8, in welcher bedeuten:
R₁ und R₂ unabhängig voneinander Wasserstoff, Fluor, Chlor, Brom, Methyl, Trifluormethyl, Methoxy oder Difluormethoxy;
R₃ Wasserstoff, C₁-C₃-Alkyl; durch Halogen oder Hydroxy substituiertes C₁-C₂-Alkyl; Cyclopropyl, oder durch Halogen und/oder Methyl ein- bis dreifach gleich oder verschieden substituiertes Cyclopropyl;
R₄ C₁-C₃-Alkyl; durch Fluor, Chlor oder C₁-C₂-Alkoxy substituiertes C₂-C₃-Alkyl; C₃-C₆-Alkenyl; C₃-C₆-Alkinyl oder C₃-C₆-Cycloalkyl;
X Sauerstoff oder Schwefel.

10. Verbindungen der Formel 1 nach Anspruch 9, in welcher bedeuten:
R₁ und R₂ unabhängig voneinander Wasserstoff, Fluor, Chlor, Methyl, Trifluormethyl, Methoxy oder Difluormethoxy;
R₃ C₁-C₃-Alkyl; durch Halogen oder Hydroxy substituiertes Methyl; Cyclopropyl oder durch Halogen oder Methyl ein- bis dreifach substituiertes Cyclopropyl;
R₄ C₁-C₃-Alkyl; durch Fluor, Chlor oder Methoxy substituiertes C₂-C₃-Alkyl; C₃-C₄-Alkenyl; C₃-C₄-Alkinyl; Cyclopropyl; Cyclohexyl;
X Sauerstoff.

11. Verbindungen der Formel nach Anspruch 10, in welcher bedeuten:
R₁ und R₂ Wasserstoff;
R₃ C₁-C₃-Alkyl; durch Fluor, Chlor oder Brom substituiertes Methyl; Cyclopropyl oder durch Chlor oder Methyl substituiertes Cyclopropyl;
R₄ C₁-C₃-Alkyl; 2-Chlorethyl, 2,2,2-Trifluorethyl; Allyl oder Propargyl;
X Sauerstoff.

12. Eine Verbindung der Formel I nach Anspruch 3 2-Phenylamino-4-cyclopropyl-6-methoxymethyl-pyrimidin.

13. Verfahren zur Herstellung von Verbindungen der Formel I nach Anspruch 1, dadurch gekennzeichnet, dass man
1. ein Phenylguanidinsalz der Formel Ila 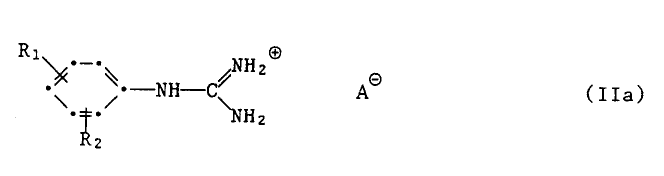 oder das zugrundeliegende Guanidin der Formel Ilb 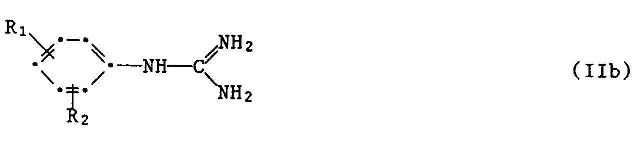 mit einem Diketon der Formel III 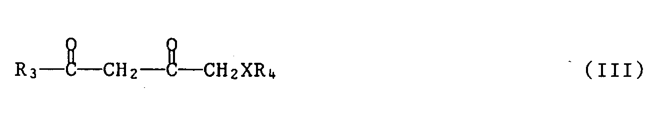 ohne Lösungsmittel oder in einem aprotischen Lösungsmittel, bevorzugt aber in einem protischen Lösungsmittel bei Temperaturen von 60°C bis 160°C umsetzt oder
2. in einem mehrstufigen Verfahren:
2.1 Harnstoff mit einem Keton der Formel III 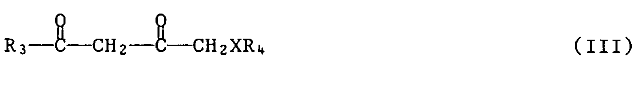 in Gegenwart einer Säure in einem inerten Lösungsmittel bei Temperaturen von 20°C bis 140°C zur Reaktion bringt und danach bei Rückflusstemperatur des verwendeten Lösungsmittels zu einer Pyrimidinverbindung der Formel V 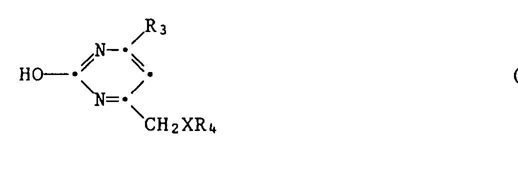 cyclisiert und
2.2 die erhaltene Verbindung der Formel V weiter mit überschüssigem POCl₃ ohne Lösungsmittel oder in einem gegen POCl₃ inerten Lösungsmittel bei Temperaturen von 50°C bis 110°C zu der Verbindung der Formel VI 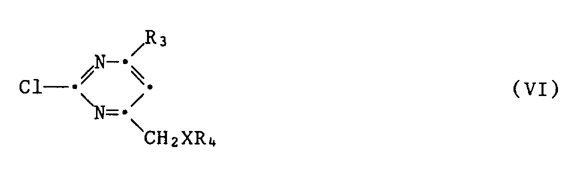 umsetzt und
2.3 die erhaltene Verbindung der Formel VI weiter mit einer Anilinverbindung der Formel VII 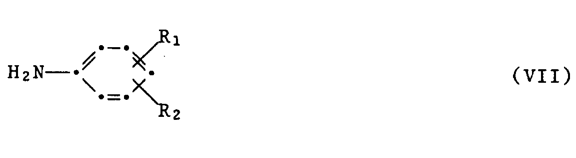 je nach Verfahrensbedingungen entweder
a) in Gegenwart eines Protonenakzeptors ohne Lösungsmittel oder in einem protischen oder aprotischen Lösungsmittel oder
b) in Gegenwart einer Säure in einem inerten Lösungsmittel bei jeweils Temperaturen von 60°C bis 120°C zur Reaktion bringt, oder
3. in einem zwei-stufigen Verfahren:
3.1 ein Guanidinsalz der Formel VIII 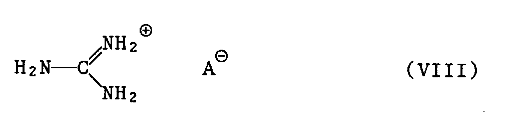 mit einem Diketon der Formel III 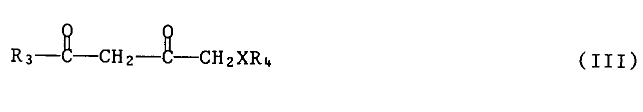
a) ohne Lösungsmittel bei Temperaturen von 100°C bis 160°C oder
b) in einem protischen oder aprotischen Lösungsmittel oder einem Gemisch aus protischen und aprotischen Lösungsmitteln bei Temperaturen von 30°C bis 140°C
zu einer Pyrimidinverbindung der Formel IX 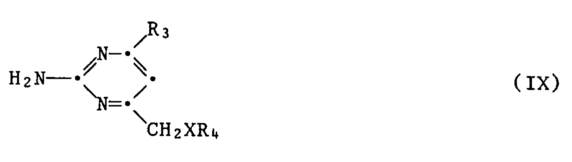 cyclisiert und
3.2 die erhaltene Verbindung der Formel IX mit einer Verbindung der Formel X 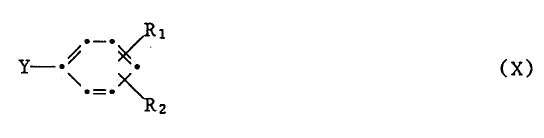 unter Abspaltung von HY in Gegenwart eines Protonenakzeptors in aprotischen Lösungsmitteln bei Temperaturen von 30 °C bis 140°C umsetzt, wobei in den Formeln 11 bis X die Substituenten R₁ bis R₄ und X die unter Formel I angegebenen Bedeutungen besitzen, A^{S} ein Säureanion und Y Halogen darstellen.

14. Mittel zur Bekämpfung oder Verhütung eines Befalls durch schädliche Insekten oder Mikroorganismen, dadurch gekennzeichnet, dass es als mindestens eine aktive Komponente eine Verbindung der Formel I gemäss Anspruch 1 enthält.

15. Mittel gemäss Anspruch 14, dadurch gekennzeichnet, dass es als mindestens eine aktive Komponente eine Verbindung der Formel I gemäss Anspruch 2 enthält.

16. Mittel gemäss Anspruch 14, dadurch gekennzeichnet, dass es als mindestens eine aktive Komponente eine Verbindung der Formel I gemäss Anspruch 3 enthält.

17. Mittel gemäss Anspruch 14, dadurch gekennzeichnet, dass es als mindestens eine aktive Komponente eine Verbindung der Formel I gemäss einem der Ansprüche 4 bis 12 enthält.

18. Mittel nach Anspruch 14, dadurch gekennzeichnet, dass es 0,1 bis 99 Gew.-% eines Wirkstoffs der Formel I, 99,9 bis 1 Gew.-% eines festen oder flüssigen Zusatzstoffes und 0 bis 25 Gew.-% eines Tensides enthält.

19. Verfahren zur Herstellung eines wie in Anspruch 14 definierten agrochemischen Mittels, dadurch gekennzeichnet, dass man mindestens eine gemäss Anspruch 1 definierte Verbindung der Formel I mit geeigneten festen oder flüssigen Zusatzstoffen und Tensiden innig vermischt.

20. Verfahren zur Bekämpfung oder Verhütung eines Befalls von Kulturpflanzen durch Schadinsekten oder phytopathogene Mikroorganismen, dadurch gekennzeichnet, dass man eine gemäss Anspruch 1 definierte Verbindung der Formel I auf die Pflanze oder deren Standort appliziert.

21. Verwendung von Verbindungen der Formel I gemäss Anspruch 1 zur Bekämpfung und/oder präventiven Verhütung eines Befalls von schädlichen Mikroorganismen.

22. Verwendung gemäss Anspruch 21, dadurch gekennzeichnet, dass es sich bei den Mikroorganismen um phytopathogene Pilze handelt.

23. Verwendung gemäss Anspruch 22 gegen Pilze aus der Klasse der Fungi imperfecti.

24. Verwendung gemäss Anspruch 23 gegen Botrytis-Befall.

## Claims (Claims for the following Contracting State(s) : AT, BE, GR, LU, SE)

1. A compound of formula I 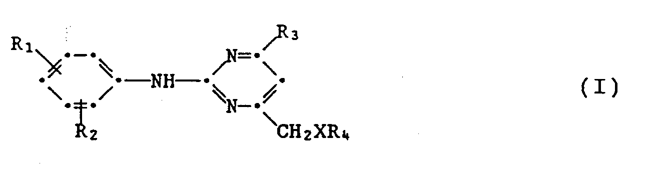 in which:
R₁ and R₂ independently of one another are hydrogen, halogen, C₁-C₃alkyl, C₁-C₂haloalkyl, C₁-C₃alkoxy or C₁-C₃haloalkoxy;
R₃ is hydrogen; C₁-C₄alkyl; C₁-C₂alkyl substituted by halogen or by hydroxy; cyclopropyl; or cyclopropyl substituted by halogen and/or by methyl;
R₄ is C₁-C₈alkyl; C₂-C₆alkyl substituted by halogen, hydroxy, cyano, C₁-C₄alkoxy, C3-C4alkoxyalkoxy or by C₁-C₃alkylthio; C₃-C₆alkenyl; C₃-C₆alkynyl; C₃-C₇cycloalkyl; or C₃-C₇cycloalkyl substituted by methyl; and
X is oxygen or sulfur.

2. A compound of formula I according to claim 1 in which R₃, R₄ and X are as defined and R₁ and R₂ are hydrogen.

3. A compound of formula I according to claim 1 in which:
R₁ and R₂ independently of one another are hydrogen, halogen, C₁-C₃alkyl, C₁-C₂haloalkyl, C₁-C₃alkoxy or C₁-C₃haloalkoxy;
R₃ is hydrogen, C₁-C₄alkyl or cyclopropyl;
R₄ is C₁-C₈alkyl; C₂-C₆alkyl substituted by halogen, hydroxy, cyano, C₁-C₄alkoxy, C3-C4alkoxyalkoxy or by C₁-C₃alkylthio; C₃-C₆alkenyl; C₃-C₆alkynyl; C₅-C₇cycloalkyl; or C₅-C₇cycloalkyl substituted by methyl; and
X is oxygen or sulfur.

4. A compound of formula I according to claim 3 in which:
R₁ and R₂ independently of one another are hydrogen, fluorine, chlorine, bromine, C₁-C₃alkyl, Ci-C₂haloalkyl, C₁-C₃alkoxy or C₁-C₂haloalkoxy;
R₃ is hydrogen, C₁-C₃alkyl or cyclopropyl;
R₄ is C₁-C₆alkyl; C₂-C₄alkyl substituted by halogen, hydroxy, cyano, C₁-C₃alkoxy, C3-C4alkoxyalkoxy or by C₁-C₂alkylthio; C₃alkenyl; C₃alkynyl; or C₆-C₇cycloalkyl that is unsubstituted or substituted by methyl; and
X is oxygen or sulfur.

5. A compound of formula I according to claim 3 in which:
R₁ and R₂ independently of one another are hydrogen, fluorine, chlorine, bromine, C₁-C₃alkyl, Ci-C₃alkoxy, trifluoromethyl, -OCHF₂, -OCF₂CHF₂, -OCF₂CHCIF, -OCF₂CHCI₂ or -OCF₂CCI₂F;
R₃ is hydrogen, methyl, ethyl or n-propyl;
R₄ is C₁-C₄alkyl; C₂-C₃alkyl substituted by halogen, hydroxy, cyano, C₁-C₂alkoxy or by C₁-C₂alkylthio;
C₃-C₆alkenyl; C₃-C₆alkynyl; or cyclohexyl; and
X is oxygen or sulfur.

6. A compound of formula I according to claim 4 in which:
R₁ and R₂ independently of one another are hydrogen, fluorine, chlorine, bromine, C₁-C₂alkyl, Ci-C₂alkoxy, trifluoromethyl, -OCHF₂, -OCF₃, -OCF₂CHF₂ or -OCF₂CHCIF;
R₃ is hydrogen or C₁-C₂alkyl;
R₄ is C₁-C₃alkyl; C₂alkyl substituted by halogen, hydroxy, cyano, C₁-C₂alkoxy or by C₁-C₂alkylthio; C₃-C₄alkenyl; or C3-C4alkynyl; and
X is oxygen or sulfur.

7. A compound of formula I according to claim 6 in which:
R₁ and R₂ independently of one another are hydrogen, fluorine, chlorine, bromine, C1-C2alkyl, Ci-C₂alkoxy, trifluoromethyl or -OCHF₂;
R₃ is methyl;
R₄ is C₁-C₂alkyl; C₂alkyl substituted by fluorine, chlorine, bromine, cyano, C₁-C₂alkoxy or by methylthio; allyl; or propargyl; and
X is oxygen or sulfur.

8. A compound of formula I according to claim 1 in which:
R₁ and R₂ independently of one another are hydrogen, halogen, C₁-C₂alkyl, halomethyl, C₁-C₂alkoxy or C₁-C₂haloalkoxy;
R₃ is hydrogen; C₁-C₃alkyl; C₁-C₂alkyl substituted by halogen or by hydroxy; cyclopropyl; or cyclopropyl mono- to tri-substituted by the same or different substituents selected from the group consisting of halogen and methyl;
R₄ is C₁-C₄alkyl; C₂-C₃alkyl substituted by halogen, cyano, C₁-C₂alkoxy or by C₁-C₂alkylthio; C₃-C₆alkenyl; C₃-C₆alkynyl; or C₃-C₇cycloalkyl; and
X is oxygen or sulfur.

9. A compound of formula I according to claim 8 in which:
R₁ and R₂ independently of one another are hydrogen, fluorine, chlorine, bromine, methyl, trifluoromethyl, methoxy or difluoromethoxy;
R₃ is hydrogen; C₁-C₃alkyl; C₁-C₂alkyl substituted by halogen or by hydroxy; cyclopropyl; or cyclopropyl mono- to tri-substituted by the same or different substituents selected from the group consisting of halogen and methyl;
R₄ is C₁-C₃alkyl; C₂-C₃alkyl substituted by fluorine, chlorine or by C₁-C₂alkoxy; C₃-C₆alkenyl; C₃-C₆alkynyl; or C₃-C₆cydoalkyl; and
X is oxygen or sulfur.

10. A compound of formula I according to claim 9 in which:
R₁ and R₂ independently of one another are hydrogen, fluorine, chlorine, methyl, trifluoromethyl, methoxy or difluoromethoxy;
R₃ is C₁-C₃alkyl; methyl substituted by halogen or by hydroxy; cyclopropyl; or cyclopropyl mono- to tri-substituted by halogen or by methyl;
R₄ is C₁-C₃alkyl; C₂-C₃alkyl substituted by fluorine, chlorine or by methoxy; C3-C4alkenyl; C₃-C₄alkynyl; cyclopropyl; or cyclohexyl; and
X is oxygen.

11. A compound of formula I according to claim 10 in which:
R₁ and R₂ are hydrogen;
R₃ is C₁-C₃alkyl; methyl substituted by fluorine, chlorine or by bromine; cyclopropyl; or cyclopropyl substituted by chlorine or by methyl;
R₄ is C1-C3alkyl, 2-chloroethyl, 2,2,2-trifluoroethyl, allyl or propargyl; and
X is oxygen.

12. A compound of formula I according to claim 3 selected from
2-phenylamino-4-methyl-6-methoxymethylpyrimidine;
2-phenylamino-4-cyclopropyl-6-methoxymethylpyrimidine.

13. A process for the preparation of a compound of formula I according to claim 1, which comprises
1. reacting a phenylguanidine salt of formula Ila 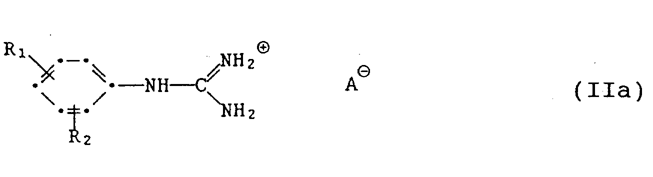 or the free guanidine base of formula IIb 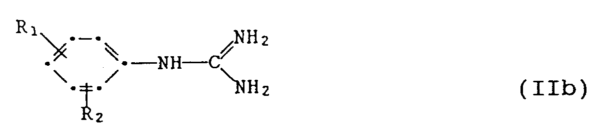 with a diketone of formula III 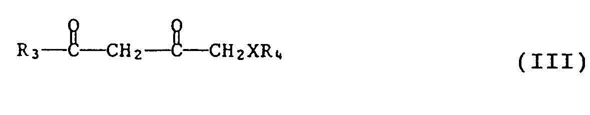 without a solvent or in an aprotic solvent, but preferably in a protic solvent, at temperatures of from 60°C to 160°C, or
2. in a multi-stage process:
2.1 reacting urea with a ketone of formula III 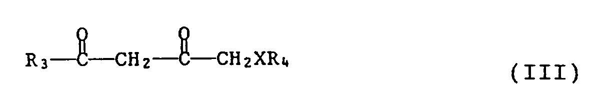 in the presence of an acid in an inert solvent at temperatures of from 20 °C to 140°C and then cyclising at the reflux temperature of the solvent used to give a pyrimidine compound of formula V 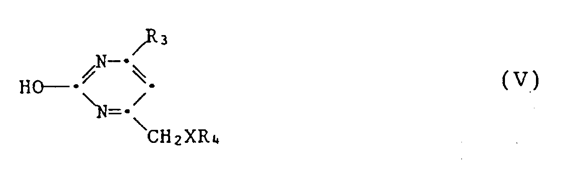 and
2.2 further reacting the resulting compound of formula V with excess POCl₃, without a solvent or in a solvent that is inert towards POCI₃, at temperatures of from 50 °C to 110°C, to give the compound of formula VI 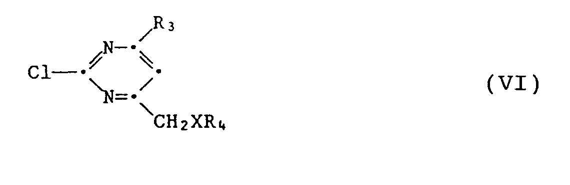 and
2.3 further reacting the resulting compound of formula VI with an aniline compound of formula VII 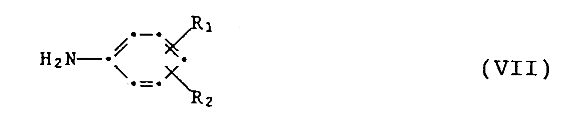 depending on the reaction conditions either
a) in the presence of a proton acceptor, without a solvent or in a protic or aprotic solvent, or
b) in the presence of an acid in an inert solvent, in each case at temperatures of from 60 _{°} C to 120 °C, or
3. in a two-stage process:
3.1 cyclising a guanidine salt of formula VIII 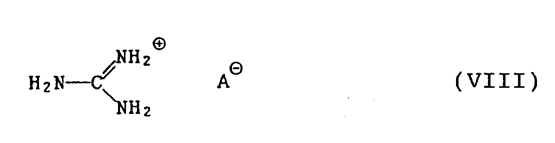 with a diketone of formula III 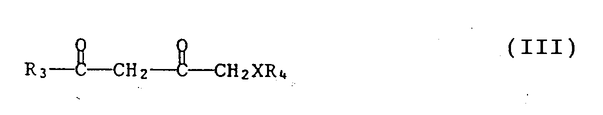
a) without a solvent, at temperatures of from 100 °C to 160 °C, or
b) in a protic or aprotic solvent or a mixture of protic and aprotic solvents, at temperatures of from 30°C to 140°C, to give a pyrimidine compound of formula IX 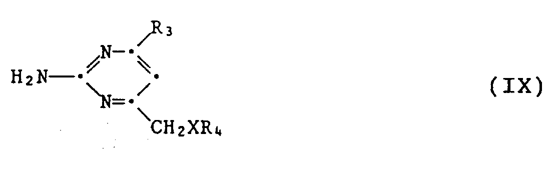 and
3.2 reacting the resulting compound of formula IX with a compound of formula X 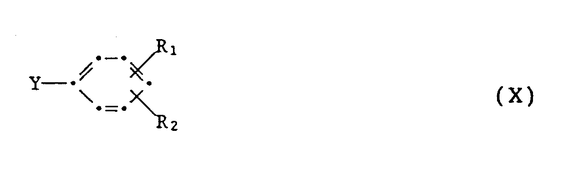 in the presence of a proton acceptor in aprotic solvents at temperatures of from 30°C to 140°C to remove HY, the substituents R₁ to R₄ and X in formulae II to X being as defined for formula I, A⊖ being an acid anion and Y being halogen.

14. A composition for controlling or preventing an attack by insect pests or microorganisms, which comprises as at least one active component a compound of formula I according to claim 1.

15. A composition according to claim 14 which comprises as at least one active component a compound of formula I according to claim 2.

16. A composition according to claim 14 which comprises as at least one active component a compound of formula I according to claim 3.

17. A composition according to claim 14 which comprises as at least one active component a compound of formula I according to any one of claims 4 to 12.

18. A composition according to claim 14 which comprises from 0.1 to 99 % by weight of a compound of formula I, from 99.9 to 1 % by weight of a solid or liquid adjuvant and from 0 to 25 % by weight of a surfactant.

19. A process for the preparation of an agrochemical composition as defined in claim 14, which comprises intimately mixing at least one compound of formula I as defined in claim 1 with suitable solid or liquid adjuvants and surfactants.

20. A method of controlling or preventing an attack on cultivated plants by insect pests or phytopathogenic microorganisms, which comprises applying a compound of formula I as defined in claim 1 to the plant or its locus.

21. The use of a compound of formula I according to claim 1 for controlling and/or preventing an attack by harmful microorganisms.

22. Use according to claim 21, wherein the microorganisms are phytopathogenic fungi.

23. Use according to claim 22 against fungi of the class Fungi imperfecti.

24. Use according to claim 23 against Botrytis attack.

## Claims (Claims for the following Contracting State(s) : ES)

1. A process for the preparation of a compound of formula I 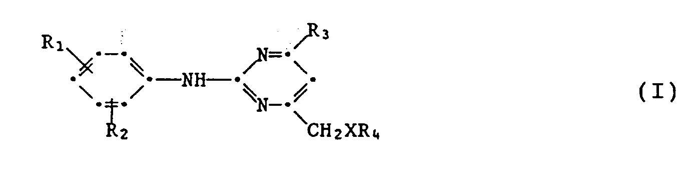 in which:
R₁ and R₂ independently of one another are hydrogen, halogen, C1-C3alkyl, C1-C2haloalkyl, Ci-C₃alkoxy or C₁-C₃haloalkoxy;
R₃ is hydrogen; C₁-C₄alkyl; C₁-C₂alkyl substituted by halogen or by hydroxy; cyclopropyl; or cyclopropyl substituted by halogen and/or by methyl;
R₄ is C₁-C₈alkyl; C₂-C₆alkyl substituted by halogen, hydroxy, cyano, C₁-C₄alkoxy, C3-C4alkoxyalkoxy or by C₁-C₃alkylthio; C₃-C₆alkenyl; C₃-C₆alkynyl; C₃-C₇cycloalkyl; or C₃-C₇cycloalkyl substituted by methyl; and
X is oxygen or sulfur,
which process comprises
1. reacting a phenylguanidine salt of formula Ila 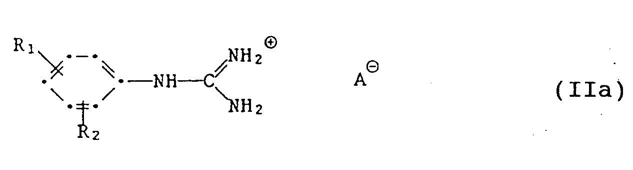 or the free guanidine base of formula IIb 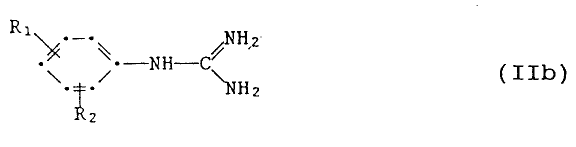 with a diketone of formula III 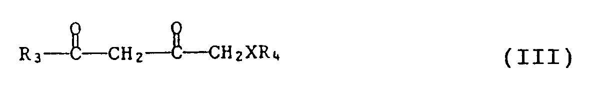 without a solvent or in an aprotic solvent, but preferably in a protic solvent, at temperatures of from 60°C to 160°C, or
2. in a multi-stage process:
2.1 reacting urea with a ketone of formula III 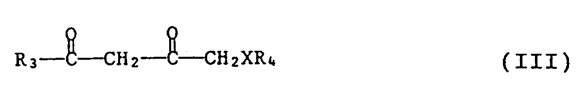 in the presence of an acid in an inert solvent at temperatures of from 20 °C to 140°C and then cyclising at the reflux temperature of the solvent used to give a pyrimidine compound of formula V 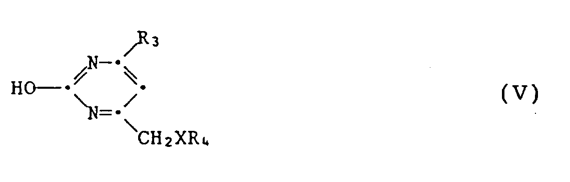 and
2.2 further reacting the resulting compound of formula V with excess POCl₃, without a solvent or in a solvent that is inert towards POCI₃, at temperatures of from 50 °C to 110°C, to give the compound of formula VI 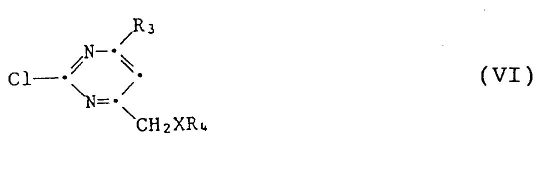 and
2.3 further reacting the resulting compound of formula VI with an aniline compound of formula VII 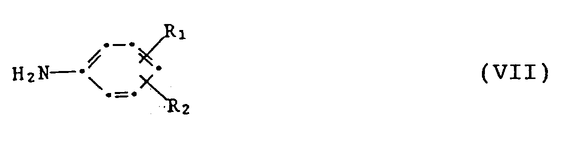 depending on the reaction conditions either
a) in the presence of a proton acceptor, without a solvent or in a protic or aprotic solvent, or
b) in the presence of an acid in an inert solvent, in each case at temperatures of from 60 _{°} C to 120°C, or
3. in a two-stage process:
3.1 cyclising a guanidine salt of formula VIII 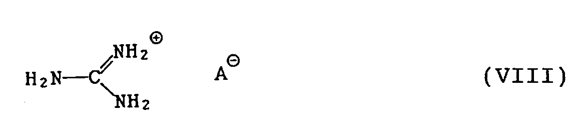 with a diketone of formula III 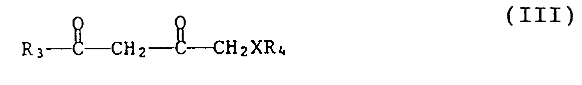
a) without a solvent, at temperatures of from 100 °C to 160 °C, or
b) in a protic or aprotic solvent or a mixture of protic and aprotic solvents, at temperatures of from 30°C to 140°C,
to give a pyrimidine compound of formula IX 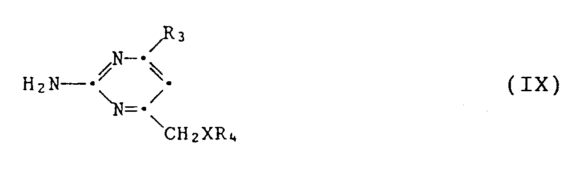 and
3.2 reacting the resulting compound of formula IX with a compound of formula X 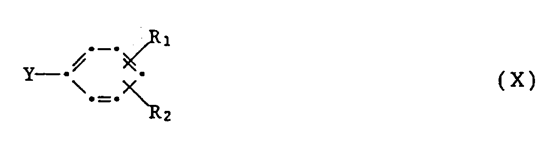 in the presence of a proton acceptor in aprotic solvents at temperatures of from 30°C to 140°C to remove HY, the substituents R₁ to R₄ and X in formulae II to X being as defined for formula I, A⊖ being an acid anion and Y being halogen.

2. A process according to claim 1 for the preparation of a compound of formula I in which R₃, R₄ and X are as defined and R₁ and R₂ are hydrogen.

3. A process according to claim 1 for the preparation of a compound of formula I in which:
R₁ and R₂ independently of one another are hydrogen, halogen, C₁-C₃alkyl, C₁-C₂haloalkyl, Ci-C₃alkoxy or C₁-C₃haloalkoxy;
R₃ is hydrogen, C₁-C₄alkyl or cyclopropyl;
R₄ is C₁-C₈alkyl; C₂-C₆alkyl substituted by halogen, hydroxy, cyano, C₁-C₄alkoxy, C3-C4alkoxyalkoxy or by C₁-C₃alkylthio; C₃-C₆alkenyl; C₃-C₆alkynyl; C₅-C₇cycloalkyl; or Cs-C₇cycloalkyl substituted by methyl; and
X is oxygen or sulfur.

4. A proccss according to claim 1 for the preparation of a compound of formula I in which:
R₁ and R₂ independently of one another are hydrogen, fluorine, chlorine, bromine, C₁-C₃alkyl, Ci-C₂haloalkyl, C₁-C₃alkoxy or C₁-C₂haloalkoxy;
R₃ is hydrogen, C₁-C₃alkyl or cyclopropyl;
R₄ is C₁-C₆alkyl; C₂-C₄alkyl substituted by halogen, hydroxy, cyano, C₁-C₃alkoxy, C3-C4alkoxyalkoxy or by C₁-C₂alkylthio; C₃alkenyl; C₃alkynyl; or C₆-C₇cycloalkyl that is unsubstituted or substituted by methyl; and
X is oxygen or sulfur.

5. A process according to claim 1 for the preparation of a compound of formula I in which:
R₁ and R₂ independently of one another are hydrogen, fluorine, chlorine, bromine, C₁-C₃alkyl, Ci-C₃alkoxy, trifluoromethyl, -OCHF₂, -OCF₂CHF₂, -OCF₂CHCIF, -OCF₂CHCI₂ or -OCF₂CCI₂F;
R₃ is hydrogen, methyl, ethyl or n-propyl;
R₄ is C₁-C₄alkyl; C₂-C₃alkyl substituted by halogen, hydroxy, cyano, C₁-C₂alkoxy or by C₁-C₂alkylthio;
C₃-C₆alkenyl; C₃-C₆alkynyl; or cyclohexyl; and
X is oxygen or sulfur.

6. A process according to claim 1 for the preparation of a compound of formula I in which:
R₁ and R₂ independently of one another are hydrogen, fluorine, chlorine, bromine, C₁-C₂alkyl, Ci-C₂alkoxy, trifluoromethyl, -OCHF₂, -OCF₃, -OCF₂CHF₂ or -OCF₂CHCIF;
R₃ is hydrogen or C₁-C₂ alkyl;
R₄ is C₁-C₃alkyl; C₂alkyl substituted by halogen, hydroxy, cyano, C₁-C₂alkoxy or by C₁-C₂alkylthio; C₃-C₄alkenyl; or C3-C4alkynyl; and
X is oxygen or sulfur.

7. A process according to claim 1 for the preparation of a compound of formula I in which:
R₁ and R₂ independently of one another are hydrogen, fluorine, chlorine, bromine, C₁-C₂alkyl, Ci-C₂alkoxy, trifluoromethyl or -OCHF₂;
R₃ is methyl;
R₄ is C₁-C₂alkyl; C₂alkyl substituted by fluorine, chlorine, bromine, cyano, C₁-C₂alkoxy or by methylthio; allyl; or propargyl; and
X is oxygen or sulfur.

8. A process according to claim 1 for the preparation of a compound of formula I in which:
R₁ and R₂ independently of one another are hydrogen, halogen, C₁-C₂alkyl, halomethyl, C₁-C₂alkoxy or C₁-C₂ haloalkoxy;
R₃ is hydrogen; C₁-C₃alkyl; C₁-C₂alkyl substituted by halogen or by hydroxy; cyclopropyl; or cyclopropyl mono- to tri-substituted by the same or different substituents selected from the group consisting of halogen and methyl;
R₄ is C₁-C₄alkyl; C₂-C₃alkyl substituted by halogen, cyano, C₁-C₂alkoxy or by C₁-C₂alkylthio; C₃-C₆alkenyl; C₃-C₆alkynyl; or C₃-C₇cycloalkyl; and
X is oxygen or sulfur.

9. A process according to claim 1 for the preparation of a compound of formula I in which:
R₁ and R₂ independently of one another are hydrogen, fluorine, chlorine, bromine, methyl, trifluoromethyl, methoxy or difluoromethoxy;
R₃ is hydrogen; C₁-C₃alkyl; C₁-C₂alkyl substituted by halogen or by hydroxy; cyclopropyl; or cyclopropyl mono- to tri-substituted by the same or different substituents selected from the group consisting of halogen and methyl;
R₄ is C₁-C₃alkyl; C₂-C₃alkyl substituted by fluorine, chlorine or by C₁-C₂alkoxy; C₃-C₆alkenyl; C₃-C₆alkynyl; or C₃-C₆cycloalkyl; and
X is oxygen or sulfur.

10. A process according to claim 1 for the preparation of a compound of formula I in which:
R₁ and R₂ independently of one another are hydrogen, fluorine, chlorine, methyl, trifluoromethyl, methoxy or difluoromethoxy;
R₃ is C₁-C₃alkyl; methyl substituted by halogen or by hydroxy; cyclopropyl; or cyclopropyl mono- to tri-substituted by halogen or by methyl;
R₄ is C₁-C₃alkyl; C₂-C₃alkyl substituted by fluorine, chlorine or by methoxy; C3-C4alkenyl; C₃-C₄alkynyl; cyclopropyl; or cyclohexyl; and
X is oxygen.

11. A process according to claim 1 for the preparation of a compound of formula I in which:
R₁ and R₂ are hydrogen;
R₃ is C₁-C₃alkyl; methyl substituted by fluorine, chlorine or by bromine; cyclopropyl; or cyclopropyl substituted by chlorine or by methyl;
R₄ is C₁-C₃alkyl, 2-chloroethyl, 2,2,2-trifluoroethyl, allyl or propargyl; and
X is oxygen.

12. A process according to claim 1 for the preparation of a compound of formula I selected from
2-phenylamino-4-methyl-6-methoxymethylpyrimidine;
2-phenylamino-4-cyclopropyl-6-methoxymethylpyrimidine.

## Claims (Claims for the following Contracting State(s) : CH, DE, FR, GB, IT, LI, NL)

1. A compound of formula I 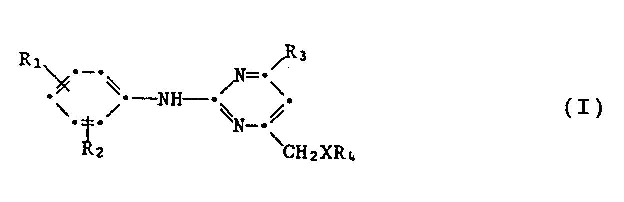 in which:
R₁ and R₂ independently of one another are hydrogen, halogen, C₁-C₃alkyl, C₁-C₂haloalkyl, C₁-C₃alkoxy or C₁-C₃haloalkoxy;
R₃ is hydrogen; C₁-C₄alkyl; C₁-C₂alkyl substituted by halogen or by hydroxy; cyclopropyl; or cyclopropyl substituted by halogen and/or by methyl;
R₄ is C₁-C₈alkyl; C₂-C₆alkyl substituted by halogen, hydroxy, cyano, C₁-C₄alkoxy, C3-C4alkoxyalkoxy or by C₁-C₃alkylthio; C₃-C₆alkenyl; C₃-C₆alkynyl; C₃-C₇cycloalkyl; or C₃-C₇cycloalkyl substituted by methyl; and
X is oxygen or sulfur;
with the proviso that, when R₁ is hydrogen, R₂ is hydrogen, halogen, C₁-C₃alkyl, C₁-C₃alkoxy or Ci-C₂haloalkyl and R₃ is halogen, C₁-C₄alkyl or C₁-C₂haloalkyl, R₄ is other than alkyl when X = sulfur and R₄ is other than alkyl, alkenyl and alkynyl when X = oxygen.

2. A compound of formula I according to claim 1 in which R₃, R₄ and X are as defined and R₁ and R₂ are hydrogen.

3. A compound of formula I according to claim 1 in which:
R₁ and R₂ independently of one another are hydrogen, halogen, C₁-C₃alkyl, C₁-C₂haloalkyl, Ci-C₃alkoxy or C₁-C₃haloalkoxy;
R₃ is hydrogen, C₁-C₄alkyl or cyclopropyl;
R₄ is C₁-C₈alkyl; C₂-C₆alkyl substituted by halogen, hydroxy, cyano, C₁-C₄alkoxy, C3-C4alkoxyalkoxy or by C₁-C₃alkylthio; C₃-C₆alkenyl; C₃-C₆alkynyl; Cs-C₇cycloalkyl; or Cs-C₇cycloalkyl substituted by methyl; and
X is oxygen or sulfur.

4. A compound of formula I according to claim 3 in which:
R₁ and R₂ independently of one another are hydrogen, fluorine, chlorine, bromine, C₁-C₃alkyl, Ci-C₂haloalkyl, C₁-C₃alkoxy or C₁-C₂haloalkoxy;
R₃ is hydrogen, C₁-C₃alkyl or cyclopropyl;
R₄ is C₁-C₆alkyl; C₂-C₄alkyl substituted by halogen, hydroxy, cyano, C₁-C₃alkoxy, C3-C4alkoxyalkoxy or by C₁-C₂alkylthio; C₃alkenyl; C₃alkynyl; or C₆-C₇cycloalkyl that is unsubstituted or substituted by methyl; and
X is oxygen or sulfur.

5. A compound of formula I according to claim 3 in which:
R₁ and R₂ independently of one another are hydrogen, fluorine, chlorine, bromine, C₁-C₃alkyl, Ci-C₃alkoxy, trifluoromethyl, -OCHF₂, -OCF₂CHF₂, -OCF₂CHCIF, -OCF₂CHCI₂ or -OCF₂CCI₂F;
R₃ is hydrogen, methyl, ethyl or n-propyl;
R₄ is C₁-C₄alkyl; C₂-C₃alkyl substituted by halogen, hydroxy, cyano, C₁-C₂alkoxy or by C₁-C₂alkylthio;
C₃-C₆alkenyl; C₃-C₆alkynyl; or cyclohexyl; and
X is oxygen or sulfur.

6. A compound of formula I according to claim 4 in which:
R₁ and R₂ independently of one another are hydrogen, fluorine, chlorine, bromine, C₁-C₂alkyl, Ci-C₂alkoxy, trifluoromethyl, -OCHF₂, -OCF₃, -OCF₂CHF₂ or -OCF₂CHCIF;
R₃ is hydrogen or C₁-C₂alkyl;
R₄ is C₁-C₃alkyl; C₂alkyl substituted by halogen, hydroxy, cyano, C₁-C₂alkoxy or by C₁-C₂alkylthio; C₃-C₄alkenyl; or C3-C4alkynyl; and
X is oxygen or sulfur.

7. A compound of formula I according to claim 6 in which:
R₁ and R₂ independently of one another are hydrogen, fluorine, chlorine, bromine, C₁-C₂alkyl, Ci-C₂alkoxy, trifluoromethyl or -OCHF₂;
R₃ is methyl;
R₄ is C₁-C₂alkyl; C₂alkyl substituted by fluorine, chlorine, bromine, cyano, C₁-C₂alkoxy or by methylthio; allyl; or propargyl; and
X is oxygen or sulfur.

8. A compound of formula I according to claim 1 in which:
R₁ and R₂ independently of one another are hydrogen, halogen, C₁-C₂alkyl, halomethyl, C₁-C₂alkoxy or C₁-C₂haloalkoxy;
R₃ is hydrogen; C₁-C₃alkyl; C₁-C₂alkyl substituted by halogen or by hydroxy; cyclopropyl; or cyclopropyl mono- to tri-substituted by the same or different substituents selected from the group consisting of halogen and methyl;
R₄ is C₁-C₄alkyl; C₂-C₃alkyl substituted by halogen, cyano, C₁-C₂alkoxy or by C₁-C₂alkylthio; C₃-C₆alkenyl; C₃-C₆alkynyl; or C₃-C₇cycloalkyl; and
X is oxygen or sulfur.

9. A compound of formula I according to claim 8 in which:
R₁ and R₂ independently of one another are hydrogen, fluorine, chlorine, bromine, methyl, trifluoromethyl, methoxy or difluoromethoxy;
R₃ is hydrogen; C₁-C₃alkyl; C₁-C₂alkyl substituted by halogen or by hydroxy; cyclopropyl; or cyclopropyl mono- to tri-substituted by the same or different substituents selected from the group consisting of halogen and methyl;
R₄ is C₁-C₃alkyl; C₂-C₃alkyl substituted by fluorine, chlorine or by C₁-C₂alkoxy; C₃-C₆alkenyl; C₃-C₆alkynyl; or C₃-C₆cycloalkyl; and
X is oxygen or sulfur.

10. A compound of formula I according to claim 9 in which:
R₁ and R₂ independently of one another are hydrogen, fluorine, chlorine₁ methyl, trifluoromethyl, methoxy or difluoromethoxy;
R₃ is C₁-C₃alkyl; methyl substituted by halogen or by hydroxy; cyclopropyl; or cyclopropyl mono- to tri-substituted by halogen or by methyl;
R₄ is C₁-C₃alkyl; C₂-C₃alkyl substituted by fluorine, chlorine or by methoxy; C3-C4alkenyl; C₃-C₄alkynyl; cyclopropyl; or cyclohexyl; and
X is oxygen.

11. A compound of formula I according to claim 10 in which:
R₁ and R₂ are hydrogen;
R₃ is C₁-C₃alkyl; methyl substituted by fluorine, chlorine or by bromine; cyclopropyl; or cyclopropyl substituted by chlorine or by methyl;
R₄ is C1-C3alkyl, 2-chloroethyl, 2,2,2-trifluoroethyl, allyl or propargyl; and
X is oxygen.

12. A compound of formula I according to claim 3 2-phenylamino-4-cyclopropyl-6-methoxymethyl- pyrimidine.

13. A process for the preparation of a compound of formula I according to claim 1, which comprises
1. reacting a phenylguanidine salt of formula Ila 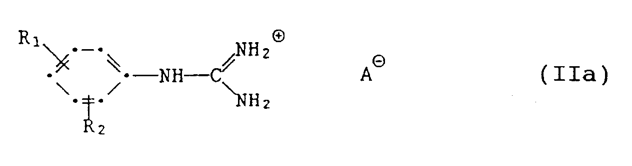 or the free guanidine base of formula IIb 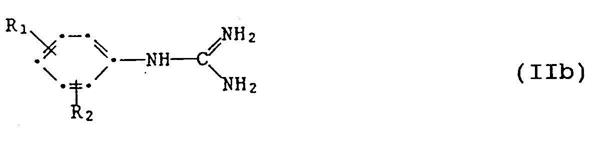 with a diketone of formula III 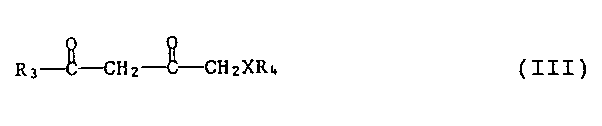 without a solvent or in an aprotic solvent, but preferably in a protic solvent, at temperatures of from 60 _{°} C to 160°C, or
2. in a multi-stage process:
2.1 reacting urea with a ketone of formula III 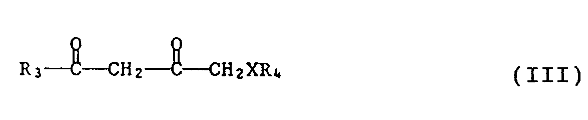 in the presence of an acid in an inert solvent at temperatures of from 20 °C to 140°C and then cyclising at the reflux temperature of the solvent used to give a pyrimidine compound of formula V 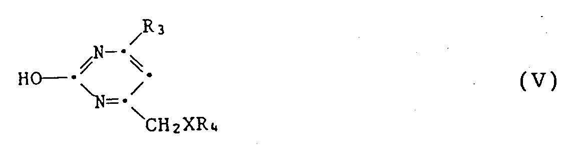 and
2.2 further reacting the resulting compound of formula V with excess POCl₃, without a solvent or in a solvent that is inert towards POCI₃, at temperatures of from 50 °C to 110°C, to give the compound of formula VI 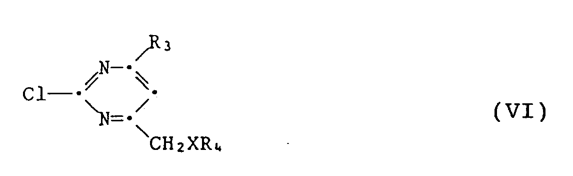 and
2.3 further reacting the resulting compound of formula VI with an aniline compound of formula VII 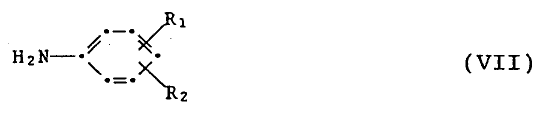 depending on the reaction conditions either
a) in the presence of a proton acceptor, without a solvent or in a protic or aprotic solvent, or
b) in the presence of an acid in an inert solvent, in each case at temperatures of from 60 _{°} C to 120°C, or
3. in a two-stage process:
3.1 cyclising a guanidine salt of formula VIII 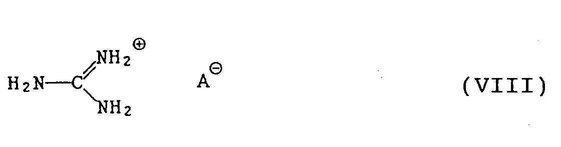 with a diketone of formula III 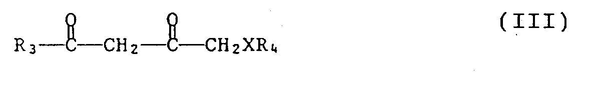
a) without a solvent, at temperatures of from 100 °C to 160 °C, or
b) in a protic or aprotic solvent or a mixture of protic and aprotic solvents, at temperatures of from 30°C to 140°C,
to give a pyrimidine compound of formula IX 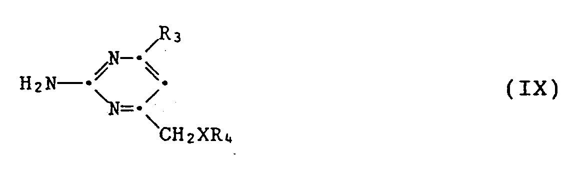 and
3.2 reacting the resulting compound of formula IX with a compound of formula X 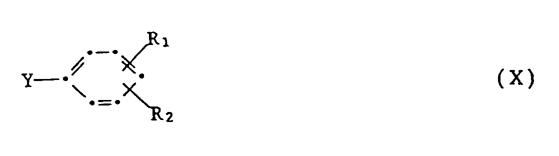 in the presence of a proton acceptor in aprotic solvents at temperatures of from 30°C to 140°C to remove HY, the substituents R₁ to R₄ and X in formulae II to X being as defined for formula I, AS being an acid anion and Y being halogen.

14. A composition for controlling or preventing an attack by insect pests or microorganisms, which comprises as at least one active component a compound of formula I according to claim 1.

15. A composition according to claim 14 which comprises as at least one active component a compound of formula I according to claim 2.

16. A composition according to claim 14 which comprises as at least one active component a compound of formula I according to claim 3.

17. A composition according to claim 14 which comprises as at least one active component a compound of formula I according to any one of claims 4 to 12.

18. A composition according to claim 14 which comprises from 0.1 to 99 % by weight of a compound of formula I, from 99.9 to 1 % by weight of a solid or liquid adjuvant and from 0 to 25 % by weight of a surfactant.

19. A process for the preparation of an agrochemical composition as defined in claim 14, which comprises intimately mixing at least one compound of formula I as defined in claim 1 with suitable solid or liquid adjuvants and surfactants.

20. A method of controlling or preventing an attack on cultivated plants by insect pests or phytopathogenic microorganisms, which comprises applying a compound of formula I as defined in claim 1 to the plant or its locus.

21. The use of a compound of formula I according to claim 1 for controlling and/or preventing an attack by harmful microorganisms.

22. Use according to claim 21, wherein the microorganisms are phytopathogenic fungi.

23. Use according to claim 22 against fungi of the class Fungi imperfecti.

24. Use according to claim 23 against Botrytis attack.

## Revendications (Revendications pour l'(les) Etat(s) contractant(s) suivant(s) : suivants : AT, BE, GR, LU, SE)

1. Composés de formule 1 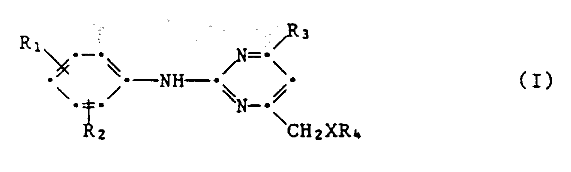 dans laquelle
R₁ et R₂ représentent chacun, indépendamment l'un de l'autre, l'hydrogène, un halogène, un groupe alkyle en C 1-C 3, halogénoalkyle en C 1-C 2, alcoxy en C 1-C 3 ou halogénoalcoxy en C 1-C 3;
R₃ représente l'hydrogène, un groupe alkyle en C 1-C 4, un groupe alkyle en C 1-C 2 à substituants halogéno ou hydroxy, un groupe cyclopropyle ou un groupe cyclopropyle à substituants halogéno et/ou méthyle;
R₄ représente un groupe alkyle en C 1-C 8; un groupe alkyle en C 2-C 6 substitué par des halogènes, des groupes hydroxy, cyano, alcoxy en C 1-C 4, alcoxyalcoxy en C 3-C 4 ou alkylthio en C 1-C 3; un groupe alcényle en C 3-C 6; un groupe alcynyle en C 3-C 6; un groupe cycloalkyle en C 3-C 7 ou un groupe cycloalkyle en C 3-C 7 substitué par des groupes méthyle;
X représente l'oxygène ou le soufre.

2. Composés de formule 1 de la revendication 1, dans laquelle R₃, R₄ et X ont les significations indiquées et R₁ et R₂ représentent l'hydrogène.

3. Composés de formule 1 de la revendication 1, dans laquelle :
R₁ et R₂ représentent chacun, indépendamment l'un de l'autre, l'hydrogène, un halogène, un groupe alkyle en C 1-C 3, halogénoalkyle en C 1-C 2, alcoxy en C 1-C 3 ou halogénoalcoxy en C 1-C 3;
R₃ représente l'hydrogène, un groupe alkyle en C 1-C 4 ou cyclopropyle;
R₄ représente un groupe alkyle en C 1-C 8; un groupe alkyle en C 2-C 6 substitué par des halogènes, des groupes hydroxy, cyano, alcoxy en C 1-C 4, alcoxyalcoxy en C 3-C 4 ou alkylthio en C 1-C 3; un groupe alcényle en C 3-C 6; un groupe alcynyle en C 3-C 6; un groupe cycloalkyle en C 5-C 7 ou un groupe cycloalkyle en C 5-C 7 substitué par des groupes méthyle;
X représente l'oxygène ou le soufre.

4. Composés de formule 1 de la revendication 3, dans laquelle
R₁ et R₂ représentent chacun, indépendamment l'un de l'autre, l'hydrogène, le fluor, le chlore, le brome, un groupe alkyle en C 1-C 3, halogénoalkyle en C 1-C 2, alcoxy en C 1-C 3 ou halogénoalcoxy en C 1-C 2;
R₃ représente l'hydrogène, un groupe alkyle en C 1-C 3 ou cyclopropyle;
R₄ représente un groupe alkyle en C 1-C 6; un groupe alkyle en C 2-C 4 substitué par des halogènes, des groupes hydroxy, cyano, alcoxy en C 1-C 3, alcoxyalcoxy en C 3-C 4 ou alkylthio en C 1-C 2; un groupe alcényle en C 3; un groupe alcynyle en C 3; ou un groupe cycloalkyle en C 6-C 7 non substitué ou substitué par des groupes méthyle;
X représente l'oxygène ou le soufre.

5. Composés de formule 1 de la revendication 3, dans laquelle
R₁ et R₂ représentent chacun, indépendamment l'un de l'autre, l'hydrogène, le fluor, le chlore, le brome, un groupe alkyle en C 1-C 3, alcoxy en C 1-C 3, trifluorométhyle, -OCHF₂, -OCF₂CHF₂, -OCF₂CHCIF, -OCF₂CHCI₂ ou -OCF₂CCI₂F;
R₃ représente l'hydrogène, un groupe méthyle, éthyle ou n-propyle;
R₄ représente un groupe alkyle en C 1-C 4; un groupe alkyle en C 2-C 3 substitué par des halogènes, des groupes hydroxy, cyano, alcoxy en C 1-C 2 ou alkylthio en C 1-C 2; un groupe alcényle en C 3-C 6; un groupe alcynyle en C 3-C 6 ou cyclohexyle;
X représente l'oxygène ou le soufre.

6. Composés de formule 1 de la revendication 4, dans laquelle
R₁ et R₂ représentent chacun, indépendamment l'un de l'autre, l'hydrogène, le fluor, le chlore, le brome, un groupe alkyle en C 1-C 2, alcoxy en C 1-C 2, trifluorométhyle, -OCHF₂, -OCF₃, -OCF₂CHF₂ ou -OCF₂CHCIF;
R₃ représente l'hydrogène ou un groupe alkyle en C 1-C 2;
R₄ représente un groupe alkyle en C 1-C 3; un groupe alkyle en C 2 substitué par des halogènes, des groupes hydroxy, cyano, alcoxy en C 1-C 2 ou alkylthio en C 1-C 2; un groupe alcényle en C 3-C 4; un groupe alcynyle en C 3-C 4;
X représente l'oxygène ou le soufre.

7. Composés de formule 1 de la revendication 6, dans laquelle
R₁ et R₂ représentent chacun, indépendamment l'un de l'autre, l'hydrogène, le fluor, le chlore, le brome, un groupe alkyle en C 1-C 2, alcoxy en C 1-C 2, trifluorométhyle ou -OCHF₂;
R₃ représente un groupe méthyle;
R₄ représente un groupe alkyle en C 1-C 2; un groupe atkyle en C 2 substitué par le fluor, le chlore, le brome, des groupes cyano, alcoxy en C 1-C 2 ou méthylthio; un groupe allyle ou propargyle;
X représente l'oxygène ou le soufre.

8. Composés de formule 1 de la revendication 1, dans laquelle :
R₁ et R₂ représentent chacun, indépendamment l'un de l'autre, l'hydrogene, un halogène, un groupe alkyle en C 1-C 2, halogénométhyle, alcoxy en C 1-C 2 ou halogénoalcoxy en C 1-C 2;
R₃ représente l'hydrogène, un groupe alkyle en C 1-C 3; un groupe alkyle en C 1-C 2 substitué par un halogène ou un groupe hydroxy; un groupe cyclopropyle ou un groupe cyclopropyle portant un à trois substituants identiques ou différents choisis parmi les halogènes et les groupes méthyle;
R₄ représente un groupe alkyle en C 1-C 4, un groupe alkyle en C 2-C 3 substitué par des halogènes, des groupes cyano, alcoxy en C 1-C 2 ou alkylthio en C 1-C 2; un groupe alcényle en C 3-C 6, alcynyle en C 3-C 6 ou cycloalkyle en C 3-C 7;
X représente l'oxygène ou le soufre.

9. Composés de formule 1 de la revendication 8, dans laquelle :
R₁ et R₂ représentent chacun, indépendamment l'un de l'autre, l'hydrogène, le fluor, le chlore, le brome, un groupe méthyle, trifluorométhyle, méthoxy ou difluorométhoxy;
R₃ représente l'hydrogène; un groupe alkyle en C 1-C 3; un groupe alkyle en C 1-C 2 substitué par des halogènes ou des groupes hydroxy; un groupe cyclopropyle ou un groupe cyclopropyle portant un à trois substituants identiques ou différents choisis parmi les halogènes et les groupes méthyle;
R₄ représente un groupe alkyle en C 1-C 3; un groupe alkyle en C 2-C 3 substitué par le fluor, le chlore ou des groupes alcoxy en C 1-C 2; un groupe alcényle en C 3-C 6; un groupe alcynyle en C 3-C 6 ou cycloalkyle en C 3-C 6;
X représente l'oxygène ou le soufre.

10. Composés de formule 1 de la revendication 9, dans laquelle
R₁ et R₂ représentent chacun, indépendamment l'un de l'autre, l'hydrogène, le fluor, le chlore, un groupe méthyle, trifluorométhyle, méthoxy ou difluorométhoxy;
R₃ représente un groupe alkyle en C 1-C 3; un groupe méthyle substitué par un halogène ou un groupe hydroxy; un groupe cyclopropyle ou un groupe cyclopropyle portant un à trois substituants halogéno ou méthyle;
R₄ représente un groupe alkyle en C 1-C 3; un groupe alkyle en C 2-C 3 substitué par le fluor, le chlore ou un groupe méthoxy; un groupe alcényle en C 3-C 4, alcynyle en C 3-C 4; un groupe cyclopropyle; un groupe cyclohexyle;
X représente l'oxygène.

11. Composés de formule 1 de la revendication 10, dans laquelle :
R₁ et R₂ représentent l'hydrogène;
R₃ représente un groupe alkyle en C 1-C 3; un groupe méthyle substitué par le fluor, le chlore ou le brome; un groupe cyclopropyle ou un groupe cyclopropyle substitué par le chlore ou des groupes méthyle;
R₄ représente un groupe alkyle en C 1-C 3; un groupe 2-chloréthyle, 2,2,2-trifluoréthyle; un groupe allyle ou propargyle;
X représente l'oxygène.

12. Un composé de formule 1 de la revendication 3, choisi parmi les suivants :
2-phénylamino-4-méthyl-6-méthoxyméthyl-pyrimidine;
2-phénylamino-4-cyclopropyl-6-méthoxyméthyl-pyrimidine.

13. Procédé de préparation des composés de formule 1 de la revendication 1, caractérisé en ce que :
1. on fait réagir à des températures de 60 à 160 °C un sel de phénylguanidine de formule Ila 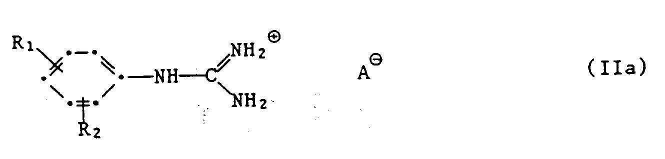 ou la guanidine dont il dérive, de formule Ilb 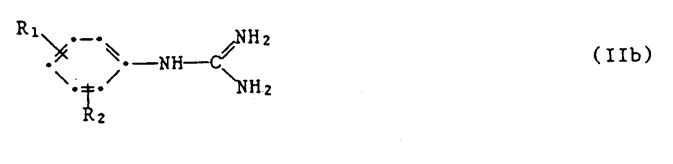 avec une dicétone de formule III 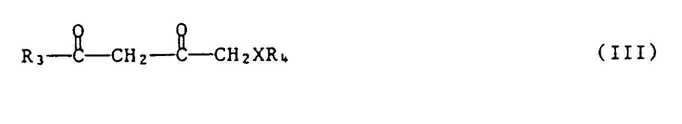 sans solvant ou dans un solvant aprotonique, mais de préférence dans un solvant protonique, ou bien
2. Dans un procédé à plusieurs stades opératoires :
2.1 on fait réagir l'urée avec une cétone de formule III 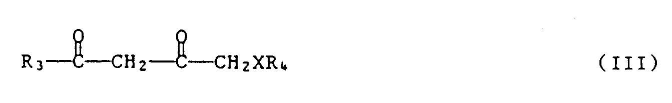 en présence d'un acide, dans un solvant inerte, à des températures de 20 à 140°C puis on cyclise à la température de reflux du solvant utilisé, en un dérivé de pyrimidine de formule V 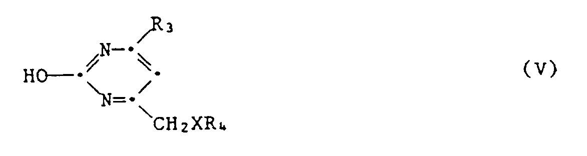 et
2.2 on convertit le composé obtenu, répondant à la formule V, à l'aide d'un excès de POCI₃, sans solvant ou dans un solvant inerte à l'égard de POCl₃, à des températures de 50 à 110 °C, en le composé de formule VI 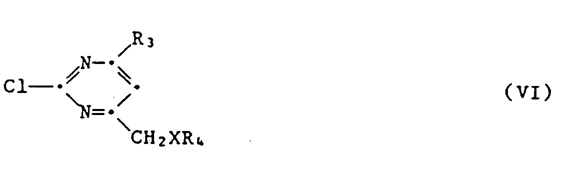 et
2.3 on fait réagir Il composé obtenu, répondant à la formule VI, avec un dérivé de l'aniline de formule VII 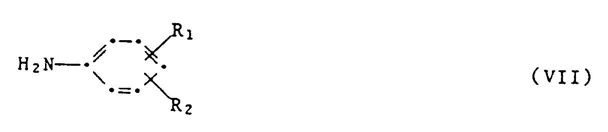 selon les conditions opératoires,
a) soit en présence d'un accepteur de protons sans solvant ou dans un solvant protonique ou aprotonique,
b) soit en présence d'un acide dans un solvantinerte,
dans les deux cas à des températures de 60 à 120°C, ou bien
3. Dans un procédé à deux stades opératoires :
3.1 on cyclise un sel de guanidine de formule VIII 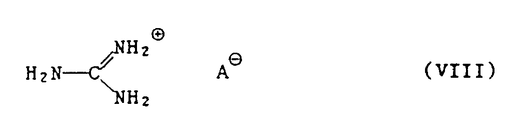 à l'aide d'une dicétone de formule III 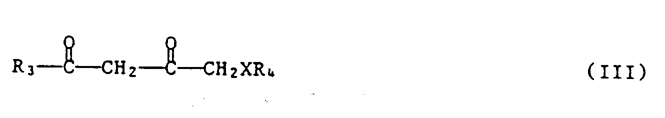
a) sans solvant à des températures de 100 à 160 °C, ou bien
b) dans un solvant protonique ou aprotonique ou dans un mélange de solyants protoniques et aprotoniques, à des températures de 30 à 140°C,
en un dérivé de pyrimidine de formule IX 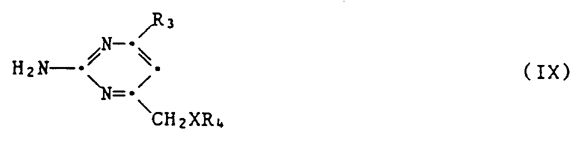 et
3.2 on fait réagir le composé obtenu, répondant à la formule X, 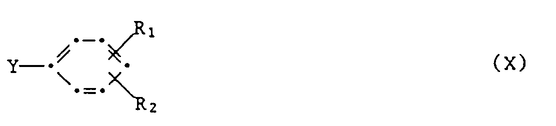 avec scission de HY, en présence d'un accepteur de protons, dans des solvants aprotoniques à des températures de 30 à 140°C,
les symboles R₁ à R₄ et X ayant dans les formules II à X les significations indiquées en référence à la formule I, A^{S} représentant un anion d'acide et Y un halogène.

14. Produit pour combattre ou prévenir une attaque par des insectes ou microorganismes nuisibles, caractérisé en ce qu'il contient au moins un composant actif consistant en un composé de formule 1 de la revendication 1.

15. Produit selon revendication 14, caractérisé en ce qu'il contient au moins un composant actif consistant en un composé de formule 1 de la revendication 2.

16. Produit selon revendication 14, caractérisé en ce qu'il contient au moins un composant actif consistant en un composé de formule 1 de la revendication 3.

17. Produit selon revendication 14, caractérisé en ce qu'il contient au moins un composant actif consistant en un composé de formule I selon l'une des revendications 4 à 12.

18. Produit selon revendication 14, caractérisé en ce qu'il contient de 0,1 à 99 % en poids d'une substance active de formule I, de 99,9 à 1 % en poids d'un additif solide ou liquide et de 0 à 25 % en poids d'un agent tensioactif .

19. Procédé de préparation d'un produit agrochimique tel que défini dans la revendication 14, caractérisé en ce que l'on mélange intimement au moins un composé de formule I tel que défini dans la revendication 1 avec des additifs solides ou liquides et des agents tensioactifs appropriés.

20. Procédé pour combattre ou prévenir une attaque de végétaux cultivés par des insectes nuisibles ou des microorganismes phytopathogènes, caractérisé en ce que l'on applique sur la plante ou son habitat un composé de formule I tel que défini dans la revendication 1.

21. Utilisation de composés de formule 1 de la revendication 1 pour combattre et/ou prévenir une attaque par des microorganismes nuisibles.

22. Utilisation selon revendication 21, caractérisé en ce que les microorganismes sont des mycètes phytopathogènes.

23. Utilisation selon revendication 22, contre des mycètes de la classe des Fungi imperfecti.

24. Utilisation selon revendication 23, contre une attaque par Botrytis.

## Revendications (Revendications pour l'(les) Etat(s) contractant(s) suivant(s) : suivant : ES)

1. Procédé de préparation des composés de formule 1 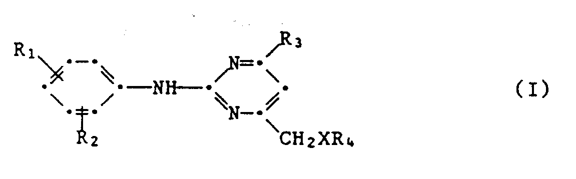 dans laquelle
R₁ et R₂ représentent chacun, indépendamment l'un de l'autre, l'hydrogène, un halogène, un groupe alkyle en C 1-C 3, halogénoalkyle en C 1-C 2, alcoxy en C 1-C 3 ou halogénoalcoxy en C 1-C 3;
R₃ représente l'hydrogène, un groupe alkyJeen C 1-C 4, un groupe alkyle en C 1-C 2 à substituants halogéno ou hydroxy, un groupe cyclopropyle ou un groupe cyclopropyle à substituants halogéno et/ou méthyle;
R₄ représente un groupe alkyle en C 1-C 8; un groupe alkyle en C 2-C 6 substitué par des halogènes, des groupes hydroxy, cyano, alcoxy en C 1-C 4, alcoxyalcoxy en C 3-C 4 ou alkylthio en C 1-C 3; un groupe alcényle en C 3-C 6; un groupe alcynyle en C 3-C 6; un groupe cycloalkyle en C 3-C 7 ou un groupe cycloalkyle en C 3-C 7 substitué par des groupes méthyle;
X représente l'oxygène ou le soufre,
caractérisé en ce que
1. on fait réagir à des températures de 60 à 160 °C un sel de phénylguanidine de formule Ila 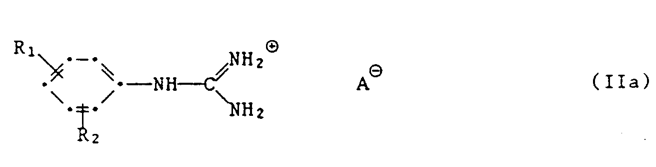 ou la guanidine dont il dérive, de formule Ilb 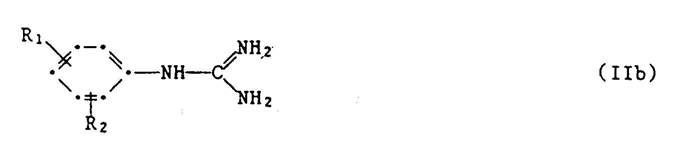 avec une dicétone de formule III 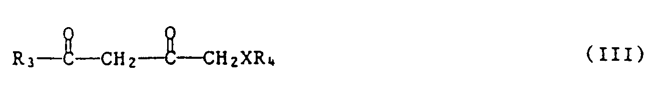 sans solvant ou dans un solvant aprotonique, mais de préférence dans un solvant protonique, ou bien
2. dans un procédé à plusieurs stades opératoires :
2.1 on fait réagir l'urée avec une cétone de formule III 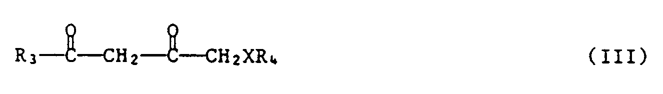 en présence d'un acide, dans un solvant inerte, à des températures de 20 à 140°C puis on cyclise à la température de reflux du solvant utilisé, en un dérivé de pyrimidine de formule V 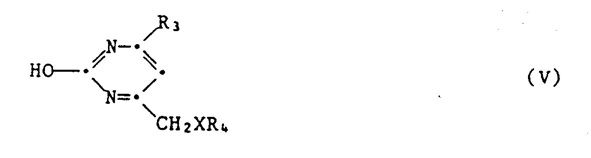 et
2.2 on convertit le composée obtenu, répondant à la formule V, à l'aide d'un excès de POCI₃, sans solvant ou dans un solvant inerte à l'égard de POCI₃, à des températures de 50 à 110° C, en le composé de formule VI 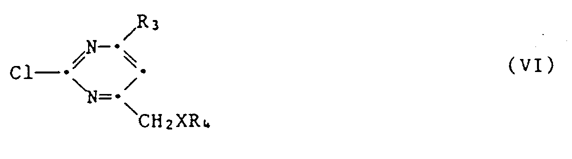 et
2.3 on fait réagir le composé obtenu, répondant à la formule VI, avec un dérivé de l'aniline de formule VII 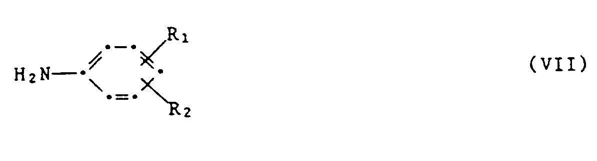 selon les conditions opératoires,
a) soit en présence d'un accepteur de protons sans solvant ou dans un solvant protonique ou aprotonique,
b) soit en présence d'un acide dans un solvant inerte,
dans les deux cas à des températures de 60 à 120°C, ou bien
3. Dans un procédé à deux stades opératoires :
3.1 on cyclise un sel de guanidine de formule VIII 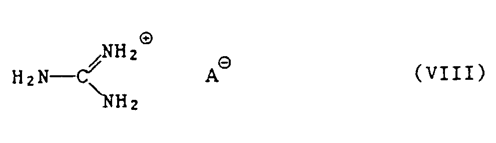 à l'aide d'une dicétone de formule III 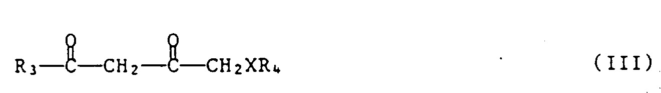
a) sans solvant à des températures de 100 à 160 °C, ou bien
b) dans un solvant protonique ou aprotonique ou dans un mélange de solvants protoniques et aprotoniques, à des températures de 30 à 140°C,
en un dérivé de pyrimidine de formule IX 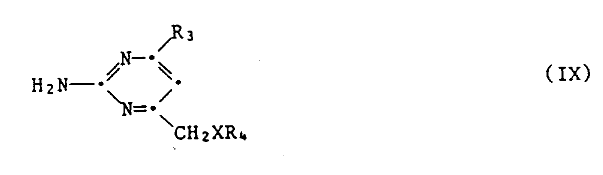 et
3.2 on fait réagir le composé obtenu, répondant à la formule X, 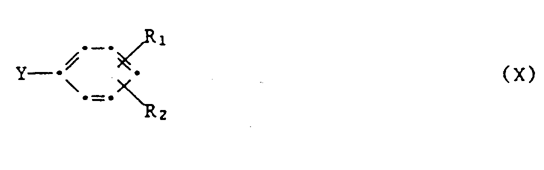 avec scission de HY, en présence d'un accepteur de protons, dans des solvants aprotoniques à des températures de 30 à 140°C,
les symboles R₁ à R₄ et X ayant dans les formules II à X les significations indiquées en référence à la formule I, A^{S} représentant un anion d'acide et Y un halogène.

2. Procédé selon la revendication 1, pour la préparation des composés de formule I dans laquelle R₃, R₄ et X ont les significations indiquées et R₁ et R₂ représentent l'hydrogène.

3. Procédé selon la revendication 1, pour la préparation des composés de formule I dans laquelle :
R₁ et R₂ représentent chacun, indépendamment l'un de l'autre, l'hydrogène, un halogène, un groupe alkyle en C 1-C 3, halogénoalkyle en C 1-C 2, alcoxy en C 1-C 3 ou halogénoalcoxy en C 1-C 3;
R₃ représente l'hydrogène, un groupe alkyle en C 1-C 4 ou cyclopropyle;
R₄ représente un groupe alkyle en C 1-C 8; un groupe alkyle en C 2-C 6 substitué par des halogènes, des groupes hydroxy, cyano, alcoxy en C 1-C 4, alcoxyalcoxy en C 3-C 4 ou alkylthio en C 1-C 3; un groupe alcényle en C 3-C 6; un groupe alcynyle en C 3-C 6; un groupe cycloalkyle en C 5-C 7 ou un groupe cycloalkyle en C 5-C 7 substitué par des groupes méthyle;
X représente l'oxygène ou le soufre;

4. Procédé selon laXrevendication 1, pour la préparation des composés de formule I dans laquelle :
R₁ et R₂ représentent chacun, indépendamment l'un de l'autre, l'hydrogène, le fluor, le chlore, le brome, un groupe alkyle en C 1-C 3, halogénoalkyle en C 1-C 2, alcoxy en C 1-C 3 ou halogénoalcoxy en C 1-C 2;
R₃ représente l'hydrogène, un groupe alkyle en C 1-C 3 ou cyclopropyle;
R₄ représente un groupe alkyle en C 1-C 6; un groupe alkyle en C 2-C 4 substitué par des halogènes, des groupes hydroxy, cyano, alcoxy en C 1-C 3, alcoxyalcoxy en C 3-C 4 ou alkylthio en C 1-C 2; un groupe alcényle en C 3; un groupe alcynyle en C 3; ou un groupe cycloalkyle en C 6-C 7 non substitué ou substitué par des groupes méthyle;
X représente l'oxygène ou le soufre.

5. Procédé de préparation selon la revendication 1, pour la préparation des composés de formule I dans laquelle :
R₁ et R₂ représentent chacun, indépendamment l'un de l'autre, l'hydrogène, le fluor, le chlore, le brome, un groupe alkyle en C 1-C 3, alcoxy en C 1-C 3, trifluorométhyle, -OCHF₂, -OCF₂CHF₂, -OCF₂CHCIF, -CCF₂CHCI₂ ou -OCF₂CCI₂F;
R₃ représente l'hydrogène, un groupe méthyle, éthyle ou n-propyle;
R₄ représente un groupe alkyle en C 1-C 4; un groupe alkyle en C 2-C 3 substitué par des halogènes, des groupes hydroxy, cyano, alcoxy en C 1-C 2 ou alkylthio en C 1-C 2; un groupe alcényle en C 3-C 6; un groupe alcynyle en C 3-C 6 ou cyclohexyle;
X représente l'oxygène ou le soufre.

6. Procédé selon revendication 1, pour la préparation des composés de formule dans laquelle
R₁ et R₂ représentent chacun, indépendamment l'un de l'autre, l'hydrogene, le fluor, le chlore, le brome, un groupe alkyle en C 1-C 2, alcoxy en C 1-C 2, trifluorométhyle, -OCHF₂, -OCF₃, -OCF₂CHF₂ ou -OCF₂CHCIF;
R₃ représente l'hydrogène ou un groupe alkyle en C 1-C 2;
R₄ représente un groupe alkyle en C 1-C 3; un groupe alkyle en C 2 substitué par des halogènes, des groupes hydroxy, cyano, alcoxy en C 1-C 2 ou alkylthio en C 1-C 2; un groupe alcényle en C 3-C 4; un groupe alcynyle en C 3-C 4;
X représente l'oxygène ou le soufre.

7. Procédé selon la revendication 1, pour la préparation des composés de formule dans laquelle
R₁ et R₂ représentent chacun, indépendamment l'un de l'autre, l'hydrogène, le fluor, le chlore, le brome, un groupe alkyle en C 1-C 2, alcoxy en C 1-C 2, trifluorométhyle ou -OCHF₂;
R₃ représente un groupe méthyle;
R₄ représente un groupe alkyle en C 1-C 2; un groupe alkyle en C 2 substitué par le fluor, le chlore, le brome, des groupes cyano, alcoxy en C 1-C 2 ou méthylthio; un groupe allyle ou propargyle;
X représente l'oxygène ou le soufre.

8. Procédé selon la revendication 1, pour la préparation des composés de formule dans laquelle :
R₁ et R₂ représentent chacun, indépendamment l'un de l'autre, l'hydrogène, un halogène, un groupe alkyle en C 1-C 2, halogénométhyle, alcoxy en C 1-C 2 ou halogénoalcoxy en C 1-C 2;
R₃ représente l'hydrogène, un groupe alkyle en C 1-C 3; un groupe alkyle en C 1-C 2 substitué par un halogène ou un groupe hydroxy; un groupe cyclopropyle ou un groupe cyclopropyle portant un à trois substituants identiques ou différents choisis parmi les halogènes et les groupes méthyle;
R₄ représente un groupe alkyle en C 1-C 4, un groupe alkyle en C 2-C 3 substitué par des halogènes, des groupes cyano, alcoxy en C 1-C 2 ou alkylthio en C 1-C 2; un groupe alcényle en C 3-C 6, alcynyle en C 3-C 6 ou cycloalkyle en C 3-C 7;
X représente l'oxygène ou le soufre.

9. Procédé selon la revendication 1, pour la préparation des composés de formule dans laquelle :
R₁ et R₂ représentent chacun, indépendamment l'un de l'autre, l'hydrogène, le fluor, le chlore, le brome, un groupe méthyle, trifluorométhyle, méthoxy ou difluorométhoxy;
R₃ représente l'hydrogène; un groupe alkyle en C 1-C 3; un groupe alkyle en C 1-C 2 substitué par des halogènes ou des groupes hydroxy; un groupe cyclopropyle ou un groupe cyclopropyle portant un à trois substituants identiques ou différents choisis parmi les halogènes et les groupes méthyle;
R₄ représente un groupe alkyle en C 1-C 3; un groupe alkyle en C 2-C 3 substitué par le fluor, le chlore ou des groupes alcoxy en C 1-C 2; un groupe alcényle en C 3-C 6; un groupe alcynyle en C 3-C 6 ou cycloalkyle en C 3-C 6;
X représente l'oxygène ou le soufre;

10. Procédé selon la revendication 1, pour la préparation des composés de formule I dans laquelle :
R₁ et R₂ représentent chacun, indépendamment l'un de l'autre, l'hydrogène, le fluor, le chlore, un groupe méthyle, trifluorométhyle, méthoxy ou difluorométhoxy;
R₃ représente un groupe alkyle en C 1-C 3; un groupe méthyle substitué par un halogène ou un groupe hydroxy; un groupe cyclopropyle ou un groupe cyclopropyle portant un à trois substituants halogéno ou méthyle;
R₄ représente un-groupe alkyle en C 1-C 3; un groupe alkyle en C 2-C 3 substitué par le fluor, le chloreou un groupe méthoxy; un groupe alcényle en C 3-C 4, alcynyle en C 3-C 4; un groupe cyclopropyle; un groupe cyclohexyle;
X représente l'oxygène.

11. Procédé selon la revendication 1, pour la préparation des composés de formule I dans laquelle :
R₁ et R₂ représentent l'hydrogène;
R₃ représente un groupe alkyle en C 1-C 3; un groupe méthyle substitué par le fluor, le chlore ou le brome; un groupe cyclopropyle ou un groupe cyclopropyle substitué par le chlore ou des groupes méthyle;
R₄ représente un groupe alkyle en C 1-C 3; un groupe 2-chloréthyle, 2,2,2-trifluoréthyle; un groupe allyle ou propargyle;
X représente l'oxygène.

12. Procédé selon revendication 1, pour la préparation d'un composé de formule 1 choisi parmi les suivants
2-phénylamino-4-méthyl-6-méthoxyméthyl-pyrimidine;
2-phénylamino-4-cyclopropyl-6-méthoxyméthyl-pyrimidine.

## Revendications (Revendications pour l'(les) Etat(s) contractant(s) suivant(s) : suivants : CH, DE, FR, GB, IT, LI, NL)

1. Composés de formule 1 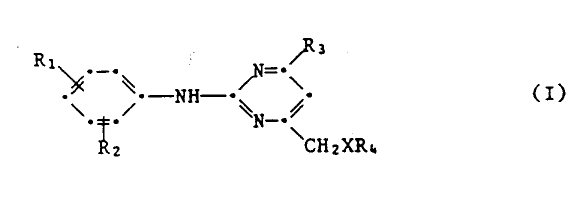 dans laquelle
R₁ et R₂ représentent chacun, indépendamment l'un de l'autre, l'hydrogène, un halogène, un groupe alkyle en C 1-C 3, halogénoalkyle en C 1-C 2, alcoxy en C 1-C 3 ou halogénoalcoxy en C 1-C 3;
R₃ représente l'hydrogène, un groupe alkyle en C 1-C 4, un groupe alkyle en C 1-C 2 à substituants halogéno ou hydroxy, un groupe cyclopropyle ou un groupe cyclopropyle à substituants halogéno et/ou méthyle;
R₄ représente un groupe alkyle en C 1-C 8; un groupe alkyle en C 2-C 6 substitué par des halogènes, des groupes hydroxy, cyano, alcoxy en C 1-C 4, alcoxyalcoxy en C 3-C 4 ou alkylthio en C 1-C 3 un groupe alcényle en C 3-C 6; un groupe alcynyle en C 3-C 6; un groupe cycloalkyle en C 3-C 7 ou un groupe cycloalkyle en C 3-C 7 substitué par des groupes méthyle;
X représente l'oxygène ou le soufre, sous réserve que, lorsque R₁ représente l'hydrogène, R₂ l'hydrogène, un halogène, un groupe alkyle en C 1-C 3, alcoxy en C 1-C 3 ou halogénoalkyle en C 1-C 2, et R₃ un halogène, un groupe alkyle en C 1-C 4 ou halogénoalkyle en C 1-C 2, R₄ ne peut représenter un groupe alkyle lorsque X représente le soufre, et ne peut représenter un groupe alkyle, alcényle ou alcynyle lorsque X représente l'oxygène.

2. Composés de formule 1 de la revendication 1, dans laquelle R₃, R₄ et X ont les significations indiquées et R₁ et R₂ représentent l'hydrogène.

3. Composés de formule 1 de la revendication 1, dans laquelle :
R₁ et R₂ représentent chacun, indépendamment l'un de l'autre, l'hydrogène, un halogène, un groupe alkyle en C 1-C 3, halogénoalkyle en C 1-C 2, alcoxy en C 1-C 3 ou halogénoalcoxy en C 1-C 3;
R₃ représente l'hydrogène, un groupe alkyle en C 1-C 4 ou cyclopropyle;
R₄ représente un groupe alkyle en C 1-C 8; un groupe alkyle en C 2-C 6 substitué par des halogènes, des groupes hydroxy, cyano, alcoxy en C 1-C 4, alcoxyalcoxy en C 3-C 4 ou alkylthio en C 1-C 3; un groupe alcényle en C 3-C 6; un groupe alcynyle en C 3-C 6; un groupe cycloalkyle en C 5-C 7 ou un groupe cycloalkyle en C 5-C 7 substitué par des groupes méthyle;
X représente l'oxygène ou le soufre.

4. Composés de formule 1 de la revendication 3, dans laquelle
R₁ et R₂ représentent chacun, indépendamment l'un de l'autre, l'hydrogène, le fluor, le chlore, le brome, un groupe alkyle en C 1-C 3, halogénoalkyle en C 1-C 2, alcoxy en C 1-C 3 ou halogénoalcoxy en C 1-C 2;
R₃ représente l'hydrogène, un groupe alkyle en C 1-C 3 ou cyclopropyle;
R₄ représente un groupe alkyle en C 1-C 6; un groupe alkyle en C 2-C 4 substitué par des halogènes, des groupes hydroxy, cyano, alcoxy en C 1-C 3, alcoxyalcoxy en C 3-C 4 ou alkylthio en C 1-C 2; un groupe alcényle en C 3; un groupe alcynyle en C 3; ou un groupe cycloalkyle en C 6-C 7 non substitué ou substitué par des groupes méthyle;
X représente l'oxygène ou le soufre;

5. Composés de formule 1 de la revendication 3, dans laquelle
R₁ et R₂ représentent chacun, indépendamment l'un de l'autre, l'hydrogène, le fiuor, le chlore, le brome, un groupe alkyle en C 1-C 3, alcoxy en C 1-C 3, trifluorométhyle, -OCHF₂, -OCF₂CHF₂, -OCF₂CHCIF, -OCF₂CHCI₂ ou -OCF₂CCI₂F;
R₃ représente l'hydrogène, un groupe méthyle, éthyle ou n-propyle;
R₄ représente un groupe alkyle en C 1-C 4; un groupe alkyle en C 2-C 3 substitué par des halogènes, des groupes hydroxy, cyano, alcoxy en C 1-C 2 ou alkylthio en C 1-C 2; un groupe alcényle en C 3-C 6; un groupe alcynyle en C 3-C 6 ou cyclohexyle;
X représente l'oxygène ou le soufre.

6. Composés de formule 1 de la revendication 4, dans laquelle
R₁ et R₂ représentent chacun, indépendamment l'un de l'autre, l'hydrogène, le fluor, le chlore, le brome, un groupe alkyle en C 1-C 2, alcoxy en C 1-C 2, trifluorométhyle, -OCHF₂, -OCF₃, -OCF₂CHF₂ ou -CF₂CHCIF;
R₃ représente l'hydrogène ou un groupe alkyle en C 1-C 2;
R₄ représente un groupe alkyle en C 1-C 3; un groupe alkyle en C 2 substitué par des halogènes, des groupes hydroxy, cyano, alcoxy en C 1-C 2 ou alkylthio en C 1-C 2; un groupe alcényle en C 3-C 4; un groupe alcynyle en C 3-C 4;
X représente l'oxygène ou le soufre.

7. Composés des formule 1 de la revendication 6, dans laquelle
R₁ et R₂ représentent chacun, indépendamment l'un de l'autre, l'hydrogène, le fluor, le chlore, le brome, un groupe alkyle en C 1-C 2, alcoxy en C 1-C 2, trifluorométhyle ou -OCHF₂;
R₃ représente un groupe méthyle;
R₄ représente un groupe alkyle en C 1-C 2; un groupe alkyle en C 2 substitué par le fluor, le chlore, le brome, des groupes cyano, alcoxy en C 1-C 2 ou méthylthio; un groupe allyle ou propargyle;
X représente l'oxygène ou le soufre.

8. Composés de formule 1 de la revendication 1, dans laquelle :
R₁ et R₂ représentent chacun, indépendamment l'un de l'autre, l'hydrogène, un halogène, un groupe alkyle en C 1-C 2, halogénométhyle, alcoxy en C 1-C 2 ou halogénoalcoxy en C 1-C 2;
R₃ représente l'hydrogène, un groupe alkyle en C 1-C 3; un groupe alkyle en C 1-C 2 substitué par un halogène ou un groupe hydroxy; un groupe cyclopropyle ou un groupe cyclopropyle portant un à trois substituants identiques ou différents choisis parmi les halogènes et les groupes méthyle;
R₄ représente un groupe alkyle en C 1-C 4, un groupe alkyle en C 2-C 3 substitué par des halogènes, des groupes cyano, alcoxy en C 1-C 2 ou alkylthio en C 1-C 2; un groupe alcényle en C 3-C 6, alcynyle en C 3-C 6 ou cycloalkyle en C 3-C 7;
X représente l'oxygène ou le soufre.

9. Composés de formule 1 de la revendication 8, dans laquelle :
R₁ et R₂ représentent chacun, indépendamment l'un de l'autre, l'hydrogène, le fluor, le chlore, le brome, un groupe méthyle, trifluorométhyle, méthoxy ou difluorométhoxy;
R₃ représente l'hydrogène; un groupe alkyle en C 1-C 3; un groupe alkyle en C 1-C 2 substitué par des halogènes ou des groupes hydroxy; un groupe cyclopropyle ou un groupe cyclopropyle portant un à trois substituants identiques ou différents choisis parmi les halogènes et les groupes méthyle;
R₄ représente un groupe alkyle en C 1-C 3; un groupe alkyle en C 2-C 3 substitué par le fluor, le chlore ou des groupes alcoxy en C 1-C 2; un groupe alcényle en C 3-C 6; un groupe alcynyle en C 3-C 6 ou cycloalkyle en C 3-C 6;
X représente l'oxygène ou le soufre.

10. Composés de formule 1 de la revendication 9, dans laquelle
R₁ et R₂ représentent chacun, indépendamment l'un de l'autre, l'hydrogène, le fluor, le chlore, un groupe méthyle, trifluorométhyle, méthoxy ou difluorométhoxy;
R₃ représente un groupe alkyle en C 1-C 3; un groupe méthyle substitué par un halogène ou un groupe hydroxy; un groupe cyclopropyle ou un groupe cyclopropyle portant un à trois substituants halogéno ou méthyle;
R₄ représente un groupe alkyle en C 1-C 3; un groupe alkyle en C 2-C 3 substitué par le fluor, le chlore ou un groupe méthoxy; un groupe alcényle en C 3-C 4, alcynyle en C 3-C 4; un groupe cyclopropyle; un groupe cyclohexyle;
X représente l'oxygène.

11. Composés de formule 1 de la revendication 10, dans laquelle :
R₁ et R₂ représentent l'hydrogène;
R₃ représente un groupe alkyle en C 1-C 3; un groupe méthyle substitué par le fluor, le chlore ou le brome; un groupe cyclopropyle ou un groupe cyclopropyle substitué par le chlore ou des groupes méthyle;
R₄ représente un groupe alkyle en C 1-C 3; un groupe 2-chloréthyle, 2,2,2-trifluoréthyle; un groupe allyle ou propargyle;
X représente l'oxygène.

12. Un composé de formule I selon revendication 3 : 1 a 2-phénylamino-4-cyclopropyl-6-méthoxyméthylpy- rimidine.

13. Procédé de préparation des composés de formule 1 de la revendication 1, caractérisé en ce que :
1. on fait réagir à des températures de 60 à 160 °C un sel de phénylguanidine de formule Ila 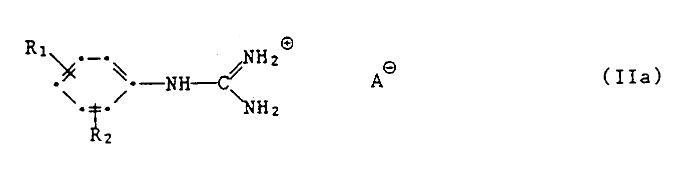 ou la guanidine dont il dérive, de formule Ilb 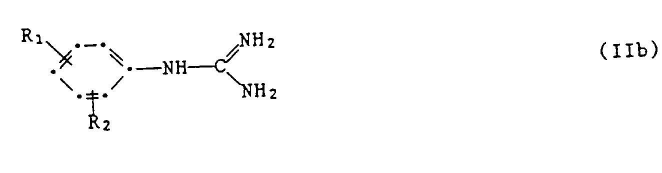 avec une dicétone de formule III 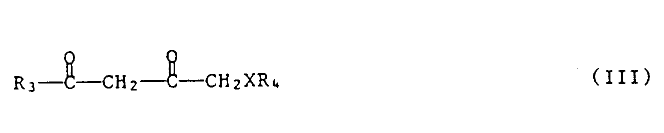 sans solvant ou dans un solvant aprotonique, mais de préférence dans un solvant protonique, ou bien
2. Dans un procédé à plusieurs stades opératoires :
2.1 on fait réagir l'urée avec une cétone de formule III 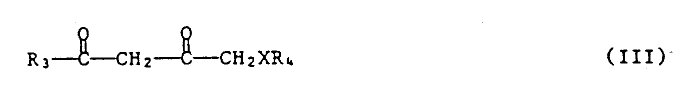 en présence d'un acide, dans un solvant inerte, à des températures de 20 à 140°C puis on cyclise à la température de reflux du solvant utilisé, en un dérivé de pyrimidine de formule V 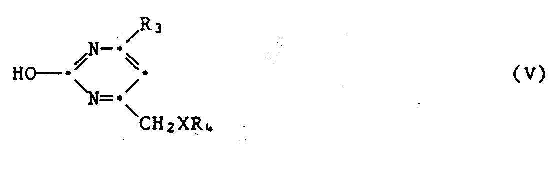 et
2.2 on convertit le composé obtenu, répondant à la formule V, à l'aide d'un excès de POCI₃, sans solvant ou dans un solvant inerte à l'égard de POC!₃, à des températures de 50 à 110 °C, en le composé de formule VI 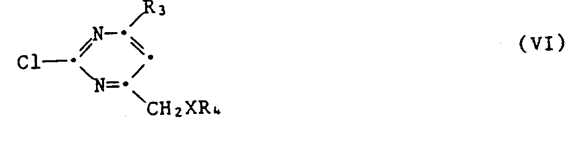 et
2.3 on fait réagir le composé obtenu, répondant à la formule VI, avec un dérivé de l'aniline de formule VII 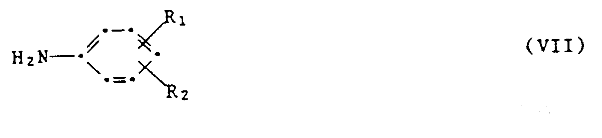 selon les conditions opératoires,
a) soit en présence d'un accepteur de protons sans solvant ou dans un solvant protonique ou aprotonique,
b) soit en présence d'un acide dans un solvant inerte,
dans les deux cas à des températures de 60 à 120°C, ou bien
3. Dans un procédé à deux stades opératoires :
3.1 on cyclise un sel de guanidine de formule VIII 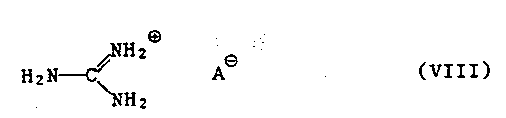 à l'aide d'une dicétone de formule III 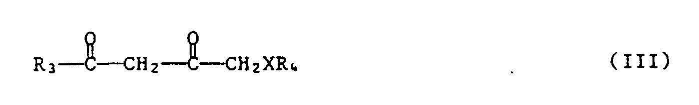
a) sans solvant à des températures de 100 à 160 °C, ou bien
b) dans un solvant protonique ou aprotonique ou dans un mélange de solvants protoniques et aprotoniques, à des températures de 30 à 140°C,
en un dérivé de pyrimidine de formule IX 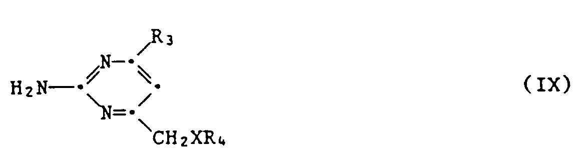 et
3.2 on fait réagir le composé obtenu, répondant à la formule X, 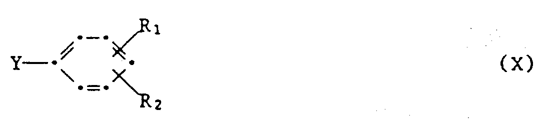 avec scissîon de HY, en présence d'un accepteur de protons, dans des solvants aprotoniques à des températures de 30 à 140°C,
les symboles R₁ à R₄ et X ayant dans les formules II à X les significations indiquées en référence à la formule I, A^{S} représentant un anion d'acide et Y un halogène.

14. Produit pour combattre ou prévenir une attaque par des insectes ou microorganismes nuisibles, caractérisé en ce qu'il contient au moins un composant actif consistant en un composé de formule 1 de la revendication 1.

15. Produit selon revendication 14, caractérisé en ce qu'il contient au moins un composant actif consistant en un composé de formule 1 de la revendication 2.

16. Produit selon revendication 14, caractérisé en ce qu'il contient au moins un composant actif consistant en un composé de formule 1 de la revendication 3.

17. Produit selon revendication 14, caractérisé en ce qu'il contient au moins un composant actif consistant en un composé de formule I selon l'une des revendications 4 à 12.

18. Produit selon revendication 14, caractérisé en ce qu'il contient de 0,1 à 99 % en poids d'une substance active de formule I, de 99,9 à 1 % en poids d'un additif solide ou liquide et de 0 à 25 % en poids d'un agent tensioactif.

19. Procédé de préparation d'un produit agrochimique tel que défini dans la revendication 14, caractérisé en ce que l'on mélange intimement au moins un composé de formule I tel que défini dans la revendication 1 avec des additifs solides ou liquides et des agents tensioactifs appropriés.

20. Procédé pour combattre ou prévenir une attaque de végétaux cultivés par des insectes nuisibles ou des microorganismes phytopathogènes, caractérisé èn ce que l'on applique sur la plante ou son habitat un composé de formule I tel que défini dans la revendication 1.

21. Utilisation de composés de formule 1 de la revendication 1 pour combattre et/ou prévenir une attaque par des microorganismes nuisibles.

22. Utilisation selon revendication 21, caractérisé en ce que les microorganismes sont des mycètes phytopathogènes.

23. Utilisation selon revendication 22, contre des mycètes de la classe des Fungi imperfecti.

24. Utilisation selon revendication 23, contre une attaque par Botrytis.
